# EUROPEAN PATENT APPLICATION

(11) **EP 2 295 574 A1**
(43) Date of publication of application: **16.03.2011**
(21) Application number: 10010658.2
(22) Date of filing: 13.10.1999
(51) Int. Cl.: C12N 15/12, C07K 14/72

(54) **Human orphan G protein-coupled receptors**

(30) Priority: 20.11.1998 US 109213 P; 16.02.1999 US 120416 P; 26.02.1999 US 121852 P; 12.03.1999 US 123946 P; 12.03.1999 US 123949 P; 28.05.1999 US 136436 P; 28.05.1999 US 136437 P; 28.05.1999 US 136439 P; 28.05.1999 US 136567 P; 28.05.1999 US 137127 P; 28.05.1999 US 137131 P; 29.06.1999 US 141448 P; 29.09.1999 US 156653 P; 29.09.1999 US 156633 P; 29.09.1999 US 156555 P; 29.09.1999 US 156634 P; 01.10.1999 US 157280 P; 01.10.1999 US 157294 P; 01.10.1999 US 157281 P; 01.10.1999 US 157293 P; 01.10.1999 US 157282 P; 12.10.1999 US 417044; 12.10.1999 US 416760
(62) Divisional of application: 07001452.7
(71) Applicant: Arena Pharmaceuticals, Inc., San Diego, CA 92121 (US)
(72) Inventor: Chen, Ruoping, San Diego CA 92130 (US); Leonard, James, N., San Diego CA 92124 (US)
(74) Representative: Cripps, Joanna Elizabeth

(57) **Abstract**

The invention disclosed in this patent document relates to transmembrane receptors, more particularly to endogenous, human orphan G protein-coupled receptors.

## Description

This patent document claims priority benefit of each of the following applications, all filed with the United States Patent and Trademark Office via U.S. Express Mail on the indicated filing dates: U.S. Provisional Number 60/121,852, filed; February 26, 1999 claiming the benefit of U.S. Provisional Number 60/109,213, filed November 20, 1998; U.S. Provisional Number 60/120,416, filed February 16, 1999; U.S. Provisional Number 60/123,946, filed March 12, 1999; U.S. Provisional Number 60/123,949, filed March 12, 1999; U.S. Provisional Number 60/136,436, filed May 28, 1999; U.S. Provisional Number 60/136,439, filed May 28, 1999; U.S. Provisional Number 60/136,567, filed May 28, 1999; U.S. Provisional Number 60/137,127, filed May 28, 1999; U.S. Provisional Number 60/137,131, filed May 28, 1999; U.S. Provisional Number 141,448, filed June 29, 1999 claiming priority from U.S. Provisional Number 60/136,437, filed May 28, 1999; U.S. Provisional Number ______ (Arena Pharmaceuticals, Inc. docket number CHN10-1), filed September 29, 1999; U.S. Provisional Number 60/156,333, filed September 29, 1999; U.S. Provisional Number 60/156,555, filed September 29, 1999; U.S. Provisional Number 60/156,634, filed September 29, 1999; U.S. Provisional Number _ (Arena Pharmaceuticals, Inc. docket number RUP6-1), filed October 1, 1999; U.S. Provisional Number __ (Arena Pharmaceuticals, Inc. docket number RUP7-1), filed October 1, 1999; U.S. Provisional Number __ (Arena Pharmaceuticals, Inc. docket number CHN6-1), filed October 1, 1999; U.S. Provisional Number __ (Arena Pharmaceuticals, Inc. docket number RUP5-1), filed October 1, 1999; U.S. Provisional Number __ (Arena Pharmaceuticals, Inc. docket number CHN9-1), filed October 1, 1999. This patent document is related to U.S. Serial Number 09/170,496 filed October 13, 1998, and U.S. Serial Number unknown (Woodcock Washburn Kurtz Mackiewicz & Norris, LLP docket number AREN-0054) filed on October 12, 1999 (via U.S. Express Mail) both being incorporated herein by reference. This patent document also is related to U.S. Serial No. 09/364,425; filed July 30, 1999, which is incorporated by reference in its entirety. This application also claims priority to U.S. Serial Number ______ (Woodcock, Washburn, Kurtz, Makiewicz & Norris, LLP docket number AREN-0050), filed on October 12, 1999 (via U.S. Express Mail), incorporated by reference herein in its entirety. Each of the foregoing applications are incorporated herein by reference in their entirety.

### FIELD OF THE INVENTION

The invention disclosed in this patent document relates to transmembrane receptors, and more particularly to endogenous, orphan, human G protein-coupled receptors ("GPCRs").

### BACKGROUND OF THE INVENTION

Although a number of receptor classes exist in humans, by far the most abundant and therapeutically relevant is represented by the G protein-coupled receptor (GPCR or GPCRs) class. It is estimated that there are some 100,000 genes within the human genome, and of these, approximately 2% or 2,000 genes, are estimated to code for GPCRs. Receptors, including GPCRs, for which the endogenous ligand has been identified are referred to as "known" receptors, while receptors for which the endogenous ligand has not been identified are referred to as "orphan" receptors. GPCRs represent an important area for the development of pharmaceutical products: from approximately 20 of the 100 known GPCRs, 60% of all prescription pharmaceuticals have been developed. This distinction is not merely semantic, particularly in the case of GPCRs. Thus, the orphan GPCRs are to the pharmaceutical industry what gold was to California in the late 19^{th} century - an opportunity to drive growth, expansion, enhancement and development.

GPCRs share a common structural motif. All these receptors have seven sequences of between 22 to 24 hydrophobic amino acids that form seven alpha helices, each of which spans the membrane (each span is identified by number, i.e., transmembrane-1 (TM-1), transmebrane-2 (TM-2), etc.). The transmembrane helices are joined by strands of amino acids between transmembrane-2 and transmembrane-3, transmembrane-4 and transmembrane-5, and transmembrane-6 and transmembrane-7 on the exterior, or "extracellular" side, of the cell membrane (these are referred to as "extracellular" regions 1, 2 and 3 (EC-1, EC-2 and EC-3), respectively). The transmembrane helices are also joined by strands of amino acids between transmembrane-1 and transmembrane-2, transmembrane-3 and transmembrane-4, and transmembrane-5 and transmembrane-6 on the interior, or "intracellular" side, of the cell membrane (these are referred to as "intracellular" regions 1, 2 and 3 (IC-1, IC-2 and IC-3), respectively). The "carboxy" ("C") terminus of the receptor lies in the intracellular space within the cell, and the "amino" ("N") terminus of the receptor lies in the extracellular space outside of the cell.

Generally, when an endogenous ligand binds with the receptor (often referred to as "activation" of the receptor), there is a change in the conformation of the intracellular region that allows for coupling between the intracellular region and an intracellular "G-protein." It has been reported that GPCRs are "promiscuous" with respect to G proteins, i.e., that a GPCR can interact with more than one G protein. See, Kenakin, T., 43 Life Sciences 1095 (1988). Although other G proteins exist, currently, Gq, Gs, Gi, and Go are G proteins that have been identified. Endogenous ligand-activated GPCR coupling with the G-protein begins a signaling cascade process (referred to as "signal transduction"). Under normal conditions, signal transduction ultimately results in cellular activation or cellular inhibition. It is thought that the IC-3 loop as well as the carboxy terminus of the receptor interact with the G protein.

Under physiological conditions, GPCRs exist in the cell membrane in equilibrium between two different conformations: an "inactive" state and an "active" state. A receptor in an inactive state is unable to link to the intracellular signaling transduction pathway to produce a biological response. Changing the receptor conformation to the active state allows linkage to the transduction pathway (via the G-protein) and produces a biological response. A receptor may be stabilized in an active state by an endogenous ligand or a compound such as a drug.

### SUMMARY OF THE INVENTION

Disclosed herein are human endogenous orphan G protein-coupled receptors.

### BRIEF DESCRIPTION OF THE DRAWINGS

**Figures 1A** and **1B** provide reference "grids" for certain dot-blots provided herein *(see also,* Figure 2A and 2B, respectively).
**Figures 2A** and **2B** provide reproductions of the results of certain dot-blot analyses resulting from hCHN3 and hCHN8, respectively *(see also,* Figures 1A and 1B, respectively).
**Figure 3** provides a reproduction of the results of RT-PCR analysis of hRUP3.
**Figure 4** provides a reproduction of the results of RT-PCR analysis of hRUP4.
**Figure 5** provides a reproduction of the results of RT-PCR analysis of hRUP6.

### DETAILED DESCRIPTION

The scientific literature that has evolved around receptors has adopted a number of terms to refer to ligands having various effects on receptors. For clarity and consistency, the following definitions will be used throughout this patent document. To the extent that these definitions conflict with other definitions for these terms, the following definitions shall control:
**AMINO ACID ABBREVIATIONS** used herein are set out in **Table 1:**

| **TABLE 1** | | |
|---|---|---|
| ALANINE | ALA | A |
| ARGININE | ARG | R |
| ASPARAGINE | ASN | N |
| ASPARTIC ACID | ASP | D |
| CYSTEINE | CYS | C |
| GLUTAMIC ACID | GLU | E |
| GLUTAMINE | GLN | Q |
| GLYCINE | GLY | G |
| HISTIDINE | HIS | H |
| ISOLEUCINE | ILE | I |
| LEUCINE | LEU | L |
| LYSINE | LYS | K |
| METHIONINE | MET | M |
| PHENYLALANINE | PHE | F |
| PROLINE | PRO | P |
| SERINE | SER | S |
| THREONINE | THR | T |
| TRYPTOPHAN | TRP | W |
| TYROSINE | TYR | Y |
| VALINE | VAL | V |

**COMPOSITION** means a material comprising at least one component.

**ENDOGENOUS** shall mean a material that a mammal naturally produces. ENDOGENOUS in reference to, for example and not limitation, the term "receptor," shall mean that which is naturally produced by a mammal (for example, and not limitation, a human) or a virus. By contrast, the term **NON-ENDOGENOUS** in this context shall mean that which is not naturally produced by a mammal (for example, and not limitation, a human) or a virus.

**HOST CELL** shall mean a cell capable of having a Plasmid and/or Vector incorporated therein. In the case of a prokaryotic Host Cell, a Plasmid is typically replicated as a autonomous molecule as the Host Cell replicates (generally, the Plasmid is thereafter isolated for introduction into a eukaryotic Host Cell); in the case of a eukaryotic Host Cell, a Plasmid is integrated into the cellular DNA of the Host Cell such that when the eukaryotic Host Cell replicates, the Plasmid replicates. Preferably, for the purposes of the invention disclosed herein, the Host Cell is eukaryotic, more preferably, mammalian, and most preferably selected from the group consisting of 293, 293T and COS-7 cells.

**LIGAND** shall mean an endogenous, naturally occurring molecule specific for an endogenous, naturally occurring receptor.

**NON-ORPHAN RECEPTOR** shall mean an endogenous naturally occurring molecule specific for an endogenous naturally occurring ligand wherein the binding of a ligand to a receptor activates an intracellular signaling pathway.

**ORPHAN RECEPTOR** shall mean an endogenous receptor for which the endogenous ligand specific for that receptor has not been identified or is not known.

**PLASMID** shall mean the combination of a Vector and cDNA. Generally, a Plasmid is introduced into a Host Cell for the purposes of replication and/or expression of the cDNA as a protein.

**VECTOR** sin reference to cDNA shall mean a circular DNA capable of incorporating at least one cDNA and capable of incorporation into a Host Cell.

The order of the following sections is set forth for presentational efficiency and is not intended, nor should be construed, as a limitation on the disclosure or the claims to follow.

### A. Identification of Human GPCRs

The efforts of the Human Genome project have led to the identification of a plethora of information regarding nucleic acid sequences located within the human genome; it has been the case in this endeavor that genetic sequence information has been made available without an understanding or recognition as to whether or not any particular genomic sequence does or may contain open-reading frame information that translate human proteins. Several methods of identifying nucleic acid sequences within the human genome are within the purview of those having ordinary skill in the art. For example, and not limitation, a variety of GPCRs, disclosed herein, were discovered by reviewing the GenBank™ database, while other GPCRs were discovered by utilizing a nucleic acid sequence of a GPCR, previously sequenced, to conduct a BLAST™ search of the EST database. **Table A**, below, lists the disclosed endogenous orphan GPCRs along with a GPCR's respective homologous GPCR:

**TABLE A**

| **Disclosed Human Orphan GPCRs** | **Accession Number Identified** | **Open Reading Frame (Base Pairs)** | **Per Cent Homology To Designated GPCR** | **Reference To Homologous GPCR (Accession No.)** |
|---|---|---|---|---|
| **hARE-3** | AL033379 | 1,260 bp | 52.3% LPAR | U92642 |
| **hARE-4** | AC006087 | 1,119 bp | 36% P2Y5 | AF000546 |
| **hARE-5** | AC006255 | 1,104 bp | 32% *Oryzias latipes* | D43633 |
| **hGPR27** | AA775870 | 1,128 bp | | |
| **hARE-1** | AI090920 | 999 bp | 43% KIAA0001 | D13626 |
| **hARE-2** | AA359504 | 1,122 bp | 53% GPR27 | |
| **hPPR1** | H67224 | 1,053 bp | 39% EBI1 | L31581 |
| **hG2A** | AA754702 | 1,113 bp | 31% GPR4 | L36148 |
| **hRUP3** | AL035423 | 1,005 bp | 30% *Drosophila melanogast* er | 2133653 |
| **hRUP4** | AI307658 | 1,296 bp | 32% pNPGPR 28% and 29 % *Zebra fish* Ya and Yb, respectivel Y | NP_004876 AAC41276 and AAB94616 |
| **hRUP5** | AC005849 | 1,413 bp | 25% DEZ 23% FMLPR | Q99788 P21462 |
| **hRUP6** | AC005871 | 1,245 bp | 48% GPR66 | NP_006047 |
| **hRUP7** | AC007922 | 1,173 bp | 43% H3R | AF140538 |
| **hCHN3** | EST 36581 | 1, 113 bp | 53% GPR27 | |
| **hCHN4** | AA804531 | 1,077 bp | 32% thrombin | 4503637 |
| **hCHN6** | EST 2134670 | 1,503 bp | 36% edg-1 | NP_001391 |
| **hCHN8** | EST 764455 | 1,029 bp | 47% KIAA0001 | D13626 |
| **hCHN9** | EST 1541536 | 1,077 bp | 41% LTB4R | NM_000752 |
| **hCHN10** | EST 1365839 | 1,055 bp | 35% P2Y | NM_002563 |

Receptor homology is useful in terms of gaining an appreciation of a role of the disclosed receptors within the human body. Additionally, such homology can provide insight as to possible endogenous ligand(s) that may be natural activators for the disclosed orphan GPCRs.

Some additional embodiments of the invention are described in the following paragraphs.
1. A cDNA encoding a human G protein-coupled receptor comprising SEQ.ID.NO.: 1.
2. A human G protein-coupled receptor encoded by the cDNA of SEQ.ID.NO.: 1 comprising SEQ.ID.NO.: 2.
3. A Plasmid comprising a Vector and the cDNA of SEQ.ID.NO.:1.
4. A Host Cell comprising the Plasmid of paragraph 3.
5. A cDNA encoding a human G protein-coupled receptor comprising SEQ.ID.NO.: 3.
6. A human G protein-coupled receptor encoded by the cDNA of SEQ.ID.NO.: 3 comprising SEQ.ID.NO.: 4.
7. A Plasmid comprising a Vector and the cDNA of SEQ.ID.NO.:3.
8. A Host Cell comprising the Plasmid of paragraph 7.
9. A cDNA encoding a human G protein-coupled receptor comprising SEQ.ID.NO.: 5.
10. A human G protein-coupled receptor encoded by the cDNA of SEQ.ID.NO.: 5 comprising SEQ.ID.NO.: 6.
11. A Plasmid comprising a Vector and the cDNA of SEQ.ID.NO.:5.
12. A Host Cell comprising the Plasmid of paragraph 11.
13. A cDNA encoding a human G protein-coupled receptor comprising SEQ.ID.NO.: 7.
14. A human G protein-coupled receptor encoded by the cDNA of SEQ.ID.NO.: 7 comprising SEQ.ID.NO.: 8.
15. A Plasmid comprising a Vector and the cDNA of SEQ.ID.NO.:7.
16. A Host Cell comprising the Plasmid of paragraph 15.
17. A cDNA encoding a human G protein-coupled receptor comprising SEQ.ID.NO.: 9.
18. A human G protein-coupled receptor encoded by the cDNA of SEQ.ID.NO.: 9 comprising SEQ.ID.NO.: 10.
19. A Plasmid comprising a Vector and the cDNA of SEQ.ID.NO.:9.
20. A Host Cell comprising the Plasmid of paragraph 19.
21. A cDNA encoding a human G protein-coupled receptor comprising SEQ.ID.NO.: 11.
22. A human G protein-coupled receptor encoded by the cDNA of SEQ.ID.NO.: 11 comprising SEQ.ID.NO.:12.
23. A Plasmid comprising a Vector and the cDNA of SEQ.ID.NO.:11.
24. A Host Cell comprising the Plasmid of paragraph 23.
25. A cDNA encoding a human G protein-coupled receptor comprising SEQ.ID.NO.: 13.
26. A human G protein-coupled receptor encoded by the cDNA of SEQ.ID.NO.: 13 comprising SEQ.ID.NO.: 14.
27. A Plasmid comprising a Vector and the cDNA of SEQ.ID.NO.:13.
28. A Host Cell comprising the Plasmid of paragraph 27.
29. A cDNA encoding a human G protein-coupled receptor comprising SEQ.ID.NO.: 15.
30. A human G protein-coupled receptor encoded by the cDNA of SEQ.ID.NO.: 15 comprising SEQ.ID.NO.: 16.
31. A Plasmid comprising a Vector and the cDNA of SEQ.ID.NO.:15.
32. A Host Cell comprising the Plasmid of paragraph 31.
33. A cDNA encoding a human G protein-coupled receptor comprising SEQ.ID.NO.: 17.
34. A human G protein-coupled receptor encoded by the cDNA of SEQ.ID.NO.: 17 comprising SEQ.ID.NO.: 18.
35. A Plasmid comprising a Vector and the cDNA of SEQ.ID.NO.:17.
36. A Host Cell comprising the Plasmid of paragraph 35.
37. A cDNA encoding a human G protein-coupled receptor comprising SEQ.ID.NO.: 19.
38. A human G protein-coupled receptor encoded by the cDNA of SEQ.ID.NO.: 19 comprising SEQ.ID.NO.: 20.
39. A Plasmid comprising a Vector and the cDNA of SEQ.ID.NO.:19.
40. A Host Cell comprising the Plasmid of paragraph 39.
41. A cDNA encoding a human G protein-coupled receptor comprising SEQ.ID.NO.: 21.
42. A human G protein-coupled receptor encoded by the cDNA of SEQ.ID.NO.: 21 comprising SEQ.ID.NO.: 22.
43. A Plasmid comprising a Vector and the cDNA of SEQ.ID.NO.:21.
44. A Host Cell comprising the Plasmid of paragraph 43.
45. A cDNA encoding a human G protein-coupled receptor comprising SEQ.ID.NO.: 23.
46. A human G protein-coupled receptor encoded by the cDNA of SEQ.ID.NO.: 23 comprising SEQ.ID.NO.: 24.
47. A Plasmid comprising a Vector and the cDNA of SEQ.ID.NO.: 23.
48. A Host Cell comprising the Plasmid of paragraph 47.
49. A cDNA encoding a human G protein-coupled receptor comprising SEQ.ID.NO.: 25.
50. A human G protein-coupled receptor encoded by the cDNA of SEQ.ID.NO.: 25 comprising SEQ.ID.NO.: 26.
51. A Plasmid comprising a Vector and the cDNA of SEQ.ID.NO.:25.
52. A Host Cell comprising the Plasmid of paragraph 51.
53. A cDNA encoding a human G protein-coupled receptor comprising SEQ.ID.NO.: 27.
54. A human G protein-coupled receptor encoded by the cDNA of SEQ.ID.NO.: 27 comprising SEQ.ID.NO.: 28.
55. A Plasmid comprising a Vector and the cDNA of SEQ.ID.NO.:27.
56. A Host Cell comprising the Plasmid of paragraph 55.
57. A cDNA encoding a human G protein-coupled receptor comprising SEQ.ID.NO.: 29.
58. A human G protein-coupled receptor encoded by the cDNA of SEQ.ID.NO.: 29 comprising SEQ.ID.NO.: 30.
59. A Plasmid comprising a Vector and the cDNA of SEQ.ID.NO.:29.
60. A Host Cell comprising the Plasmid of paragraph 59.
61. A cDNA encoding a human G protein-coupled receptor comprising SEQ.ID.NO.: 31.
62. A human G protein-coupled receptor encoded by the cDNA of SEQ.ID.NO.: 31 comprising SEQ.ID.NO.: 32.
63. A Plasmid comprising a Vector and the cDNA of SEQ.ID.NO.:31.
64. A Host Cell comprising the Plasmid of paragraph 63.
65. A cDNA encoding a human G protein-coupled receptor comprising SEQ.ID.NO.: 33.
66. A human G protein-coupled receptor encoded by the cDNA of SEQ.ID.NO.: 33 comprising SEQ.ID.NO.: 34.
67. A Plasmid comprising a Vector and the cDNA of SEQ.ID.NO.:33.
68. A Host Cell comprising the Plasmid of paragraph 67.
69. A cDNA encoding a human G protein-coupled receptor comprising SEQ.ID.NO.: 35.
70. A human G protein-coupled receptor encoded by the cDNA of SEQ.ID.NO.: 35 comprising SEQ.ID.NO.: 36.
71. A Plasmid comprising a Vector and the cDNA of SEQ.ID.NO.:35.
72. A Host Cell comprising the Plasmid of paragraph 71. A cDNA encoding a human G protein-coupled receptor comprising SEQ.ID.NO.: 37.
73. A human G protein-coupled receptor encoded by the cDNA of SEQ.ID.NO.: 37 comprising SEQ.ID.NO.: 38.
74. A Plasmid comprising a Vector and the cDNA of SEQ.ID.NO.:37.
75. A Host Cell comprising the Plasmid of paragraph 75.

### B. Receptor Screening

Techniques have become more readily available over the past few years for endogenous-ligand identification (this, primarily, for the purpose of providing a means of conducting receptor-binding assays that require a receptor's endogenous ligand) because the traditional study of receptors has always proceeded from the a priori assumption (historically based) that the endogenous ligand must first be identified before discovery could proceed to find antagonists and other molecules that could affect the receptor. Even in cases where an antagonist might have been known first, the search immediately extended to looking for the endogenous ligand. This mode of thinking has persisted in receptor research even after the discovery of constitutively activated receptors. What has not been heretofore recognized is that it is the active state of the receptor that is most useful for discovering agonists, partial agonists, and inverse agonists of the receptor. For those diseases which result from an overly active receptor or an under-active receptor, what is desired in a therapeutic drug is a compound which acts to diminish the active state of a receptor or enhance the activity of the receptor, respectively, not necessarily a drug which is an antagonist to the endogenous ligand. This is because a compound that reduces or enhances the activity of the active receptor state need not bind at the same site as the endogenous ligand. Thus, as taught by a method of this invention, any search for therapeutic compounds should start by screening compounds against the ligand-independent active state.

As is known in the art, GPCRs can be "active" in their endogenous state even without the binding of the receptor's endogenous ligand thereto. Such naturally-active receptors can be screened for the direct identification (i.e., without the need for the receptor's endogenous ligand) of, in particular, inverse agonists. Alternatively, the receptor can be "activated" via, e.g., mutation of the receptor to establish a non-endogenous version of the receptor that is active in the absence of the receptor's endogenous ligand.

Screening candidate compounds against an endogenous or non-endogenous, constitutively activated version of the human orphan GPCRs disclosed herein can provide for the direct identification of candidate compounds which act at this cell surface receptor, without requiring use of the receptor's endogenous ligand. By determining areas within the body where the endogenous version of human GPCRs disclosed herein is expressed and/or over-expressed, it is possible to determine related disease/disorder states which are associated with the expression and/or over-expression of the receptor; such an approach is disclosed in this patent document.

With respect to creation of a mutation that may evidence constitutive activation of human orphan GPCRs disclosed herein is based upon the distance from the proline residue at which is presumed to be located within TM6 of the GPCR typically nears the TM6/IC3 interface (such proline residue appears to be quite conserved). By mutating the amino acid residue located 16 amino acid residues from this residue (presumably located in the IC3 region of the receptor) to, most preferably, a lysine residue, such activation may be obtained. Other amino acid residues may be useful in the mutation at this position to achieve this objective.

### C. Disease/Disorder Identification and/or Selection

Preferably, the DNA sequence of the human orphan GPCR can be used to make a probe for (a) dot-blot analysis against tissue-mRNA, and/or (b) RT-PCR identification of the expression of the receptor in tissue samples. The presence of a receptor in a tissue source, or a diseased tissue, or the presence of the receptor at elevated concentrations in diseased tissue compared to a normal tissue, can be preferably utilized to identify a correlation with a treatment regimen, including but not limited to, a disease associated with that disease. Receptors can equally well be localized to regions of organs by this technique. Based on the known functions of the specific tissues to which the receptor is localized, the putative functional role of the receptor can be deduced.

### D. Screening of Candidate Compounds

### 1. Generic GPCR screening assay techniques

When a G protein receptor becomes constitutively active (i.e., active in the absence of endogenous ligand binding thereto), it binds to a G protein (e.g., Gq, Gs, Gi, Go) and stimulates the binding of GTP to the G protein. The G protein then acts as a GTPase and slowly hydrolyzes the GTP to GDP, whereby the receptor, under normal conditions, becomes deactivated. However, constitutively activated receptors continue to exchange GDP to GTP. A non-hydrolyzable analog of GTP, (³⁵S]GTPγS, can be used to monitor enhanced binding to membranes which express constitutively activated receptors. It is reported that [³⁵S]GTPγS can be used to monitor G protein coupling to membranes in the absence and presence of ligand. An example of this monitoring, among other examples well-known and available to those in the art, was reported by Traynor and Nahorski in 1995. The preferred use of this assay system is for initial screening of candidate compounds because the system is generically applicable to all G protein-coupled receptors regardless of the particular G protein that interacts with the intracellular domain of the receptor.

### 2. Specific GPCR screening assay techniques

Once candidate compounds are identified using the "generic" G protein-coupled receptor assay (i.e., an assay to select compounds that are agonists, partial agonists, or inverse agonists), further screening to confirm that the compounds have interacted at the receptor site is preferred. For example, a compound identified by the "generic" assay may not bind to the receptor, but may instead merely "uncouple" the G protein from the intracellular domain.

### a. Gs and Gi.

Gs stimulates the enzyme adenylyl cyclase. Gi (and Go), on the other hand, inhibit this enzyme. Adenylyl cyclase catalyzes the conversion of ATP to cAMP; thus, constitutively activated GPCRs that couple the Gs protein are associated with increased cellular levels of cAMP. On the other hand, constitutively activated GPCRs that couple the Gi (or Go) protein are associated with decreased cellular levels of cAMP. *See, generally,* "Indirect Mechanisms of Synaptic Transmission," Chpt. 8, From Neuron To Brain (3rd Ed.) Nichols, J.G. et al eds. Sinauer Associates, Inc. (1992). Thus, assays that detect cAMP can be utilized to determine if a candidate compound is, *e.g.,* an inverse agonist to the receptor *(i.e.,* such a compound would decrease the levels of cAMP). A variety of approaches known in the art for measuring cAMP can be utilized; a most preferred approach relies upon the use of anti-cAMP antibodies in an ELISA-based format. Another type of assay that can be utilized is a whole cell second messenger reporter system assay. Promoters on genes drive the expression of the proteins that a particular gene encodes. Cyclic AMP drives gene expression by promoting the binding of a cAMP-responsive DNA binding protein or transcription factor (CREB) which then binds to the promoter at specific sites called cAMP response elements and drives the expression of the gene. Reporter systems can be constructed which have a promoter containing multiple cAMP response elements before the reporter gene, e.g., β-galactosidase or luciferase. Thus, a constitutively activated Gs-linked receptor causes the accumulation of cAMP that then activates the gene and expression of the reporter protein. The reporter protein such as ß-galactosidase or luciferase can then be detected using standard biochemical assays (Chen et al. 1995).

### b. Go and Gq.

Gq and Go are associated with activation of the enzyme phospholipase C, which in turn hydrolyzes the phospholipid PIP₂, releasing two intracellular messengers: diacycloglycerol (DAG) and inistol 1,4,5-triphoisphate (IP₃). Increased accumulation of IP₃ is associated with activation of Gq- and Go-associated receptors. See, *generally,* "Indirect Mechanisms of Synaptic Transmission," Chpt. 8, From Neuron To Brain (3rd Ed.) Nichols, J.G. et al eds. Sinauer Associates, Inc. (1992). Assays that detect IP₃ accumulation can be utilized to determine if a candidate compound is, *e.g.,* an inverse agonist to a Gq- or Go-associated receptor (i.e., such a compound would decrease the levels of IP₃). Gq-associated receptors can also been examined using an AP1 reporter assay in that Gq-dependent phospholipase C causes activation of genes containing AP1 elements; thus, activated Gq-associated receptors will evidence an increase in the expression of such genes, whereby inverse agonists thereto will evidence a decrease in such expression, and agonists will evidence an increase in such expression. Commercially available assays for such detection are available.

### 3. GPCR Fusion Protein

The use of an endogenous, constitutively activated orphan GPCR, or a non-endogenous, constitutively activated orphan GPCR, for screening of candidate compounds for the direct identification of inverse agonists, agonists and partial agonists provides a unique challenge in that, by definition, the receptor is active even in the absence of an endogenous ligand bound thereto. Thus, it is often useful that an approach be utilized that can enhance the signal obtained by the activated receptor. A preferred approach is the use of a GPCR Fusion Protein.

Generally, once it is determined that a GPCR is or has been constitutively activated, using the assay techniques set forth above (as well as others), it is possible to determine the predominant G protein that couples with the endogenous GPCR. Coupling of the G protein to the GPCR provides a signaling pathway that can be assessed. Because it is most preferred that screening take place by use of a mammalian expression system, such a system will be expected to have endogenous G protein therein. Thus, by definition, in such a system, the constitutively activated orphan GPCR will continuously signal. In this regard, it is preferred that this signal be enhanced such that in the presence of, e.g., an inverse agonist to the receptor, it is more likely that it will be able to more readily differentiate, particularly in the context of screening, between the receptor when it is contacted with the inverse agonist.

The GPCR Fusion Protein is intended to enhance the efficacy of G protein coupling with the GPCR. The GPCR Fusion Protein is preferred for screening with a non-endogenous, constitutively activated GPCR because such an approach increases the signal that is most preferably utilized in such screening techniques, although the GPCR Fusion Protein can also be (and preferably is) used with an endogenous, constitutively activated GPCR. This is important in facilitating a significant "signal to noise" ratio;
such a significant ratio is import preferred for the screening of candidate compounds as disclosed herein.

The construction of a construct useful for expression of a GPCR Fusion Protein is within the purview of those having ordinary skill in the art. Commercially available expression vectors and systems offer a variety of approaches that can fit the particular needs of an investigator. The criteria of importance for such a GPCR Fusion Protein construct is that the GPCR sequence and the G protein sequence both be in-frame (preferably, the sequence for the GPCR is upstream of the G protein sequence) and that the "stop" codon of the GPCR must be deleted or replaced such that upon expression of the GPCR, the G protein can also be expressed. The GPCR can be linked directly to the G protein, or there can be spacer residues between the two (preferably, no more than about 12, although this number can be readily ascertained by one of ordinary skill in the art). We have a preference (based upon convenience) of use of a spacer in that some restriction sites that are not used will, effectively, upon expression, become a spacer. Most preferably, the G protein that couples to the GPCR will have been identified prior to the creation of the GPCR Fusion Protein construct. Because there are only a few G proteins that have been identified, it is preferred that a construct comprising the sequence of the G protein (i.e., a universal G protein construct) be available for insertion of an endogenous GPCR sequence therein; this provides for efficiency in the context of large-scale screening of a variety of different endogenous GPCRs having different sequences.

### E. Other Utility

Although a preferred use of the human orphan GPCRs disclosed herein may be for the direct identification of candidate compounds as inverse agonists, agonists or partial agonists (preferably for use as pharmaceutical agents), these versions of human GPCRs can also be utilized in research settings. For example, in vitro and *in vivo* systems incorporating GPCRs can be utilized to further elucidate and understand the roles these receptors play in the human condition, both normal and diseased, as well as understanding the role of constitutive activation as it applies to understanding the signaling cascade. The value in human orphan GPCRs is that its utility as a research tool is enhanced in that by determining the location(s) of such receptors within the body, the GPCRs can be used to understand the role of these receptors in the human body before the endogenous ligand therefor is identified. Other uses of the disclosed receptors will become apparent to those in the art based upon, inter *alia,* a review of this patent document.

### EXAMPLES

The following examples are presented for purposes of elucidation, and not limitation, of the present invention. While specific nucleic acid and amino acid sequences are disclosed herein, those of ordinary skill in the art are credited with the ability to make minor modifications to these sequences while achieving the same or substantially similar results reported below. Unless otherwise indicated below, all nucleic acid sequences for the disclosed endogenous orphan human GPCRs have been sequenced and verified. For purposes of equivalent receptors, those of ordinary skill in the art will readily appreciate that conservative substitutions can be made to the disclosed sequences to obtain a functionally equivalent receptor.

### Example 1

### ENDOGENOUS HUMAN GPCRS

### 1. Identification of Human GPCRs

Several of the disclosed endogenous human GPCRs were identified based upon a review of the GenBank database information. While searching the database, the following cDNA clones were identified as evidenced below.

| **Disclos ed Human Orphan GPCRs** | **Accession Number** | **Complete DNA Sequence (Base Pairs)** | **Open Reading Frame (Base Pairs)** | **Nucleic Acid SEQ.ID. NO.** | **Amino Acid SEQ.ID .NO.** |
|---|---|---|---|---|---|
| **hARE-3** | AL033379 | 111,389 bp | 1,260 bp | 1 | 2 |
| **hARE-4** | AC006087 | 226,925 bp | 1,119 bp | 3 | 4 |
| **hARE-5** | AC006255 | 127,605 bp | 1,104 bp | 5 | 6 |
| **hRUP3** | AL035423 | 140,094 bp | 1,005 bp | 7 | 8 |
| **hRUPS** | AC005849 | 169,144 bp | 1,413 bp | 9 | 10 |
| **hRUP6** | AC005871 | 218,807 bp | 1,245 bp | 11 | 12 |
| **hRUP7** | AC007922 | 158,858 bp | 1,173 bp | 13 | 14 |

Other disclosed endogenous human GPCRs were identified by conducting a BLAST search of EST database (dbest) using the following EST clones as query sequences. The following EST clones identified were then used as a probe to screen a human genomic library.

| **Disclosed Human Orphan GPCRs** | **Query (Sequence)** | **EST Clone/ Accession No. Identified** | **Open Reading Frame (Base Pairs)** | **Nucleic Acid SEQ.ID.NO.** | **Amino Acid SEQ.ID.NO.** |
|---|---|---|---|---|---|
| **hGPCR27** | Mouse GPCR27 | AA775870 | 1,125 bp | 15 | 16 |
| **hARE-1** | TDAG | 1689643 AI090920 | 999 bp | 17 | 18 |
| **hARE-2** | GPCR27 | 68530 AA359504 | 1,122 bp | 19 | 20 |
| **hPPR1** | Bovine PPR1 | 238667 H67224 | 1,053 bp | 21 | 22 |
| **hG2A** | Mouse 1179426 | *See Example 2(a), below* | 1,113 bp | 23 | 24 |
| **hCHN3** | N.A. | EST 36581 (full length) | 1,113 bp | 25 | 26 |
| **hCHN4** | TDAG | 1184934 AA804531 | 1,077 bp | 27 | 28 |
| **hCHN6** | N.A. | EST 2134670 (full length) | 1,503 bp | 29 | 30 |
| **hCHN8** | KIAA0001 | EST 764455 | 1,029 bp | 31 | 32 |
| **hCHN 9** | 1365839 | EST 1541536 | 1,077 bp | 33 | 34 |
| **hCHN10** | Mouse EST 1365839 | Human 1365839 | 1,005 bp | 35 | 36 |
| **hRUP4** | N.A. | AI307658 | 1,296 bp | 37 | 38 |

| | | | | | |
|---|---|---|---|---|---|
| *N.A. - = "not applicable".* | | | | | |

### 2. Full Length Cloning

### a. hG2A (Seq. Id. Nos. 23 & 24)

Mouse EST clone 1179426 was used to obtain a human genomic clone containing all but three amino acid hG2A coding sequences. The 5'end of this coding sequence was obtained by using 5 'RACE™, and the template for PCR was Clontech's Human Spleen Marathon-ready™ cDNA. The disclosed human G2A was amplified by PCR using the G2A cDNA specific primers for the first and second round PCR as shown in SEQ.ID.NO.: 39 and SEQ.ID.NO.:40 as follows:
5'-CTGTGTACAGCAGTTCGCAGAGTG-3' (SEQ.ID.NO.: 39; 1^{st} round PCR)
5'-GAGTGCCAGGCAGAGCAGGTAGAC-3' (SEQ.ID.NO.: 40; second round PCR).
   PCR was performed using Advantage™ GC Polymerase Kit (Clontech; manufacturing instructions will be followed), at 94°C for 30 sec followed by 5 cycles of 94°C for 5 sec and 72°C for 4 min; and 30 cycles of 94° for 5 sec and 70° for 4 min. An approximate 1.3 Kb PCR fragment was purified from agarose gel, digested with Hind III and Xba I and cloned into the expression vector pRC/CMV2 (Invitrogen). The cloned-insert was sequenced using the T7 Sequenase™ kit (USB Amersham; manufacturer instructions will be followed) and the sequence was compared with the presented sequence. Expression of the human G2A will be detected by probing an RNA dot blot (Clontech; manufacturer instructions will be followed) with the P³²-labeled fragment.

### b. hCHN9 (Seq. Id. Nos. 33 & 34)

Sequencing of the EST clone 1541536 indicated that hCHN9 is a partial cDNA clone having only an initiation codon; i.e., the termination codon was missing. When hCHN9 was used to "blast" against the data base (nr), the 3' sequence of hCHN9 was 100% homologous to the 5' untranslated region of the leukotriene B4 receptor cDNA, which contained a termination codon in the frame with hCHN9 coding sequence. To determine whether the 5' untranslated region of LTB4R cDNA was the 3' sequence of hCHN9, PCR was performed using primers based upon the 5' sequence flanking the initiation codon found in hCHN9 and the 3' sequence around the termination codon found in the LTB4R 5' untranslated region. The 5' primer sequence utilized was as follows:
5'-CCCGAATTCCTGCTTGCTCCCAGCTTGGCCC-3' (SEQ.ID.NO.: 41; sense) and
5'-TGTGGATCCTGCTGTCAAAGGTCCCATTCCGG-3' (SEQ.ID.NO.: 42; antisense).
   PCR was performed using thymus cDNA as a template and rTth polymerase (Perkin Elmer) with the buffer system provided by the manufacturer, 0.25 uM of each primer, and 0.2 mM of each 4 nucleotides. The cycle condition was 30 cycles of 94°C for 1 min, 65°C for 1min and 72 °C for 1 min and 10 sec. A 1.1kb fragment consistent with the predicted size was obtained from PCR. This PCR fragment was subcloned into pCMV (see below) and sequenced (see, SEQ.ID.NO.: 33).

### c. hRUP 4 (Seq. Id. Nos. 37 & 38)

The full length hRUP4 was cloned by RT-PCR with human brain cDNA (Clontech) as templates:
5'-TCACAATGCTAGGTGTGGTC-3' (SEQ.ID.NO.: 43; sense) and
5'-TGCATAGACAATGGGATTACAG-3' (SEQ.ID.NO.: 44; antisense).
   PCR was performed using TaqPlUS™ Precision™ polymerase (Stratagene; manufacturing instructions will be followed) by the following cycles: 94°C for 2 min; 94°C 30 sec; 55°C for 30 sec, 72°C for 45 sec, and 72°C for 10 min. Cycles 2 through 4 were repeated 30 times.

The PCR products were separated on a 1% agarose gel and a 500 bp PCR fragment was isolated and cloned into the pCRII-TOPO vector (Invitrogen) and sequenced using the T7 DNA Sequenase™ kit (Amsham) and the SP6/T7 primers (Stratagene). Sequence analysis revealed that the PCR fragment was indeed an alternatively spliced form of AI307658 having a continuous open reading frame with similarity to other GPCRs. The completed sequence of this PCR fragment was as follows:

5′-TCACAATGCTAGGTGTGGTCTGGCTGGTGGCAGTCATCGTAGGATCACCCATGTGGCAC
GTGCAACAACTTGAGATCAAATATGACTTCCTATATGAAAAGGAACACATCTGCTGCTTAGAAGAGTGG
ACCAGCCCTGTGCACCAGAAGATCTACACCACCTTCATCCTTGTCATCCTCTTCCTCCTGCCTCTTATG
GTGATGCTTATTCTGTACGTAAAATTGGTTATGAACTTTGGATAAAGAAAAGAGTTGGGGATGGTTCAG
TGCTTCGAACTATTCATGGAAAAGAAATGTCCAAAATAGCCAGGAAGAAGAAACGAGCTGTCATTATGA
TGGTGACAGTGGTGGCTCTCTTTGCTGTGTGCTGGGCACCATTCCATGTTGTCCATATGATGATTGAAT

ACAGTAATTTTGAAAAGGAATATGATGATGTCACAATCAAGATGATTTTTGCTATCGTGCAAATTATTG
GATTTTCCAACTCCATCTGTAATCCCATTGTCTATGCA-3′ (SEQ.ID.NO.: 45)

Based on the above sequence, two sense oligonucleotide primer sets:
5'-CTGCTTAGAAGAGTGGACCAG-3' (SEQ.ID.NO.: 46; oligo 1),
5'-CTGTGCACCAGAAGATCTACAC-3' (SEQ.ID.NO.: 47; oligo 2)
   and two antisense oligonucleotide primer sets:
5'-CAAGGATGAAGGTGGTGTAGA-3' (SEQ.ID.NO.: 48; oligo 3)
5'-GTGTAGATCTTCTGGTGCACAGG-3' (SEQ.ID.NO.: 49; oligo 4)
   were used for 3'- and 5'-race PCR with a human brain Mara thon- Ready™ cDNA (Clontech, Cat# 7400-1) as template, according to manufacture's instructions. DNA fragments generated by the RACE PCR were cloned into the pCRII-TOPO™ vector (Invitrogen) and sequenced using the SP6/T7 primers (Stratagene) and some internal primers. The 3' RACE product contained a poly(A) tail and a completed open reading frame ending at a TAA stop codon. The 5' RACE product contained an incomplete 5' end; i.e., the ATG initiation codon was not present.

Based on the new 5' sequence, oligo 3 and the following primer:
5'-GCAATGCAGGTCATAGTGAGC -3' (SEQ.ID.NO.: 50; oligo 5)
   were used for the second round of 5' RACE PCR and the PCR products were analyzed as above. A third round of 5' RACE PCR was carried out utilizing antisense primers: 5'-TGGAGCATGGTGACGGGAATGCAGAAG-3' (SEQ.ID.NO.: 51; oligo 6) and 5'-GTGATGAGCAGGTCACTGAGCGCCAAG-3' (SEQ.ID.NO.: 52; oligo7).

The sequence of the 5' RACE PCR products revealed the presence of the initiation codon ATG, and further round of 5' RACE PCR did not generate any more 5' sequence. The completed 5' sequence was confirmed by RT-PCR using sense primer 5'-GCAATGCAGGCGCTTAACATTAC-3' (SEQ.ID.NO.: 53; oligo 8) and oligo 4 as primers and sequence analysis of the 650 bp PCR product generated from human brain and heart cDNA templates (Clontech, Cat# 7404-1). The completed 3' sequence was confirmed by RT-PCR using oligo 2 and the following antisense primer:
5'-TTGGGTTACAATCTGAAGGGCA-3' (SEQ.ID.NO.: 54; oligo 9)
   and sequence analysis of the 670 bp PCR product generated from human brain and heart cDNA templates. (Clontech, Cat# 7404-1).

### d. hRUP5 (Seq. Id. Nos. 9 & 10)

The full length hRUP5 was cloned by RT-PCR using a sense primer upstream from ATG, the initiation codon (SEQ.ID.NO.: 55), and an antisense primer containing TCA as the stop codon (SEQ.ID.NO.: 56), which had the following sequences:
5'-ACTCCGTGTCCAGCAGGACTCTG-3' (SEQ.ID.NO.:55)
5'-TGCGTGTTCCTGGACCCTCACGTG-3' (SEQ.ID.NO.: 56)
   and human peripheral leukocyte cDNA (Clontech) as a template. Advantage cDNA polymerase (Clontech) was used for the amplification in a 50ul reaction by the following cycle with step 2 through step 4 repeated 30 times: 94°C for 30 sec; 94° for 15 sec; 69° for 40 sec; 72°C for 3 min; and 72°C fro 6 min. A 1.4kb PCR fragment was isolated and cloned with the pCRII-TOPO™ vector (Invitrogen) and completely sequenced using the T7 DNA Sequenase™ kit (Amsham). See, SEQ.ID.NO.: 9.

### e. hRUP6 (Seq. Id. Nos. 11 & 12)

The full length hRUP6 was cloned by RT-PCR using primers:
5'-CAGGCCTTGGATTTTAATGTCAGGGATGG-3' (SEQ.ID.NO.: 57) and
5'-GGAGAGTCAGCTCTGAAAGAATTCAGG-3' (SEQ.ID.NO.: 58);
   and human thymus Marathon-Ready™ cDNA (Clontech) as a template. Advantage cDNA polymerase (Clontech, according to manufacturer's instructions) was used for the amplification in a 50ul reaction by the following cycle: 94°C for 30sec; 94°C for 5 sec; 66°C for 40sec; 72°C for 2.5 sec and 72°C for 7 min. Cycles 2 through 4 were repeated 30 times. A 1.3 Kb PCR fragment was isolated and cloned into the pCRII-TOPO™ vector (Invitrogen) and completely sequenced (see, SEQ.ID.NO.: 11) using the ABI Big Dye Terminator TM kit (P.E. Biosystem).

### f. hRUP7 (Seq. Id. Nos. 13 & 14)

The full length RUP7 was cloned by RT-PCR using primers:
5'-TGATGTGATGCCAGATACTAATAGCAC-3' (SEQ.ID.NO.: 59; sense) and
5'-CCTGATTCATTTAGGTGAGATTGAGAC-3' (SEQ.ID.NO.: 60; antisense)
   and human peripheral leukocyte cDNA (Clontech) as a template. Advantage™ cDNA polymerase (Clontech) was used for the amplification in a 50 ul reaction by the following cycle with step 2 to step 4 repeated 30 times: 94°C for 2 minutes; 94°C for 15 seconds; 60°C for 20 seconds; 72°C for 2 minutes; 72°C for 10 minutes. A 1.25 Kb PCR fragment was isolated and cloned into the pCRII-TOPO™ vector (Invitrogen) and completely sequenced using the ABI Big Dye Terminator™ kit (P.E. Biosystem). *See,* SEQ.ID.NO.: 13.

### g. hARE-5 (Seq. Id. Nos. 5 & 6)

The full length hARE-5 was cloned by PCR using the hARE5 specific primers 5'-CAGCGCAGGGTGAAGCCTGAGAGC-3' SEQ.ID.NO.: 69 (sense, 5' of initiation codon ATG) and 5'-GGCACCTGCTGTGACCTGTGCAGG-3' SEQ.ID.NO.: 70 (antisense, 3' of stop codon TGA) and human genomic DNA as template. TaqPlus Precision™ DNA polymerase (Stratagene) was used for the amplification by the following cycle with step 2 to step 4 repeated 35 times: 96°C, 2 minutes; 96°C, 20 seconds; 58°C, 30 seconds; 72°C, 2 minutes; and 72°C, 10 minutes A 1.1 Kb PCR fragment of predicated size was isolated and cloned into the pCRII-TOPO™ vector (Invitrogen) and completely sequenced (SEQ.ID.NO.:5) using the T7 DNA Sequenase™ kit (Amsham).

### h. hARE-4 (Seq. Id. Nos.: 3 & 4)

The full length hARE-4 was cloned by PCR using the hARE-4 specific primers 5'-CTGGTGTGCTCCATGGCATCCC-3' SEQ.ID.NO.:67 (sense, 5' of initiation codon ATG) and 5'-GTAAGCCTCCCAGAACGAGAGG-3' SEQ.ID.NO.: 68 (antisense, 3' of stop codon TGA) and human genomic DNA as template. Taq DNA polymerase (Stratagene) and 5% DMSO was used for the amplification by the following cycle with step 2 to step 3 repeated 35 times: 94°C, 3 minutes; 94°C, 30 seconds; 59°C, 2 minutes; 72°C, 10 minutes

A 1.12 Kb PCR fragment of predicated size was isolated and cloned into the pCRII-TOPO™ vector (Invitrogen) and completely sequenced (SEQ.ID.NO.:3) using the T7 DNA Sequenase™ kit (Amsham).

### i. hARE-3 (Seq.Id.Nos.: 1 & 2)

The full length hARE-3 was cloned by PCR using the hARE-3 specific primers 5'-gatcaagcttCCATCCTACTGAAACC**ATG**GTC-3' SEQ.ID.NO.:65 (sense, lower case nucleotides represent Hind III overhang, **ATG** as initiation codon) and 5'-gatcagatctCAGTTCCAATAT**TCA**CACCACCGTC-3' SEQ.ID.NO.:66 (antisense, lower case nucleotides represent Xba I overhang, **TCA** as stop codon) and human genomic DNA as template. TaqPlus Precision™ DNA polymerase (Stratagene) was used for the amplification by the following cycle with step 2 to step 4 repeated 35 times: 94°C, 3 minutes; 94°C, 1 minute; 55°C, 1 minute; 72°C, 2 minutes; 72°C, 10 minutes.

A 1.3 Kb PCR fragment of predicated size was isolated and digested with Hind III and Xba I, cloned into the pRC/CMV2 vector (Invitrogen) at the Hind III and Xba I sites and completely sequenced (SEQ.ID.NO.:1) using the T7 DNA Sequenase kit (Amsham).

### j. hRUP3 (Seq. Id. Nos.:7 & 8)

The full length hRUP3 was cloned by PCR using the hRUP3 specific primers 5'-GTCCTGCCACTTCGAGAC**ATG**G-3' SEQ.ID.NO.:71 (sense, **ATG** as initiation codon) and 5'-GAAACTTCTCTGCCCTTACCGTC-3' SEQ.ID.NO.:72 (antisense), 3' of stop codon TAA) and human genomic DNA as template. TaqPlus Precision™ DNA polymerase (Stratagene) was used for the amplification by the following cycle with step 2 to step 4 repeated 35 times: 94°C, 3 minutes; 94°C, 1 minute; 58°C, 1 minute; 72°C, 2 minutes; 72°C, 10 minutes

A 1.0 Kb PCR fragment of predicated size was isolated and cloned into the pCRII-TOPO™ vector (Invitrogen) and completely sequenced (SEQ.ID.NO.: 7)using the T7 DNA sequenase kit (Amsham).

### Example 2

### RECEPTOR EXPRESSION

Although a variety of cells are available to the art for the expression of proteins, it is most preferred that mammalian cells be utilized. The primary reason for this is predicated upon practicalities, i.e., utilization of, e.g., yeast cells for the expression of a GPCR, while possible, introduces into the protocol a non-mammalian cell which may not (indeed, in the case of yeast, does not) include the receptor-coupling, genetic-mechanism and secretary pathways that have evolved for mammalian systems - thus, results obtained in non-mammalian cells, while of potential use, are not as preferred as that obtained from mammalian cells. Of the mammalian cells, COS-7, 293 and 293T cells are particularly preferred, although the specific mammalian cell utilized can be predicated upon the particular needs of the artisan. The general procedure for expression of the disclosed GPCRs is as follows.

On day one, 1X10⁷ 293T cells per 150mm plate were plated out. On day two, two reaction tubes will be prepared (the proportions to follow for each tube are per plate): tube A will be prepared by mixing 20µg DNA (e.g., pCMV vector; pCMV vector with receptor cDNA, etc.) in 1.2ml serum free DMEM (Irvine Scientific, Irvine, CA); tube B will be prepared by mixing 120µl lipofectamine (Gibco BRL) in 1.2ml serum free DMEM . Tubes A and B are admixed by inversions (several times), followed by incubation at room temperature for 30-45min. The admixture can be referred to as the "transfection mixture". Plated 293T cells are washed with 1XPBS, followed by addition of 10ml serum free DMEM. 2.4ml of the transfection mixture will then be added to the cells, followed by incubation for 4hrs at 37°C/5% CO₂. The transfection mixture was then be removed by aspiration, followed by the addition of 25ml of DMEM/10% Fetal Bovine Serum. Cells will then be incubated at 37°C/5% CO₂. After 72hr incubation, cells can then be harvested and utilized for analysis.

### Example 3

### TISSUE DISTRIBUTION OF THE DISCLOSED HUMAN GPCRS

Several approaches can be used for determination of the tissue distribution of the GPCRs disclosed herein.

### 1. Dot-Blot Analysis

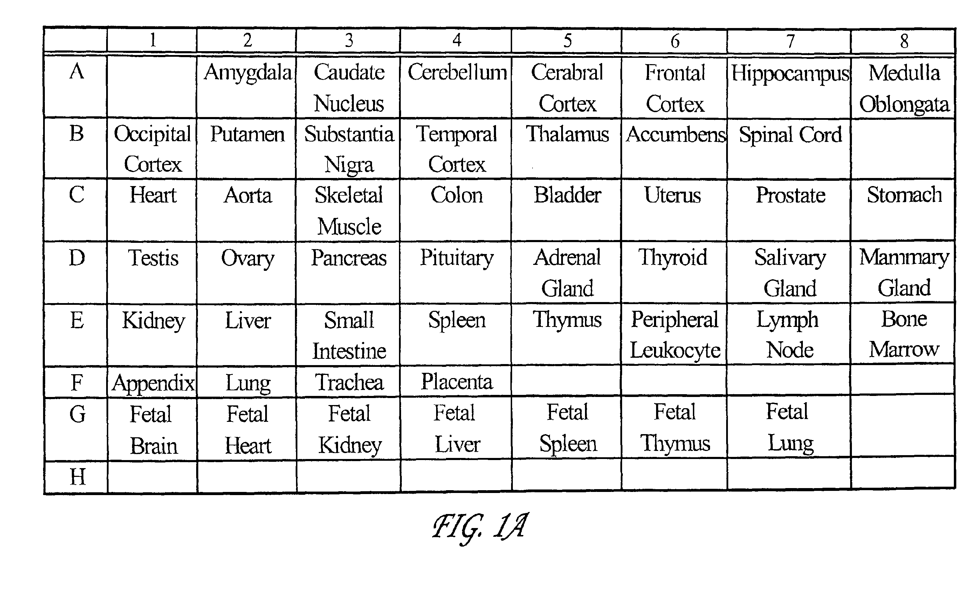

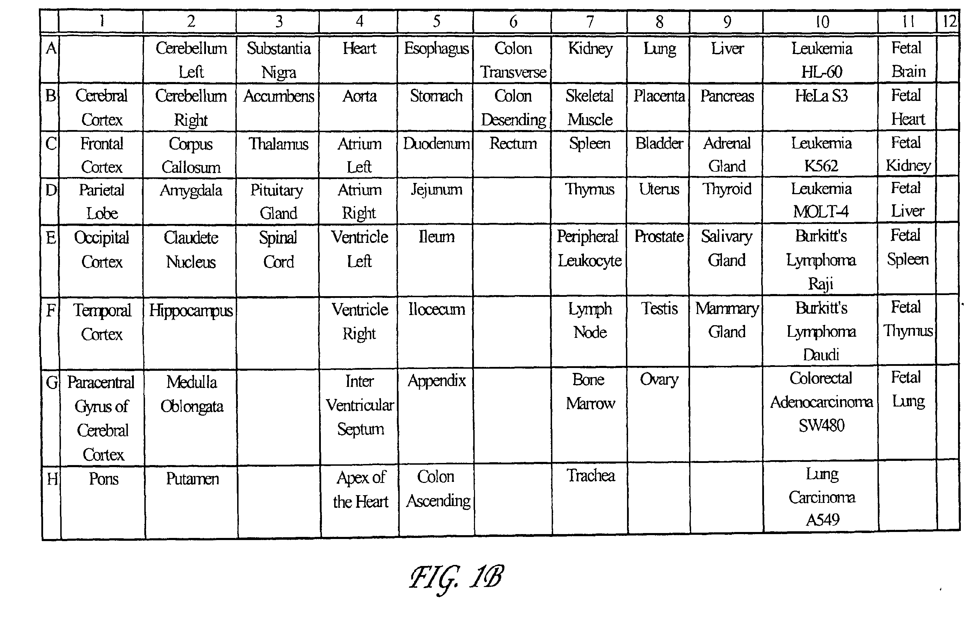

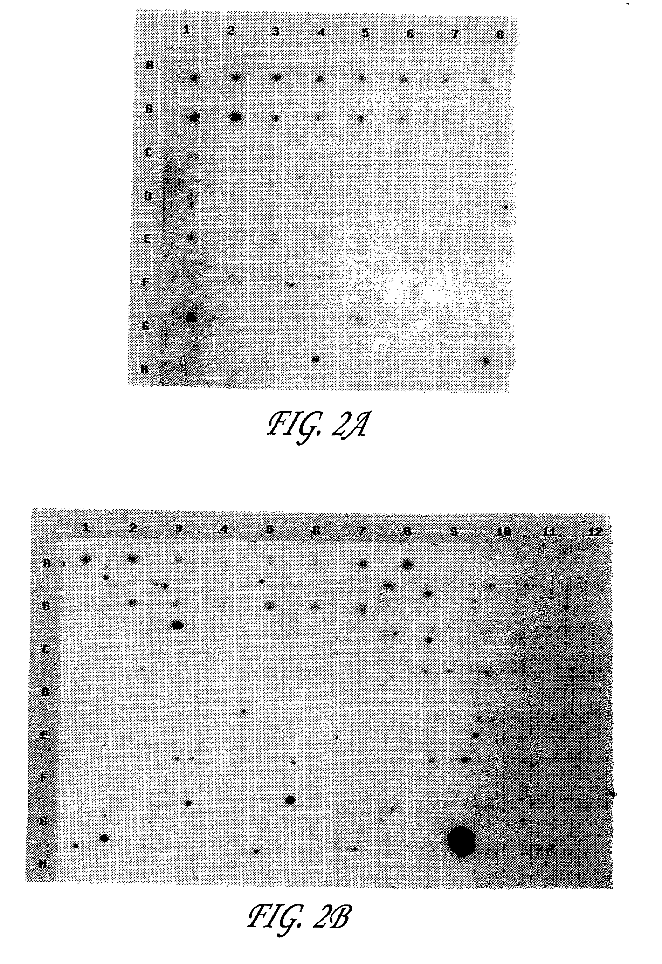

Using a commercially available human-tissue dot-blot format, endogenous orphan GPCRs were probed for a determination of the areas where such receptors are localized. cDNA fragments from the GPCRs of Example 1 (radiolabelled) were (or can be) used as the probe: radiolabeled probe was (or can be) generated using the complete receptor cDNA (excised from the vector) using a Prime-It II™ Random Primer Labeling Kit (Stratagene, #300385), according to manufacturer's instructions. A human RNA Master Blot™ (Clontech, #7770-1) was hybridized with the endogenous human GPCR radiolabeled probe and washed under stringent conditions according manufacturer's instructions. The blot was exposed to Kodak BioMax™ Autoradiography film overnight at -80°C. Results are summarized for several receptors in Table B and C *(see* Figures 1A and 1B for a grid identifying the various tissues and their locations, respectively). Exemplary dot-blots are provided in Figure 2A and 2B for results derived using hCHN3 and hCHN8, respectively.

**TABLE B**

| **ORPHAN GPCR** | **Tissue Distribution** **(highest levels, relative to other tissues in the dot-blot)** |
|---|---|
| hGPCR27 | Fetal brain, Putamen, Pituitary gland, Caudate nucleus |
| hARE-1 | Spleen, Peripheral leukocytes, Fetal spleen |
| hPPR1 | Pituitary gland, Heart, salivary gland, Small intestine, Testis |
| hRUP3 | Pancreas |
| hCHN3 | Fetal brain, Putamen, Occipital cortex |
| hCHN9 | Pancreas, Small intestine, Liver |
| hCHN10 | Kidney, Thryoid |

**TABLE C**

| **ORPHAN GPCR** | **Tissue Distribution** **(highest levels, relative to other tissues in the dot-blot)** |
|---|---|
| hARE-3 | Cerebellum left, Cerebellum right, Testis, Accumbens |
| hGPCR3 | Corpus collusum, Caudate nucleus, Liver, Heart, Inter-Ventricular Septum |
| hARE-2 | Cerebellum left, Cerebellum right, Substantia |
| hCHN8 | Cerebellum left, Cerebellum right, Kidney, Lung |

### 2. RT-PCR

### a. hRUP3

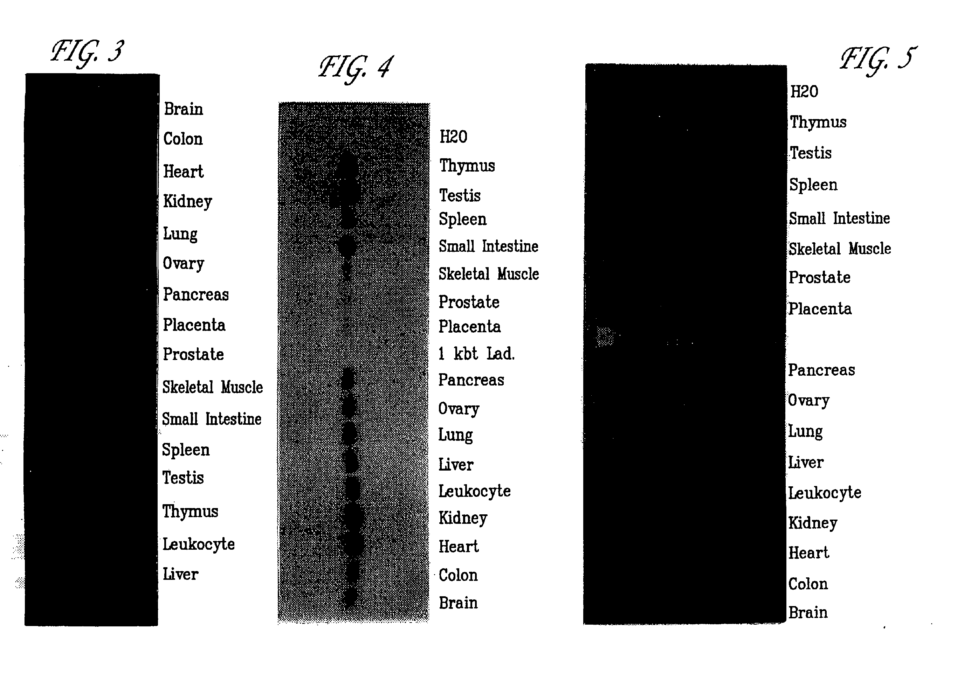

To ascertain the tissue distribution of hRUP3 mRNA, RT-PCR was performed using hRUP3-specific primers and human multiple tissue cDNA panels (MTC, Clontech) as templates. Taq DNA polymerase (Stratagene) was utilized for the PCR reaction, using the following reaction cycles in a 40ul reaction: 94°C for 2 min; 94°C for 15 sec; 55°C for 30 sec; 72°C for 1 min; 72° C, for 10 min. Primers were as follows:
5'-GACAGGTACCTTGCCATCAAG-3' (SEQ.ID.NO.: 61; sense)
5'-CTGCACAATGCCAGTGATAAGG-3' (SEQ.ID.NO.: 62; antisense).
   20ul of the reaction was loaded onto a 1% agarose gel; results are set forth in Figure 3.

As is supported by the data of Figure 3, of the 16 human tissues in the cDNA panel utilized (brain, colon, heart, kidney, lung, ovary, pancreas, placenta, prostate, skeleton, small intestine, spleen, testis, thymus leukocyte, and liver) a single hRUP3 band is evident only from the pancreas. Additional comparative analysis of the protein sequence of hRUP3 with other GPCRs suggest that hRUP3 is related to GPCRs having small molecule endogenous ligand such that it is predicted that the endogenous ligand for hRUP3 is a small molecule.

### b. hRUP4

RT-PCR was performed using hRUP4 oligo's 8 and 4 as primers and the human multiple tissue cDNA panels (MTC, Clontech) as templates. Taq DNA polymerase (Stratagene) was used for the amplification in a 40ul reaction by the following cycles: 94°C for 30 seconds, 94°C for 10 seconds, 55°C for 30 seconds, 72°C for 2 minutes, and 72°C for 5 minutes with cycles 2 through 4 repeated 30 times.

20 µl of the reaction were loaded on a 1% agarose gel to analyze the RT-PCR products, and hRUP4 mRNA was found expressed in many human tissues, with the strongest expression in heart and kidney. (see, Figure 4). To confirm the authenticity of the PCR fragments, a 300 bp fragment derived from the 5' end of hRUP4 was used as a probe for the Southern Blot analysis. The probe was labeled with ³²P-dCTP using the Prime-It II™ Random Primer Labeling Kit (Stratagene) and purified using the ProbeQuant™ G-50 micro columns (Amersham). Hybridization was done overnight at 42° C following a 12 hr pre-hybridization. The blot was finally washed at 65°C with 0.1 x SSC. The Southern blot did confirm the PCR fragments as hRUP4.

### c . hRUP 5

RT-PCR was performed using the following hRUP5 specific primers:
5'-CTGACTTCTTGTTCCTGGCAGCAGCGG-3' (SEQ.ID.NO.: 63; sense)
5'-AGACCAGCCAGGGCACGCTGAAGAGTG-3' (SEQ.ID.NO.: 64; antisense)
   and the human multiple tissue cDNA panels (MTC, Clontech) as templates. Taq DNA polymerase (Stratagene) was used for the amplification in a 40ul reaction by the following cycles: 94°C for 30 sec, 94°C for 10 sec, 62°C for 1.5 min, 72°C for 5 min, and with cycles 2 through 3 repeated 30 times. 20 µl of the reaction were loaded on a 1.5% agarose gel to analyze the RT-PCR products, and hRUP5 mRNA was found expressed only in the peripheral blood leukocytes *(data not shown).*

### d. hRUP 6

RT-PCR was applied to confirm the expression and to determine the tissue distribution of hRUP6. Oligonucleotides used, based on an alignment of AC005871 and GPR66 segments, had the following sequences:
5'-CCAACACCAGCATCCATGGCATCAAG-3' (SEQ.ID.NO.: 73; sense),
5'-GGAGAGTCAGCTCTGAAAGAATTCAGG-3' (SEQ.ID.NO.: 74; antisense)
   and the human multiple tissue cDNA panels (MTC, Clontech) were used as templates.
   PCR was performed using TaqPlus Precision™ polymerase (Stratagene; manufacturing instructions will be followed) in a 40ul reaction by the following cycles: 94°C for 30 sec; 94°C 5 sec; 66°C for 40 sec, 72°C for 2.5 min, and 72°C for 7 min. Cycles 2 through 4 were repeated 30 times. 20 ul of the reaction were loaded on a 1.2% agarose gel to analyze the RT-PCR products, and a specific 760bp DNA fragment representing hRUP6 was expressed predominantly in the thymus and with less expression in the heart, kidney, lung, prostate small intestine and testis. *(see,* Figure 5).

It is intended that each of the patents, applications, and printed publications mentioned in this patent document be hereby incorporated by reference in their entirety.

As those skilled in the art will appreciate, numerous changes and modifications may be made to the preferred embodiments of the invention without departing from the spirit of the invention. It is intended that all such variations fall within the scope of the invention and the claims that follow.

Although a variety of Vectors are available to those in the art, for purposes of utilization for both endogenous and non-endogenous human GPCRs, it is most preferred that the Vector utilized be pCMV. This vector was deposited with the American Type Culture Collection (ATCC) on October 13, 1998 (10801 University Blvd., Manassas, VA 20110-2209 USA) under the provisions of the Budapest Treaty for the International Recognition of the Deposit of Microorganisms for the Purpose of Patent Procedure. The DNA was tested by the ATCC and determined to be. The ATCC has assigned the following deposit number to pCMV: ATCC #203351.

SEQUENCE LISTING

(1) GENERAL INFORMATION:

(i) APPLICANT: Chen, Ruoping
Dang, Huong T.
Liaw, Chen W.
Lin, I-Lin

(ii) TITLE OF INVENTION: Human Orphan G Protein-Coupled Receptors

(iii) NUMBER OF SEQUENCES: 74

(iv) CORRESPONDENCE ADDRESS:
(A) ADDRESSEE: Arena Pharmaceuticals, Inc.
(B) STREET: 6166 Nancy Ridge Drive
(C) CITY: San Diego
(D) STATE: CA
(E) COUNTRY: USA
(F) ZIP: 92121

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(vi) CURRENT APPLICATION DATA:
(A) APPLICATION NUMBER: US
(B) FILING DATE:
(C) CLASSIFICATION:

(viii) ATTORNEY/AGENT INFORMATION:
(A) NAME: Burgoon, Richard P.
(B) REGISTRATION NUMBER: 34,787

(ix) TELECOMMUNICATION INFORMATION:
(A) TELEPHONE: (858)453-7200
(B) TELEFAX: (858)453-7210

(2) INFORMATION FOR SEQ ID NO:1:

(i) SEQUENCE CHARACTERISTICS:
(A) LENGTH: 1260 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:1:

ATGGTCTTCT CGGCAGTGTT GACTGCGTTC CATACCGGGA CATCCAACAC AACATTTGTC 60

GTGTATGAAA ACACCTACAT GAATATTACA CTCCCTCCAC CATTCCAGCA TCCTGACCTC 120

AGTCCATTGC TTAGATATAG TTTTGAAACC ATGGCTCCCA CTGGTTTGAG TTCCTTGACC 180

GTGAATAGTA CAGCTGTGCC CACAACACCA GCAGCATTTA AGAGCCTAAA CTTGCCTCTT 240

CAGATCACCC TTTCTGCTAT AATGATATTC ATTCTGTTTG TGTCTTTTCT TGGGAACTTG 300

GTTGTTTGCC TCATGGTTTA CCAAAAAGCT GCCATGAGGT CTGCAATTAA CATCCTCCTT 360

GCCAGCCTAG CTTTTGCAGA CATGTTGCTT GCAGTGCTGA ACATGCCCTT TGCCCTGGTA 420

ACTATTCTTA CTACCCGATG GATTTTTGGG AAATTCTTCT GTAGGGTATC TGCTATGTTT 480

TTCTGGTTAT TTGTGATAGA AGGAGTAGCC ATCCTGCTCA TCATTAGCAT AGATAGGTTC 540

CTTATTATAG TCCAGAGGCA GGATAAGCTA AACCCATATA GAGCTAAGGT TCTGATTGCA 600

GTTTCTTGGG CAACTTCCTT TTGTGTAGCT TTTCCTTTAG CCGTAGGAAA CCCCGACCTG 660

CAGATACCTT CCCGAGCTCC CCAGTGTGTG TTTGGGTACA CAACCAATCC AGGCTACCAG 720

GCTTATGTGA TTTTGATTTC TCTCATTTCT TTCTTCATAC CCTTCCTGGT AATACTGTAC 780

TCATTTATGG GCATACTCAA CACCCTTCGG CACAATGCCT TGAGGATCCA TAGCTACCCT 840

GAAGGTATAT GCCTCAGCCA GGCCAGCAAA CTGGGTCTCA TGAGTCTGCA GAGACCTTTC 900

CAGATGAGCA TTGACATGGG CTTTAAAACA CGTGCCTTCA CCACTATTTT GATTCTCTTT 960

GCTGTCTTCA TTGTCTGCTG GGCCCCATTC ACCACTTACA GCCTTGTGGC
AACATTCAGT1020

AAGCACTTTT ACTATCAGCA CAACTTTTTT GAGATTAGCA CCTGGCTACT GTGGCTCTGC1080

TACCTCAAGT CTGCATTGAA TCCGCTGATC TACTACTGGA GGATTAAGAA ATTCCATGAT1140

GCTTGCCTGG ACATGATGCC TAAGTCCTTC AAGTTTTTGC CGCAGCTCCC TGGTCACACA1200

AAGCGACGGA TACGTCCTAG TGCTGTCTAT GTGTGTGGGG AACATCGGAC GGTGGTGTGA1260

(3) INFORMATION FOR SEQ ID NO:2:

(i) SEQUENCE CHARACTERISTICS:
(A) LENGTH: 419 amino acids
(B) TYPE: amino acid
(C) STRANDEDNESS: single
(D) TOPOLOGY: linear

(ii) MOLECULE TYPE: DNA (genomic)

(xi) SEQUENCE DESCRIPTION: SEQ ID NO:2:

Met Val Phe Ser Ala Val Leu Thr Ala Phe His Thr Gly Thr Ser Asn
1               5                   10                  15

Thr Thr Phe Val Val Tyr Glu Asn Thr Tyr Met Asn Ile Thr Leu Pro
            20                  25                  30

Pro Pro Phe Gln His Pro Asp Leu Ser Pro Leu Leu Arg Tyr Ser Phe
        35                  40                  45

Glu Thr Met Ala Pro Thr Gly Leu Ser Ser Leu Thr Val Asn Ser Thr
    50                  55                  60

Ala Val Pro Thr Thr Pro Ala Ala Phe Lys Ser Leu Asn Leu Pro Leu
65                  70                  75                  80

Gln Ile Thr Leu Ser Ala Ile Met Ile Phe Ile Leu Phe Val Ser Phe
                85                  90                  95

Leu Gly Asn Leu Val Val Cys Leu Met Val Tyr Gln Lys Ala Ala Met
            100                 105                 110

Arg Ser Ala Ile Asn Ile Leu Leu Ala Ser Leu Ala Phe Ala Asp Met
        115                 120                 125

Leu Leu Ala Val Leu Asn Met Pro Phe Ala Leu Val Thr Ile Leu Thr
    130                 135                 140

Thr Arg Trp Ile Phe Gly Lys Phe Phe Cys Arg Val Ser Ala Met Phe
145                 150                 155                 160

Phe Trp Leu Phe Val Ile Glu Gly Val Ala Ile Leu Leu Ile Ile Ser
                165                 170                 175

Ile Asp Arg Phe Leu Ile Ile Val Gln Arg Gln Asp Lys Leu Asn Pro
            180                 185                 190

Tyr Arg Ala Lys Val Leu Ile Ala Val Ser Trp Ala Thr Ser Phe Cys
        195                 200                 205

Val Ala Phe Pro Leu Ala Val Gly Asn Pro Asp Leu Gln Ile Pro Ser
    210                 215                 220

Arg Ala Pro Gln Cys Val Phe Gly Tyr Thr Thr Asn Pro Gly Tyr Gln
225                 230                 235                 240

Ala Tyr Val Ile Leu Ile Ser Leu Ile Ser Phe Phe Ile Pro Phe Leu
                245                 250                 255

Val Ile Leu Tyr Ser Phe Met Gly Ile Leu Asn Thr Leu Arg His Asn
            260                 265                 270

Ala Leu Arg Ile His Ser Tyr Pro Glu Gly Ile Cys Leu Ser Gln Ala
        275                 280                 285

Ser Lys Leu Gly Leu Met Ser Leu Gln Arg Pro Phe Gln Met Ser Ile
    290                 295                 300

Asp Met Gly Phe Lys Thr Arg Ala Phe Thr Thr Ile Leu Ile Leu Phe

305                 310                 315                 320

Ala Val Phe Ile Val Cys Trp Ala Pro Phe Thr Thr Tyr Ser Leu Val
                325                 330                 335

Ala Thr Phe Ser Lys His Phe Tyr Tyr Gln His Asn Phe Phe Glu Ile
            340                 345                 350

Ser Thr Trp Leu Leu Trp Leu Cys Tyr Leu Lys Ser Ala Leu Asn Pro
        355                 360                 365

Leu Ile Tyr Tyr Trp Arg Ile Lys Lys Phe His Asp Ala Cys Leu Asp
    370                 375                 380

Met Met Pro Lys Ser Phe Lys Phe Leu Pro Gln Leu Pro Gly His Thr
385                 390                 395                 400

Lys Arg Arg Ile Arg Pro Ser Ala Val Tyr Val Cys Gly Glu His Arg
                405                 410                 415

Thr Val Val

(4) INFORMATION FOR SEQ ID NO:3:

(i) SEQUENCE CHARACTERISTICS:
(A) LENGTH: 1119 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:3:

ATGTTAGCCA ACAGCTCCTC AACCAACAGT TCTGTTCTCC CGTGTCCTGA CTACCGACCT 60

ACCCACCGCC TGCACTTGGT GGTCTACAGC TTGGTGCTGG CTGCCGGGCT CCCCCTCAAC 120

GCGCTAGCCC TCTGGGTCTT CCTGCGCGCG CTGCGCGTGC ACTCGGTGGT GAGCGTGTAC 180

ATGTGTAACC TGGCGGCCAG CGACCTGCTC TTCACCCTCT CGCTGCCCGT TCGTCTCTCC 240

TACTACGCAC TGCACCACTG GCCCTTCCCC GACCTCCTGT GCCAGACGAC GGGCGCCATC 300

TTCCAGATGA ACATGTACGG CAGCTGCATC TTCCTGATGC TCATCAACGT GGACCGCTAC 360

GCCGCCATCG TGCACCCGCT GCGACTGCGC CACCTGCGGC GGCCCCGCGT GGCGCGGCTG 420

CTCTGCCTGG GCGTGTGGGC GCTCATCCTG GTGTTTGCCG TGCCCGCCGC CCGCGTGCAC 480

AGGCCCTCGC GTTGCCGCTA CCGGGACCTC GAGGTGCGCC TATGCTTCGA GAGCTTCAGC 540

GACGAGCTGT GGAAAGGCAG GCTGCTGCCC CTCGTGCTGC TGGCCGAGGC GCTGGGCTTC 600

CTGCTGCCCC TGGCGGCGGT GGTCTACTCG TCGGGCCGAG TCTTCTGGAC GCTGGCGCGC 660

CCCGACGCCA CGCAGAGCCA GCGGCGGCGG AAGACCGTGC GCCTCCTGCT GGCTAACCTC 720

GTCATCTTCC TGCTGTGCTT CGTGCCCTAC AACAGCACGC TGGCGGTCTA CGGGCTGCTG 780

CGGAGCAAGC TGGTGGCGGC CAGCGTGCCT GCCCGCGATC GCGTGCGCGG GGTGCTGATG 840

5 GTGATGGTGC TGCTGGCCGG CGCCAACTGC GTGCTGGACC CGCTGGTGTA CTACTTTAGC 900

GCCGAGGGCT TCCGCAACAC CCTGCGCGGC CTGGGCACTC CGCACCGGGC CAGGACCTCG 960

GCCACCAACG GGACGCGGGC GGCGCTCGCG CAATCCGAAA GGTCCGCCGT CACCACCGAC1020

GCCACCAGGC CGGATGCCGC CAGTCAGGGG CTGCTCCGAC CCTCCGACTC CCACTCTCTG1080

TCTTCCTTCA CACAGTGTCC CCAGGATTCC GCCCTCTGA 1119

(5) INFORMATION FOR SEQ ID NO:4:

(i) SEQUENCE CHARACTERISTICS:
(A) LENGTH: 372 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:4:

(6) INFORMATION FOR SEQ ID NO:5:

    (i) SEQUENCE CHARACTERISTICS:
        (A) LENGTH: 1107 base pairs
        (B) TYPE: nucleic acid
        (C) STRANDEDNESS: single
        (D) TOPOLOGY: linear

(ii) MOLECULE TYPE: DNA (genomic)

(xi) SEQUENCE DESCRIPTION: SEQ ID NO:5:

ATGGCCAACT CCACAGGGCT GAACGCCTCA GAAGTCGCAG GCTCGTTGGG GTTGATCCTG 60

GCAGCTGTCG TGGAGGTGGG GGCACTGCTG GGCAACGGCG CGCTGCTGGT CGTGGTGCTG 120

CGCACGCCGG GACTGCGCGA CGCGCTCTAC CTGGCGCACC TGTGCGTCGT GGACCTGCTG 180

GCGGCCGCCT CCATCATGCC GCTGGGCCTG CTGGCCGCAC CGCCGCCCGG GCTGGGCCGC 240

GTGCGCCTGG GCCCCGCGCC ATGCCGCGCC GCTCGCTTCC TCTCCGCCGC TCTGCTGCCG 300

GCCTGCACGC TCGGGGTGGC CGCACTTGGC CTGGCACGCT ACCGCCTCAT CGTGCACCCG 360

CTGCGGCCAG GCTCGCGGCC GCCGCCTGTG CTCGTGCTCA CCGCCGTGTG GGCCGCGGCG 420

GGACTGCTGG GCGCGCTCTC CCTGCTCGGC CCGCCGCCCG CACCGCCCCC TGCTCCTGCT 480

CGCTGCTCGG TCCTGGCTGG GGGCCTCGGG CCCTTCCGGC CGCTCTGGGC CCTGCTGGCC 540

TTCGCGCTGC CCGCCCTCCT GCTGCTCGGC GCCTACGGCG GCATCTTCGT GGTGGCGCGT 600

CGCGCTGCCC TGAGGCCCCC ACGGCCGGCG CGCGGGTCCC GACTCCGCTC GGACTCTCTG 660

GATAGCCGCC TTTCCATCTT GCCGCCGCTC CGGCCTCGCC TGCCCGGGGG CAAGGCGGCC 720

CTGGCCCCAG CGCTGGCCGT GGGCCAATTT GCAGCCTGCT GGCTGCCTTA TGGCTGCGCG 780

TGCCTGGCGC CCGCAGCGCG GGCCGCGGAA GCCGAAGCGG CTGTCACCTG GGTCGCCTAC 840

TCGGCCTTCG CGGCTCACCC CTTCCTGTAC GGGCTGCTGC AGCGCCCCGT GCGCTTGGCA 900

CTGGGCCGCC TCTCTCGCCG TGCACTGCCT GGACCTGTGC GGGCCTGCAC TCCGCAAGCC 960

TGGCACCCGC GGGCACTCTT GCAATGCCTC CAGAGACCCC CAGAGGGCCC TGCCGTAGGC1020

CCTTCTGAGG CTCCAGAACA GACCCCCGAG TTGGCAGGAG GGCGGAGCCC CGCATACCAG1080

GGGCCACCTG AGAGTTCTCT CTCCTGA 1107

(7) INFORMATION FOR SEQ ID NO:6:

(i) SEQUENCE CHARACTERISTICS:
(A) LENGTH: 368 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:6:

Met Ala Asn Ser Thr Gly Leu Asn Ala Ser Glu Val Ala Gly Ser Leu
1               5                   10                  15

Gly Leu Ile Leu Ala Ala Val Val Glu Val Gly Ala Leu Leu Gly Asn
            20                  25                  30

Gly Ala Leu Leu Val Val Val Leu Arg Thr Pro Gly Leu Arg Asp Ala
        35                  40                  45

Leu Tyr Leu Ala His Leu Cys Val Val Asp Leu Leu Ala Ala Ala Ser
    50                  55                  60

Ile Met Pro Leu Gly Leu Leu Ala Ala Pro Pro Pro Gly Leu Gly Arg
65                  70                  75                  80

Val Arg Leu Gly Pro Ala Pro Cys Arg Ala Ala Arg Phe Leu Ser Ala
                85                  90                  95

Ala Leu Leu Pro Ala Cys Thr Leu Gly Val Ala Ala Leu Gly Leu Ala
            100                 105                 110

Arg Tyr Arg Leu Ile Val His Pro Leu Arg Pro Gly Ser Arg Pro Pro
        115                 120                 125

Pro Val Leu Val Leu Thr Ala Val Trp Ala Ala Ala Gly Leu Leu Gly
    130                 135                 140

Ala Leu Ser Leu Leu Gly Pro Pro Pro Ala Pro Pro Pro Ala Pro Ala
145                 150                 155                 160

Arg Cys Ser Val Leu Ala Gly Gly Leu Gly Pro Phe Arg Pro Leu Trp
                165                 170                 175

Ala Leu Leu Ala Phe Ala Leu Pro Ala Leu Leu Leu Leu Gly Ala Tyr
            180                 185                 190

Gly Gly Ile Phe Val Val Ala Arg Arg Ala Ala Leu Arg Pro Pro Arg
        195                 200                 205

Pro Ala Arg Gly Ser Arg Leu Arg Ser Asp Ser Leu Asp Ser Arg Leu
    210                 215                 220

Ser Ile Leu Pro Pro Leu Arg Pro Arg Leu Pro Gly Gly Lys Ala Ala
225                 230                 235                 240

Leu Ala Pro Ala Leu Ala Val Gly Gln Phe Ala Ala Cys Trp Leu Pro
                245                 250                 255

Tyr Gly Cys Ala Cys Leu Ala Pro Ala Ala Arg Ala Ala Glu Ala Glu
            260                 265                 270

Ala Ala Val Thr Trp Val Ala Tyr Ser Ala Phe Ala Ala His Pro Phe
        275                 280                 285

Leu Tyr Gly Leu Leu Gln Arg Pro Val Arg Leu Ala Leu Gly Arg Leu

290                    295                    300

Ser Arg Arg Ala Leu Pro Gly Pro Val Arg Ala Cys Thr Pro Gln Ala
305                 310                 315                 320

Trp His Pro Arg Ala Leu Leu Gln Cys Leu Gln Arg Pro Pro Glu Gly
                325                 330                 335

Pro Ala Val Gly Pro Ser Glu Ala Pro Glu Gln Thr Pro Glu Leu Ala
            340                 345                 350

Gly Gly Arg Ser Pro Ala Tyr Gln Gly Pro Pro Glu Ser Ser Leu Ser
        355                 360                 365

(8) INFORMATION FOR SEQ ID NO:7:

    (i) SEQUENCE CHARACTERISTICS:
        (A) LENGTH: 1008 base pairs
        (B) TYPE: nucleic acid
        (C) STRANDEDNESS: single
        (D) TOPOLOGY: linear

    (ii) MOLECULE TYPE: DNA (genomic)

    (xi) SEQUENCE DESCRIPTION: SEQ ID NO:7:

ATGGAATCAT CTTTCTCATT TGGAGTGATC CTTGCTGTCC TGGCCTCCCT CATCATTGCT  60

ACTAACACAC TAGTGGCTGT GGCTGTGCTG CTGTTGATCC ACAAGAATGA TGGTGTCAGT 120

CTCTGCTTCA CCTTGAATCT GGCTGTGGCT GACACCTTGA TTGGTGTGGC CATCTCTGGC 180

CTACTCACAG ACCAGCTCTC CAGCCCTTCT CGGCCCACAC AGAAGACCCT GTGCAGCCTG 240

CGGATGGCAT TTGTCACTTC CTCCGCAGCT GCCTCTGTCC TCACGGTCAT GCTGATCACC 300

TTTGACAGGT ACCTTGCCAT CAAGCAGCCC TTCCGCTACT TGAAGATCAT GAGTGGGTTC 360

GTGGCCGGGG CCTGCATTGC CGGGCTGTGG TTAGTGTCTT ACCTCATTGG CTTCCTCCCA 420

CTCGGAATCC CCATGTTCCA GCAGACTGCC TACAAAGGGC AGTGCAGCTT CTTTGCTGTA 480

TTTCACCCTC ACTTCGTGCT GACCCTCTCC TGCGTTGGCT TCTTCCCAGC CATGCTCCTC 540

TTTGTCTTCT TCTACTGCGA CATGCTCAAG ATTGCCTCCA TGCACAGCCA GCAGATTCGA 600

AAGATGGAAC ATGCAGGAGC CATGGCTGGA GGTTATCGAT CCCCACGGAC TCCCAGCGAC 660

TTCAAAGCTC TCCGTACTGT GTCTGTTCTC ATTGGGAGCT TTGCTCTATC CTGGACCCCC 720

TTCCTTATCA CTGGCATTGT GCAGGTGGCC TGCCAGGAGT GTCACCTCTA CCTAGTGCTG 780

GAACGGTACC TGTGGCTGCT CGGCGTGGGC AACTCCCTGC TCAACCCACT CATCTATGCC 840

TATTGGCAGA AGGAGGTGCG ACTGCAGCTC TACCACATGG CCCTAGGAGT GAAGAAGGTG 900

CTCACCTCAT TCCTCCTCTT TCTCTCGGCC AGGAATTGTG GCCCAGAGAG GCCCAGGGAA 960

AGTTCCTGTC ACATCGTCAC TATCTCCAGC TCAGAGTTTG ATGGCTAA 1008

(9) INFORMATION FOR SEQ ID NO:8:

(i) SEQUENCE CHARACTERISTICS:
(A) LENGTH: 335 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:8:

Met Glu Ser Ser Phe Ser Phe Gly Val Ile Leu Ala Val Leu Ala Ser
1               5                   10                  15

Leu Ile Ile Ala Thr Asn Thr Leu Val Ala Val Ala Val Leu Leu Leu
            20                  25                  30

Ile His Lys Asn Asp Gly Val Ser Leu Cys Phe Thr Leu Asn Leu Ala
        35                  40                  45

Val Ala Asp Thr Leu Ile Gly Val Ala Ile Ser Gly Leu Leu Thr Asp
    50                  55                  60

Gln Leu Ser Ser Pro Ser Arg Pro Thr Gln Lys Thr Leu Cys Ser Leu
65                  70                  75                  80

Arg Met Ala Phe Val Thr Ser Ser Ala Ala Ala Ser Val Leu Thr Val
                85                  90                  95

Met Leu Ile Thr Phe Asp Arg Tyr Leu Ala Ile Lys Gln Pro Phe Arg
            100                 105                 110

Tyr Leu Lys Ile Met Ser Gly Phe Val Ala Gly Ala Cys Ile Ala Gly
        115                 120                 125

Leu Trp Leu Val Ser Tyr Leu Ile Gly Phe Leu Pro Leu Gly Ile Pro
    130                 135                 140

Met Phe Gln Gln Thr Ala Tyr Lys Gly Gln Cys Ser Phe Phe Ala Val
145                 150                 155                 160

Phe His Pro His Phe Val Leu Thr Leu Ser Cys Val Gly Phe Phe Pro
                165                 170                 175

Ala Met Leu Leu Phe Val Phe Phe Tyr Cys Asp Met Leu Lys Ile Ala
            180                 185                 190

Ser Met His Ser Gln Gln Ile Arg Lys Met Glu His Ala Gly Ala Met
        195                 200                 205

Ala Gly Gly Tyr Arg Ser Pro Arg Thr Pro Ser Asp Phe Lys Ala Leu
    210                 215                 220

Arg Thr Val Ser Val Leu Ile Gly Ser Phe Ala Leu Ser Trp Thr Pro
225                 230                 235                 240

Phe Leu Ile Thr Gly Ile Val Gln Val Ala Cys Gln Glu Cys His Leu
                245                 250                 255

Tyr Leu Val Leu Glu Arg Tyr Leu Trp Leu Leu Gly Val Gly Asn Ser
            260                 265                 270

Leu Leu Asn Pro Leu Ile Tyr Ala Tyr Trp Gln Lys Glu Val Arg Leu
        275                 280                 285

Gln Leu Tyr His Met Ala Leu Gly Val Lys Lys Val Leu Thr Ser Phe
    290                 295                 300

Leu Leu Phe Leu Ser Ala Arg Asn Cys Gly Pro Glu Arg Pro Arg Glu
305                 310                 315                 320

Ser Ser Cys His Ile Val Thr Ile Ser Ser Ser Glu Phe Asp Gly
                325                 330                 335

(10) INFORMATION FOR SEQ ID NO:9:

(i) SEQUENCE CHARACTERISTICS:
(A) LENGTH: 1413 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:9:

ATGGACACTA CCATGGAAGC TGACCTGGGT GCCACTGGCC ACAGGCCCCG CACAGAGCTT 60

GATGATGAGG ACTCCTACCC CCAAGGTGGC TGGGACACGG TCTTCCTGGT GGCCCTGCTG 120

CTCCTTGGGC TGCCAGCCAA TGGGTTGATG GCGTGGCTGG CCGGCTCCCA GGCCCGGCAT 180

30 GGAGCTGGCA CGCGTCTGGC GCTGCTCCTG CTCAGCCTGG CCCTCTCTGA CTTCTTGTTC 240

CTGGCAGCAG CGGCCTTCCA GATCCTAGAG ATCCGGCATG GGGGACACTG GCCGCTGGGG 300

ACAGCTGCCT GCCGCTTCTA CTACTTCCTA TGGGGCGTGT CCTACTCCTC CGGCCTCTTC 360

CTGCTGGCCG CCCTCAGCCT CGACCGCTGC CTGCTGGCGC TGTGCCCACA CTGGTACCCT 420

GGGCACCGCC CAGTCCGCCT GCCCCTCTGG GTCTGCGCCG GTGTCTGGGT GCTGGCCACA 480

CTCTTCAGCG TGCCCTGGCT GGTCTTCCCC GAGGCTGCCG TCTGGTGGTA CGACCTGGTC 540

ATCTGCCTGG ACTTCTGGGA CAGCGAGGAG CTGTCGCTGA GGATGCTGGA GGTCCTGGGG 600

GGCTTCCTGC CTTTCCTCCT GCTGCTCGTC TGCCACGTGC TCACCCAGGC CACAGCCTGT 660

CGCACCTGCC ACCGCCAACA GCAGCCCGCA GCCTGCCGGG GCTTCGCCCG TGTGGCCAGG 720

ACCATTCTGT CAGCCTATGT GGTCCTGAGG CTGCCCTACC AGCTGGCCCA GCTGCTCTAC 780

CTGGCCTTCC TGTGGGACGT CTACTCTGGC TACCTGCTCT GGGAGGCCCT GGTCTACTCC 840

GACTACCTGA TCCTACTCAA CAGCTGCCTC AGCCCCTTCC TCTGCCTCAT GGCCAGTGCC 900

GACCTCCGGA CCCTGCTGCG CTCCGTGCTC TCGTCCTTCG CGGCAGCTCT CTGCGAGGAG 960

CGGCCGGGCA GCTTCACGCC CACTGAGCCA CAGACCCAGC TAGATTCTGA GGGTCCAACT1020

CTGCCAGAGC CGATGGCAGA GGCCCAGTCA CAGATGGATC CTGTGGCCCA GCCTCAGGTG1080

AACCCCACAC TCCAGCCACG ATCGGATCCC ACAGCTCAGC CACAGCTGAA CCCTACGGCC1140

CAGCCACAGT CGGATCCCAC AGCCCAGCCA CAGCTGAACC TCATGGCCCA GCCACAGTCA1200

GATTCTGTGG CCCAGCCACA GGCAGACACT AACGTCCAGA CCCCTGCACC TGCTGCCAGT1260

TCTGTGCCCA GTCCCTGTGA TGAAGCTTCC CCAACCCCAT CCTCGCATCC TACCCCAGGG1320

15 GCCCTTGAGG ACCCAGCCAC ACCTCCTGCC TCTGAAGGAG AAAGCCCCAG CAGCACCCCG1380

CCAGAGGCGG CCCCGGGCGC AGGCCCCACG TGA 1413

(11) INFORMATION FOR SEQ ID NO:10:

(i) SEQUENCE CHARACTERISTICS:
(A) LENGTH: 468 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:10:

Met Asp Thr Thr Met Glu Ala Asp Leu Gly Ala Thr Gly His Arg Pro
1               5                   10                  15

Arg Thr Glu Leu Asp Asp Glu Asp Ser Tyr Pro Gln Gly Gly Trp Asp
            20                  25                  30

Thr Val Phe Leu Val Ala Leu Leu Leu Leu Gly Leu Pro Ala Asn Gly
        35                  40                  45

Leu Met Ala Trp Leu Ala Gly Ser Gln Ala Arg His Gly Ala Gly Thr

50                  55                  60

Arg Leu Ala Leu Leu Leu Leu Ser Leu Ala Leu Ser Asp Phe Leu Phe
65                  70                  75                  80

Leu Ala Ala Ala Ala Phe Gln Ile Leu Glu Ile Arg His Gly Gly His
                85                  90                  95

Trp Pro Leu Gly Thr Ala Ala Cys Arg Phe Tyr Tyr Phe Leu Trp Gly
            100                 105                 110

Val Ser Tyr Ser Ser Gly Leu Phe Leu Leu Ala Ala Leu Ser Leu Asp
        115                 120                 125

Arg Cys Leu Leu Ala Leu Cys Pro His Trp Tyr Pro Gly His Arg Pro
    130                 135                 140

Val Arg Leu Pro Leu Trp Val Cys Ala Gly Val Trp Val Leu Ala Thr
145                 150                 155                 160

Leu Phe Ser Val Pro Trp Leu Val Phe Pro Glu Ala Ala Val Trp Trp
                165                 170                 175

Tyr Asp Leu Val Ile Cys Leu Asp Phe Trp Asp Ser Glu Glu Leu Ser
            180                 185                 190

Leu Arg Met Leu Glu Val Leu Gly Gly Phe Leu Pro Phe Leu Leu Leu
        195                 200                 205

Leu Val Cys His Val Leu Thr Gln Ala Thr Arg Thr Cys His Arg Gln
    210                 215                 220

Gln Gln Pro Ala Ala Cys Arg Gly Phe Ala Arg Val Ala Arg Thr Ile
225                 230                 235                 240

Leu Ser Ala Tyr Val Val Leu Arg Leu Pro Tyr Gln Leu Ala Gln Leu
                245                 250                 255

Leu Tyr Leu Ala Phe Leu Trp Asp Val Tyr Ser Gly Tyr Leu Leu Trp
            260                 265                 270

Glu Ala Leu Val Tyr Ser Asp Tyr Leu Ile Leu Leu Asn Ser Cys Leu
        275                 280                 285

Ser Pro Phe Leu Cys Leu Met Ala Ser Ala Asp Leu Arg Thr Leu Leu
    290                 295                 300

Arg Ser Val Leu Ser Ser Phe Ala Ala Ala Leu Cys Glu Glu Arg Pro
305                 310                 315                 320

Gly Ser Phe Thr Pro Thr Glu Pro Gln Thr Gln Leu Asp Ser Glu Gly
                325                 330                 335

Pro Thr Leu Pro Glu Pro Met Ala Glu Ala Gln Ser Gln Met Asp Pro
            340                 345                 350

Val Ala Gln Pro Gln Val Asn Pro Thr Leu Gln Pro Arg Ser Asp Pro
        355                 360                 365

Thr Ala Gln Pro Gln Leu Asn Pro Thr Ala Gln Pro Gln Ser Asp Pro
    370                 375                 380

Thr Ala Gln Pro Gln Leu Asn Leu Met Ala Gln Pro Gln Ser Asp Ser
385                 390                 395                 400

Val Ala Gln Pro Gln Ala Asp Thr Asn Val Gln Thr Pro Ala Pro Ala
                405                 410                 415

Ala Ser Ser Val Pro Ser Pro Cys Asp Glu Ala Ser Pro Thr Pro Ser
            420                 425                 430

Ser His Pro Thr Pro Gly Ala Leu Glu Asp Pro Ala Thr Pro Pro Ala
        435                 440                 445

Ser Glu Gly Glu Ser Pro Ser Ser Thr Pro Pro Glu Ala Ala Pro Gly
    450                 455                 460

Ala Gly Pro Thr
465

(12) INFORMATION FOR SEQ ID NO:11:

(i) SEQUENCE CHARACTERISTICS:
(A) LENGTH: 1248 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:11:

ATGTCAGGGA TGGAAAAACT TCAGAATGCT TCCTGGATCT ACCAGCAGAA ACTAGAAGAT 60

CCATTCCAGA AACACCTGAA CAGCACCGAG GAGTATCTGG CCTTCCTCTG CGGACCTCGG 120

CGCAGCCACT TCTTCCTCCC CGTGTCTGTG GTGTATGTGC CAATTTTTGT GGTGGGGGTC 180

ATTGGCAATG TCCTGGTGTG CCTGGTGATT CTGCAGCACC AGGCTATGAA GACGCCCACC 240

AACTACTACC TCTTCAGCCT GGCGGTCTCT GACCTCCTGG TCCTGCTCCT TGGAATGCCC 300

CTGGAGGTCT ATGAGATGTG GCGCAACTAC CCTTTCTTGT TCGGGCCCGT GGGCTGCTAC 360

TTCAAGACGG CCCTCTTTGA GACCGTGTGC TTCGCCTCCA TCCTCAGCAT CACCACCGTC 420

AGCGTGGAGC GCTACGTGGC CATCCTACAC CCGTTCCGCG CCAAACTGCA GAGCACCCGG 480

CGCCGGGCCC TCAGGATCCT CGGCATCGTC TGGGGCTTCT CCGTGCTCTT CTCCCTGCCC 540

AACACCAGCA TCCATGGCAT CAAGTTCCAC TACTTCCCCA ATGGGTCCCT GGTCCCAGGT 600

TCGGCCACCT GTACGGTCAT CAAGCCCATG TGGATCTACA ATTTCATCAT CCAGGTCACC 660

TCCTTCCTAT TCTACCTCCT CCCCATGACT GTCATCAGTG TCCTCTACTA CCTCATGGCA 720

CTCAGACTAA AGAAAGACAA ATCTCTTGAG GCAGATGAAG GGAATGCAAA TATTCAAAGA 780

5 CCCTGCAGAA AATCAGTCAA CAAGATGCTG TTTGTCTTGG TCTTAGTGTT TGCTATCTGT 840

TGGGCCCCGT TCCACATTGA CCGACTCTTC TTCAGCTTTG TGGAGGAGTG GAGTGAATCC 900

CTGGCTGCTG TGTTCAACCT CGTCCATGTG GTGTCAGGTG TCTTCTTCTA CCTGAGCTCA 960

GCTGTCAACC CCATTATCTA TAACCTACTG TCTCGCCGCT TCCAGGCAGC ATTCCAGAAT1020

GTGATCTCTT CTTTCCACAA ACAGTGGCAC TCCCAGCATG ACCCACAGTT GCCACCTGCC1080

10 CAGCGGAACA TCTTCCTGAC AGAATGCCAC TTTGTGGAGC TGACCGAAGA TATAGGTCCC1140

CAATTCCCAT GTCAGTCATC CATGCACAAC TCTCACCTCC CAACAGCCCT CTCTAGTGAA1200

CAGATGTCAA GAACAAACTA TCAAAGCTTC CACTTTAACA AAACCTGA 1248

(13) INFORMATION FOR SEQ ID NO:12:

(i) SEQUENCE CHARACTERISTICS:
(A) LENGTH: 415 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:12:

(14) INFORMATION FOR SEQ ID NO:13:

(i) SEQUENCE CHARACTERISTICS:
(A) LENGTH: 1173 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:13:

ATGCCAGATA CTAATAGCAC AATCAATTTA TCACTAAGCA CTCGTGTTAC TTTAGCATTT 60

TTTATGTCCT TAGTAGCTTT TGCTATAATG CTAGGAAATG CTTTGGTCAT TTTAGCTTTT 120

GTGGTGGACA AAAACCTTAG ACATCGAAGT AGTTATTTTT TTCTTAACTT GGCCATCTCT 180

GACTTCTTTG TGGGTGTGAT CTCCATTCCT TTGTACATCC CTCACACGCT GTTCGAATGG 240

GATTTTGGAA AGGAAATCTG TGTATTTTGG CTCACTACTG ACTATCTGTT ATGTACAGCA 300

TCTGTATATA ACATTGTCCT CATCAGCTAT GATCGATACC TGTCAGTCTC AAATGCTGTG 360

TCTTATAGAA CTCAACATAC TGGGGTCTTG AAGATTGTTA CTCTGATGGT GGCCGTTTGG 420

GTGCTGGCCT TCTTAGTGAA TGGGCCAATG ATTCTAGTTT CAGAGTCTTG GAAGGATGAA 480

GGTAGTGAAT GTGAACCTGG ATTTTTTTCG GAATGGTACA TCCTTGCCAT CACATCATTC 540

TTGGAATTCG TGATCCCAGT CATCTTAGTC GCTTATTTCA ACATGAATAT TTATTGGAGC 600

CTGTGGAAGC GTGATCATCT CAGTAGGTGC CAAAGCCATC CTGGACTGAC TGCTGTCTCT 660

TCCAACATCT GTGGACACTC ATTCAGAGGT AGACTATCTT CAAGGAGATC TCTTTCTGCA 720

TCGACAGAAG TTCCTGCATC CTTTCATTCA GAGAGACAGA GGAGAAAGAG TAGTCTCATG 780

TTTTCCTCAA GAACCAAGAT GAATAGCAAT ACAATTGCTT CCAAAATGGG TTCCTTCTCC 840

CAATCAGATT CTGTAGCTCT TCACCAAAGG GAACATGTTG AACTGCTTAG AGCCAGGAGA 900

TTAGCCAAGT CACTGGCCAT TCTCTTAGGG GTTTTTGCTG TTTGCTGGGC TCCATATTCT 960

CTGTTCACAA TTGTCCTTTC ATTTTATTCC TCAGCAACAG GTCCTAAATC AGTTTGGTAT1020

AGAATTGCAT TTTGGCTTCA GTGGTTCAAT TCCTTTGTCA ATCCTCTTTT GTATCCATTG1080

TGTCACAAGC GCTTTCAAAA GGCTTTCTTG AAAATATTTT GTATAAAAAA GCAACCTCTA1140

CCATCACAAC ACAGTCGGTC AGTATCTTCT TAA 1173

(15) INFORMATION FOR SEQ ID NO:14:

(i) SEQUENCE CHARACTERISTICS:
(A) LENGTH: 390 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:14:

Met Pro Asp Thr Asn Ser Thr Ile Asn Leu Ser Leu Ser Thr Arg Val
1               5                   10                  15

Thr Leu Ala Phe Phe Met Ser Leu Val Ala Phe Ala Ile Met Leu Gly
            20                  25                  30

Asn Ala Leu Val Ile Leu Ala Phe Val Val Asp Lys Asn Leu Arg His
        35                  40                  45

Arg Ser Ser Tyr Phe Phe Leu Asn Leu Ala Ile Ser Asp Phe Phe Val
    50                  55                  60

Gly Val Ile Ser Ile Pro Leu Tyr Ile Pro His Thr Leu Phe Glu Trp
65                  70                  75                  80

Asp Phe Gly Lys Glu Ile Cys Val Phe Trp Leu Thr Thr Asp Tyr Leu
                85                  90                  95

Leu Cys Thr Ala Ser Val Tyr Asn Ile Val Leu Ile Ser Tyr Asp Arg
            100                 105                 110

Tyr Leu Ser Val Ser Asn Ala Val Ser Tyr Arg Thr Gln His Thr Gly
        115                 120                 125

Val Leu Lys Ile Val Thr Leu Met Val Ala Val Trp Val Leu Ala Phe
    130                 135                 140

Leu Val Asn Gly Pro Met Ile Leu Val Ser Glu Ser Trp Lys Asp Glu
145                 150                 155                 160

Gly Ser Glu Cys Glu Pro Gly Phe Phe Ser Glu Trp Tyr Ile Leu Ala
                165                 170                 175

Ile Thr Ser Phe Leu Glu Phe Val Ile Pro Val Ile Leu Val Ala Tyr
            180                 185                 190

Phe Asn Met Asn Ile Tyr Trp Ser Leu Trp Lys Arg Asp His Leu Ser
        195                 200                 205

Arg Cys Gln Ser His Pro Gly Leu Thr Ala Val Ser Ser Asn Ile Cys
    210                 215                 220

Gly His Ser Phe Arg Gly Arg Leu Ser Ser Arg Arg Ser Leu Ser Ala
225                 230                 235                 240

Ser Thr Glu Val Pro Ala Ser Phe His Ser Glu Arg Gln Arg Arg Lys
                245                 250                 255

Ser Ser Leu Met Phe Ser Ser Arg Thr Lys Met Asn Ser Asn Thr Ile
            260                 265                 270

Ala Ser Lys Met Gly Ser Phe Ser Gln Ser Asp Ser Val Ala Leu His
        275                 280                 285

Gln Arg Glu His Val Glu Leu Leu Arg Ala Arg Arg Leu Ala Lys Ser
    290                 295                 300

Leu Ala Ile Leu Leu Gly Val Phe Ala Val Cys Trp Ala Pro Tyr Ser
305                 310                 315                 320

Leu Phe Thr Ile Val Leu Ser Phe Tyr Ser Ser Ala Thr Gly Pro Lys
                325                 330                 335

Ser Val Trp Tyr Arg Ile Ala Phe Trp Leu Gln Trp Phe Asn Ser Phe
            340                 345                 350

Val Asn Pro Leu Leu Tyr Pro Leu Cys His Lys Arg Phe Gln Lys Ala
        355                 360                 365

Phe Leu Lys Ile Phe Cys Ile Lys Lys Gln Pro Leu Pro Ser Gln His
    370                 375                 380

Ser Arg Ser Val Ser Ser
385                 390

(16) INFORMATION FOR SEQ ID NO:15:

(i) SEQUENCE CHARACTERISTICS:
(A) LENGTH: 1128 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:15:

ATGGCGAACG CGAGCGAGCC GGGTGGCAGC GGCGGCGGCG AGGCGGCCGC CCTGGGCCTC  60

AAGCTGGCCA CGCTCAGCCT GCTGCTGTGC GTGAGCCTAG CGGGCAACGT GCTGTTCGCG 120

CTGCTGATCG TGCGGGAGCG CAGCCTGCAC CGCGCCCCGT ACTACCTGCT GCTCGACCTG 180

TGCCTGGCCG ACGGGCTGCG CGCGCTCGCC TGCCTCCCGG CCGTCATGCT GGCGGCGCGG 240

CGTGCGGCGG CCGCGGCGGG GGCGCCGCCG GGCGCGCTGG GCTGCAAGCT GCTCGCCTTC 300

CTGGCCGCGC TCTTCTGCTT CCACGCCGCC TTCCTGCTGC TGGGCGTGGG CGTCACCCGC 360

TACCTGGCCA TCGCGCACCA CCGCTTCTAT GCAGAGCGCC TGGCCGGCTG GCCGTGCGCC 420

GCCATGCTGG TGTGCGCCGC CTGGGCGCTG GCGCTGGCCG CGGCCTTCCC GCCAGTGCTG 480

GACGGCGGTG GCGACGACGA GGACGCGCCG TGCGCCCTGG AGCAGCGGCC CGACGGCGCC 540

CCCGGCGCGC TGGGCTTCCT GCTGCTGCTG GCCGTGGTGG TGGGCGCCAC GCACCTCGTC 600

TACCTCCGCC TGCTCTTCTT CATCCACGAC CGCCGCAAGA TGCGGCCCGC GCGCCTGGTG 660

CCCGCCGTCA GCCACGACTG GACCTTCCAC GGCCCGGGCG CCACCGGCCA GGCGGCCGCC 720

AACTGGACGG CGGGCTTCGG CCGCGGGCCC ACGCCGCCCG CGCTTGTGGG CATCCGGCCC 780

GCAGGGCCGG GCCGCGGCGC GCGCCGCCTC CTCGTGCTGG AAGAATTCAA GACGGAGAAG 840

AGGCTGTGCA AGATGTTCTA CGCCGTCACG CTGCTCTTCC TGCTCCTCTG GGGGCCCTAC 900

GTCGTGGCCA GCTACCTGCG GGTCCTGGTG CGGCCCGGCG CCGTCCCCCA GGCCTACCTG 960

ACGGCCTCCG TGTGGCTGAC CTTCGCGCAG GCCGGCATCA ACCCCGTCGT GTGCTTCCTC1020

TTCAACAGGG AGCTGAGGGA CTGCTTCAGG GCCCAGTTCC CCTGCTGCCA GAGCCCCCGG1080

ACCACCCAGG CGACCCATCC CTGCGACCTG AAAGGCATTG GTTTATGA 1128

(17) INFORMATION FOR SEQ ID NO:16:

(i) SEQUENCE CHARACTERISTICS:
(A) LENGTH: 375 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:16:

Met Ala Asn Ala Ser Glu Pro Gly Gly Ser Gly Gly Gly Glu Ala Ala
1               5                   10                  15

Ala Leu Gly Leu Lys Leu Ala Thr Leu Ser Leu Leu Leu Cys Val Ser
            20                  25                  30

Leu Ala Gly Asn Val Leu Phe Ala Leu Leu Ile Val Arg Glu Arg Ser
        35                  40                  45

Leu His Arg Ala Pro Tyr Tyr Leu Leu Leu Asp Leu Cys Leu Ala Asp
    50                  55                  60

Gly Leu Arg Ala Leu Ala Cys Leu Pro Ala Val Met Leu Ala Ala Arg
65                  70                  75                  80

Arg Ala Ala Ala Ala Ala Gly Ala Pro Pro Gly Ala Leu Gly Cys Lys
                85                  90                  95

Leu Leu Ala Phe Leu Ala Ala Leu Phe Cys Phe His Ala Ala Phe Leu
            100                 105                 110

Leu Leu Gly Val Gly Val Thr Arg Tyr Leu Ala Ile Ala His His Arg
        115                 120                 125

Phe Tyr Ala Glu Arg Leu Ala Gly Trp Pro Cys Ala Ala Met Leu Val
    130                 135                 140

Cys Ala Ala Trp Ala Leu Ala Leu Ala Ala Ala Phe Pro Pro Val Leu
145                 150                 155                 160

Asp Gly Gly Gly Asp Asp Glu Asp Ala Pro Cys Ala Leu Glu Gln Arg
                165                 170                 175

Pro Asp Gly Ala Pro Gly Ala Leu Gly Phe Leu Leu Leu Leu Ala Val
            180                 185                 190

Val Val Gly Ala Thr His Leu Val Tyr Leu Arg Leu Leu Phe Phe Ile
        195                 200                 205

His Asp Arg Arg Lys Met Arg Pro Ala Arg Leu Val Pro Ala Val Ser
    210                 215                 220

His Asp Trp Thr Phe His Gly Pro Gly Ala Thr Gly Gln Ala Ala Ala
225                 230                 235                 240

Asn Trp Thr Ala Gly Phe Gly Arg Gly Pro Thr Pro Pro Ala Leu Val
                245                 250                 255

Gly Ile Arg Pro Ala Gly Pro Gly Arg Gly Ala Arg Arg Leu Leu Val
            260                 265                 270

Leu Glu Glu Phe Lys Thr Glu Lys Arg Leu Cys Lys Met Phe Tyr Ala
        275                 280                 285

Val Thr Leu Leu Phe Leu Leu Leu Trp Gly Pro Tyr Val Val Ala Ser
    290                 295                 300

Tyr Leu Arg Val Leu Val Arg Pro Gly Ala Val Pro Gln Ala Tyr Leu
305                 310                 315                 320

Thr Ala Ser Val Trp Leu Thr Phe Ala Gln Ala Gly Ile Asn Pro Val
                325                 330                 335

Val Cys Phe Leu Phe Asn Arg Glu Leu Arg Asp Cys Phe Arg Ala Gln
            340                 345                 350

Phe Pro Cys Cys Gln Ser Pro Arg Thr Thr Gln Ala Thr His Pro Cys
        355                 360                 365

Asp Leu Lys Gly Ile Gly Leu

370                    375

(18) INFORMATION FOR SEQ ID NO:17:

(i) SEQUENCE CHARACTERISTICS:
(A) LENGTH: 1002 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:17:

ATGAACACCA CAGTGATGCA AGGCTTCAAC AGATCTGAGC GGTGCCCCAG AGACACTCGG 60

ATAGTACAGC TGGTATTCCC AGCCCTCTAC ACAGTGGTTT TCTTGACCGG CATCCTGCTG 120

AATACTTTGG CTCTGTGGGT GTTTGTTCAC ATCCCCAGCT CCTCCACCTT CATCATCTAC 180

CTCAAAAACA CTTTGGTGGC CGACTTGATA ATGACACTCA TGCTTCCTTT CAAAATCCTC 240

TCTGACTCAC ACCTGGCACC CTGGCAGCTC AGAGCTTTTG TGTGTCGTTT TTCTTCGGTG 300

15 ATATTTTATG AGACCATGTA TGTGGGCATC GTGCTGTTAG GGCTCATAGC CTTTGACAGA 360

TTCCTCAAGA TCATCAGACC TTTGAGAAAT ATTTTTCTAA AAAAACCTGT TTTTGCAAAA 420

ACGGTCTCAA TCTTCATCTG GTTCTTTTTG TTCTTCATCT CCCTGCCAAA TACGATCTTG 480

AGCAACAAGG AAGCAACACC ATCGTCTGTG AAAAAGTGTG CTTCCTTAAA GGGGCCTCTG 540

GGGCTGAAAT GGCATCAAAT GGTAAATAAC ATATGCCAGT TTATTTTCTG GACTGTTTTT 600

ATCCTAATGC TTGTGTTTTA TGTGGTTATT GCAAAAAAAG TATATGATTC TTATAGAAAG 660

TCCAAAAGTA AGGACAGAAA AAACAACAAA AAGCTGGAAG GCAAAGTATT TGTTGTCGTG 720

GCTGTCTTCT TTGTGTGTTT TGCTCCATTT CATTTTGCCA GAGTTCCATA TACTCACAGT 780

CAAACCAACA ATAAGACTGA CTGTAGACTG CAAAATCAAC TGTTTATTGC TAAAGAAACA 840

ACTCTCTTTT TGGCAGCAAC TAACATTTGT ATGGATCCCT TAATATACAT ATTCTTATGT 900

AAAAAATTCA CAGAAAAGCT ACCATGTATG CAAGGGAGAA AGACCACAGC ATCAAGCCAA 960

GAAAATCATA GCAGTCAGAC AGACAACATA ACCTTAGGCT GA 1002

(19) INFORMATION FOR SEQ ID NO:18:

(i) SEQUENCE CHARACTERISTICS:
(A) LENGTH: 333 amino acids
(B) TYPE: amino acid
(C) STRANDEDNESS:

(D) TOPOLOGY: not relevant

(ii) MOLECULE TYPE: protein

(xi) SEQUENCE DESCRIPTION: SEQ ID NO:18:

Met Asn Thr Thr Val Met Gln Gly Phe Asn Arg Ser Glu Arg Cys Pro
1               5                   10                  15

Arg Asp Thr Arg Ile Val Gln Leu Val Phe Pro Ala Leu Tyr Thr Val
            20                  25                  30

Val Phe Leu Thr Gly Ile Leu Leu Asn Thr Leu Ala Leu Trp Val Phe
        35                  40                  45

Val His Ile Pro Ser Ser Ser Thr Phe Ile Ile Tyr Leu Lys Asn Thr
    50                  55                  60

Leu Val Ala Asp Leu Ile Met Thr Leu Met Leu Pro Phe Lys Ile Leu
65                  70                  75                  80

Ser Asp Ser His Leu Ala Pro Trp Gln Leu Arg Ala Phe Val Cys Arg
                85                  90                  95

Phe Ser Ser Val Ile Phe Tyr Glu Thr Met Tyr Val Gly Ile Val Leu
            100                 105                 110

Leu Gly Leu Ile Ala Phe Asp Arg Phe Leu Lys Ile Ile Arg Pro Leu
        115                 120                 125

Arg Asn Ile Phe Leu Lys Lys Pro Val Phe Ala Lys Thr Val Ser Ile
    130                 135                 140

Phe Ile Trp Phe Phe Leu Phe Phe Ile Ser Leu Pro Asn Thr Ile Leu
145                 150                 155                 160

Ser Asn Lys Glu Ala Thr Pro Ser Ser Val Lys Lys Cys Ala Ser Leu
                165                 170                 175

Lys Gly Pro Leu Gly Leu Lys Trp His Gln Met Val Asn Asn Ile Cys
            180                 185                 190

Gln Phe Ile Phe Trp Thr Val Phe Ile Leu Met Leu Val Phe Tyr Val
        195                 200                 205

Val Ile Ala Lys Lys Val Tyr Asp Ser Tyr Arg Lys Ser Lys Ser Lys
    210                 215                 220

Asp Arg Lys Asn Asn Lys Lys Leu Glu Gly Lys Val Phe Val Val Val
225                 230                 235                 240

Ala Val Phe Phe Val Cys Phe Ala Pro Phe His Phe Ala Arg Val Pro
                245                 250                 255

Tyr Thr His Ser Gln Thr Asn Asn Lys Thr Asp Cys Arg Leu Gln Asn
            260                 265                 270

Gln Leu Phe Ile Ala Lys Glu Thr Thr Leu Phe Leu Ala Ala Thr Asn
        275                 280                 285

Ile Cys Met Asp Pro Leu Ile Tyr Ile Phe Leu Cys Lys Lys Phe Thr
    290                 295                 300

Glu Lys Leu Pro Cys Met Gln Gly Arg Lys Thr Thr Ala Ser Ser Gln
305                 310                 315                 320

Glu Asn His Ser Ser Gln Thr Asp Asn Ile Thr Leu Gly
                325                 330

(20) INFORMATION FOR SEQ ID NO:19:

(i) SEQUENCE CHARACTERISTICS:
(A) LENGTH: 1122 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:19:

ATGGCCAACA CTACCGGAGA GCCTGAGGAG GTGAGCGGCG CTCTGTCCCC ACCGTCCGCA 60

TCAGCTTATG TGAAGCTGGT ACTGCTGGGA CTGATTATGT GCGTGAGCCT GGCGGGTAAC 120

GCCATCTTGT CCCTGCTGGT GCTCAAGGAG CGTGCCCTGC ACAAGGCTCC TTACTACTTC 180

CTGCTGGACC TGTGCCTGGC CGATGGCATA CGCTCTGCCG TCTGCTTCCC CTTTGTGCTG 240

GCTTCTGTGC GCCACGGCTC TTCATGGACC TTCAGTGCAC TCAGCTGCAA GATTGTGGCC 300

TTTATGGCCG TGCTCTTTTG CTTCCATGCG GCCTTCATGC TGTTCTGCAT CAGCGTCACC 360

CGCTACATGG CCATCGCCCA CCACCGCTTC TACGCCAAGC GCATGACACT CTGGACATGC 420

GCGGCTGTCA TCTGCATGGC CTGGACCCTG TCTGTGGCCA TGGCCTTCCC ACCTGTCTTT 480

GACGTGGGCA CCTACAAGTT TATTCGGGAG GAGGACCAGT GCATCTTTGA GCATCGCTAC 540

TTCAAGGCCA ATGACACGCT GGGCTTCATG CTTATGTTGG CTGTGCTCAT GGCAGCTACC 600

CATGCTGTCT ACGGCAAGCT GCTCCTCTTC GAGTATCGTC ACCGCAAGAT GAAGCCAGTG 660

CAGATGGTGC CAGCCATCAG CCAGAACTGG ACATTCCATG GTCCCGGGGC CACCGGCCAG 720

GCTGCTGCCA ACTGGATCGC CGGCTTTGGC CGTGGGCCCA TGCCACCAAC CCTGCTGGGT 780

ATCCGGCAGA ATGGGCATGC AGCCAGCCGG CGGCTACTGG GCATGGACGA GGTCAAGGGT 840

GAAAAGCAGC TGGGCCGCAT GTTCTACGCG ATCACACTGC TCTTTCTGCT CCTCTGGTCA 900

CCCTACATCG TGGCCTGCTA CTGGCGAGTG TTTGTGAAAG CCTGTGCTGT GCCCCACCGC 960

TACCTGGCCA CTGCTGTTTG GATGAGCTTC GCCCAGGCTG CCGTCAACCC AATTGTCTGC1020

TTCCTGCTCA ACAAGGACCT CAAGAAGTGC CTGACCACTC ACGCCCCCTG CTGGGGCACA1080

GGAGGTGCCC CGGCTCCCAG AGAACCCTAC TGTGTCATGT GA 1122

(21) INFORMATION FOR SEQ ID NO:20:

(i) SEQUENCE CHARACTERISTICS:
(A) LENGTH: 373 amino acids
(B) TYPE: amino acid
(C) STRANDEDNESS:
(D) TOPOLOGY: not relevant

(ii) MOLECULE TYPE: DNA (genomic)

(xi) SEQUENCE DESCRIPTION: SEQ ID NO:20:

Met Ala Asn Thr Thr Gly Glu Pro Glu Glu Val Ser Gly Ala Leu Ser
1               5                   10                  15

Pro Pro Ser Ala Ser Ala Tyr Val Lys Leu Val Leu Leu Gly Leu Ile
            20                  25                  30

Met Cys Val Ser Leu Ala Gly Asn Ala Ile Leu Ser Leu Leu Val Leu
        35                  40                  45

Lys Glu Arg Ala Leu His Lys Ala Pro Tyr Tyr Phe Leu Leu Asp Leu
    50                  55                  60

Cys Leu Ala Asp Gly Ile Arg Ser Ala Val Cys Phe Pro Phe Val Leu
65                  70                  75                  80

Ala Ser Val Arg His Gly Ser Ser Trp Thr Phe Ser Ala Leu Ser Cys
                85                  90                  95

Lys Ile Val Ala Phe Met Ala Val Leu Phe Cys Phe His Ala Ala Phe
            100                 105                 110

Met Leu Phe Cys Ile Ser Val Thr Arg Tyr Met Ala Ile Ala His His
        115                 120                 125

Arg Phe Tyr Ala Lys Arg Met Thr Leu Trp Thr Cys Ala Ala Val Ile
    130                 135                 140

Cys Met Ala Trp Thr Leu Ser Val Ala Met Ala Phe Pro Pro Val Phe
145                 150                 155                 160

Asp Val Gly Thr Tyr Lys Phe Ile Arg Glu Glu Asp Gln Cys Ile Phe
                165                 170                 175

Glu His Arg Tyr Phe Lys Ala Asn Asp Thr Leu Gly Phe Met Leu Met
            180                 185                 190

Leu Ala Val Leu Met Ala Ala Thr His Ala Val Tyr Gly Lys Leu Leu
        195                 200                 205

Leu Phe Glu Tyr Arg His Arg Lys Met Lys Pro Val Gln Met Val Pro
    210                 215                 220

Ala Ile Ser Gln Asn Trp Thr Phe His Gly Pro Gly Ala Thr Gly Gln
225                 230                 235                 240

Ala Ala Ala Asn Trp Ile Ala Gly Phe Gly Arg Gly Pro Met Pro Pro
                245                 250                 255

Thr Leu Leu Gly Ile Arg Gln Asn Gly His Ala Ala Ser Arg Arg Leu
            260                 265                 270

Leu Gly Met Asp Glu Val Lys Gly Glu Lys Gln Leu Gly Arg Met Phe
        275                 280                 285

Tyr Ala Ile Thr Leu Leu Phe Leu Leu Leu Trp Ser Pro Tyr Ile Val
    290                 295                 300

Ala Cys Tyr Trp Arg Val Phe Val Lys Ala Cys Ala Val Pro His Arg
305                 310                 315                 320

Tyr Leu Ala Thr Ala Val Trp Met Ser Phe Ala Gln Ala Ala Val Asn
                325                 330                 335

Pro Ile Val Cys Phe Leu Leu Asn Lys Asp Leu Lys Lys Cys Leu Thr
            340                 345                 350

Thr His Ala Pro Cys Trp Gly Thr Gly Gly Ala Pro Ala Pro Arg Glu
        355                 360                 365

Pro Tyr Cys Val Met
    370

(22) INFORMATION FOR SEQ ID NO:21:

(i) SEQUENCE CHARACTERISTICS:
(A) LENGTH: 1053 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:21:

35 ATGGCTTTGG AACAGAACCA GTCAACAGAT TATTATTATG AGGAAAATGA AATGAATGGC 60

ACTTATGACT ACAGTCAATA TGAATTGATC TGTATCAAAG AAGATGTCAG AGAATTTGCA 120

AAAGTTTTCC TCCCTGTATT CCTCACAATA GCTTTCGTCA TTGGACTTGC AGGCAATTCC 180

ATGGTAGTGG CAATTTATGC CTATTACAAG AAACAGAGAA CCAAAACAGA TGTGTACATC 240

CTGAATTTGG CTGTAGCAGA TTTACTCCTT CTATTCACTC TGCCTTTTTG GGCTGTTAAT 300

GCAGTTCATG GGTGGGTTTT AGGGAAAATA ATGTGCAAAA TAACTTCAGC CTTGTACACA 360

CTAAACTTTG TCTCTGGAAT GCAGTTTCTG GCTTGCATCA GCATAGACAG ATATGTGGCA 420

GTAACTAATG TCCCCAGCCA ATCAGGAGTG GGAAAACCAT GCTGGATCAT CTGTTTCTGT 480

GTCTGGATGG CTGCCATCTT GCTGAGCATA CCCCAGCTGG TTTTTTATAC AGTAAATGAC 540

AATGCTAGGT GCATTCCCAT TTTCCCCCGC TACCTAGGAA CATCAATGAA AGCATTGATT 600

CAAATGCTAG AGATCTGCAT TGGATTTGTA GTACCCTTTC TTATTATGGG GGTGTGCTAC 660

TTTATCACGG CAAGGACACT CATGAAGATG CCAAACATTA AAATATCTCG ACCCCTAAAA 720

GTTCTGCTCA CAGTCGTTAT AGTTTTCATT GTCACTCAAC TGCCTTATAA CATTGTCAAG 780

TTCTGCCGAG CCATAGACAT CATCTACTCC CTGATCACCA GCTGCAACAT GAGCAAACGC 840

ATGGACATCG CCATCCAAGT CACAGAAAGC ATTGCACTCT TTCACAGCTG CCTCAACCCA 900

ATCCTTTATG TTTTTATGGG AGCATCTTTC AAAAACTACG TTATGAAAGT GGCCAAGAAA 960

TATGGGTCCT GGAGAAGACA GAGACAAAGT GTGGAGGAGT TTCCTTTTGA TTCTGAGGGT1020

CCTACAGAGC CAACCAGTAC TTTTAGCATT TAA 1053

(23) INFORMATION FOR SEQ ID NO:22:

(i) SEQUENCE CHARACTERISTICS:
(A) LENGTH: 350 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:22:

(24) INFORMATION FOR SEQ ID NO:23:

(i) SEQUENCE CHARACTERISTICS:
(A) LENGTH: 1116 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:23:

ATGCCAGGAA ACGCCACCCC AGTGACCACC ACTGCCCCGT GGGCCTCCCT GGGCCTCTCC 60

GCCAAGACCT GCAACAACGT GTCCTTCGAA GAGAGCAGGA TAGTCCTGGT CGTGGTGTAC 120

AGCGCGGTGT GCACGCTGGG GGTGCCGGCC AACTGCCTGA CTGCGTGGCT GGCGCTGCTG 180

CAGGTACTGC AGGGCAACGT GCTGGCCGTC TACCTGCTCT GCCTGGCACT CTGCGAACTG 240

CTGTACACAG GCACGCTGCC ACTCTGGGTC ATCTATATCC GCAACCAGCA CCGCTGGACC 300

CTAGGCCTGC TGGCCTCGAA GGTGACCGCC TACATCTTCT TCTGCAACAT CTACGTCAGC 360

ATCCTCTTCC TGTGCTGCAT CTCCTGCGAC CGCTTCGTGG CCGTGGTGTA CGCGCTGGAG 420

AGTCGGGGCC GCCGCCGCCG GAGGACCGCC ATCCTCATCT CCGCCTGCAT CTTCATCCTC 480

GTCGGGATCG TTCACTACCC GGTGTTCCAG ACGGAAGACA AGGAGACCTG CTTTGACATG 540

CTGCAGATGG ACAGCAGGAT TGCCGGGTAC TACTACGCCA GGTTCACCGT TGGCTTTGCC 600

ATCCCTCTCT CCATCATCGC CTTCACCAAC CACCGGATTT TCAGGAGCAT CAAGCAGAGC 660

ATGGGCTTAA GCGCTGCCCA GAAGGCCAAG GTGAAGCACT CGGCCATCGC GGTGGTTGTC 720

ATCTTCCTAG TCTGCTTCGC CCCGTACCAC CTGGTTCTCC TCGTCAAAGC CGCTGCCTTT 780

TCCTACTACA GAGGAGACAG GAACGCCATG TGCGGCTTGG AGGAAAGGCT GTACACAGCC 840

TCTGTGGTGT TTCTGTGCCT GTCCACGGTG AACGGCGTGG CTGACCCCAT TATCTACGTG 900

CTGGCCACGG ACCATTCCCG CCAAGAAGTG TCCAGAATCC ATAAGGGGTG GAAAGAGTGG 960

TCCATGAAGA CAGACGTCAC CAGGCTCACC CACAGCAGGG ACACCGAGGA GCTGCAGTCG1020

CCCGTGGCCC TTGCAGACCA CTACACCTTC TCCAGGCCCG TGCACCCACC AGGGTCACCA1080

TGCCCTGCAA AGAGGCTGAT TGAGGAGTCC TGCTGA 1116

(25) INFORMATION FOR SEQ ID NO:24:

(i) SEQUENCE CHARACTERISTICS:
(A) LENGTH: 371 amino acids

(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:24:

Met Pro Gly Asn Ala Thr Pro Val Thr Thr Thr Ala Pro Trp Ala Ser
1               5                   10                  15

Leu Gly Leu Ser Ala Lys Thr Cys Asn Asn Val Ser Phe Glu Glu Ser
            20                  25                  30

Arg Ile Val Leu Val Val Val Tyr Ser Ala Val Cys Thr Leu Gly Val
        35                  40                  45

Pro Ala Asn Cys Leu Thr Ala Trp Leu Ala Leu Leu Gln Val Leu Gln
    50                  55                  60

Gly Asn Val Leu Ala Val Tyr Leu Leu Cys Leu Ala Leu Cys Glu Leu
65                  70                  75                  80

Leu Tyr Thr Gly Thr Leu Pro Leu Trp Val Ile Tyr Ile Arg Asn Gln
                85                  90                  95

His Arg Trp Thr Leu Gly Leu Leu Ala Ser Lys Val Thr Ala Tyr Ile
            100                 105                 110

Phe Phe Cys Asn Ile Tyr Val Ser Ile Leu Phe Leu Cys Cys Ile Ser
        115                 120                 125

Cys Asp Arg Phe Val Ala Val Val Tyr Ala Leu Glu Ser Arg Gly Arg
    130                 135                 140

Arg Arg Arg Arg Thr Ala Ile Leu Ile Ser Ala Cys Ile Phe Ile Leu
145                 150                 155                 160

Val Gly Ile Val His Tyr Pro Val Phe Gln Thr Glu Asp Lys Glu Thr
                165                 170                 175

Cys Phe Asp Met Leu Gln Met Asp Ser Arg Ile Ala Gly Tyr Tyr Tyr
            180                 185                 190

Ala Arg Phe Thr Val Gly Phe Ala Ile Pro Leu Ser Ile Ile Ala Phe
        195                 200                 205

Thr Asn His Arg Ile Phe Arg Ser Ile Lys Gln Ser Met Gly Leu Ser
    210                 215                 220

Ala Ala Gln Lys Ala Lys Val Lys His Ser Ala Ile Ala Val Val Val
225                 230                 235                 240

Ile Phe Leu Val Cys Phe Ala Pro Tyr His Leu Val Leu Leu Val Lys
                245                 250                 255

Ala Ala Ala Phe Ser Tyr Tyr Arg Gly Asp Arg Asn Ala Met Cys Gly
            260                 265                 270

Leu Glu Glu Arg Leu Tyr Thr Ala Ser Val Val Phe Leu Cys Leu Ser
        275                 280                 285

Thr Val Asn Gly Val Ala Asp Pro Ile Ile Tyr Val Leu Ala Thr Asp
    290                 295                 300

His Ser Arg Gln Glu Val Ser Arg Ile His Lys Gly Trp Lys Glu Trp
305                 310                 315                 320

Ser Met Lys Thr Asp Val Thr Arg Leu Thr His Ser Arg Asp Thr Glu
                325                 330                 335

Glu Leu Gln Ser Pro Val Ala Leu Ala Asp His Tyr Thr Phe Ser Arg
            340                 345                 350

Pro Val His Pro Pro Gly Ser Pro Cys Pro Ala Lys Arg Leu Ile Glu
        355                 360                 365

Glu Ser Cys
    370

(26) INFORMATION FOR SEQ ID NO:25:

     (i) SEQUENCE CHARACTERISTICS:
          (A) LENGTH: 1113 base pairs
          (B) TYPE: nucleic acid
          (C) STRANDEDNESS: single
          (D) TOPOLOGY: linear

    (ii) MOLECULE TYPE: DNA (genomic)

    (xi) SEQUENCE DESCRIPTION: SEQ ID NO:25:

ATGGCGAACT ATAGCCATGC AGCTGACAAC ATTTTGCAAA ATCTCTCGCC TCTAACAGCC   60

TTTCTGAAAC TGACTTCCTT GGGTTTCATA ATAGGAGTCA GCGTGGTGGG CAACCTCCTG  120

ATCTCCATTT TGCTAGTGAA AGATAAGACC TTGCATAGAG CACCTTACTA CTTCCTGTTG  180

GATCTTTGCT GTTCAGATAT CCTCAGATCT GCAATTTGTT TCCCATTTGT GTTCAACTCT  240

GTCAAAAATG GCTCTACCTG GACTTATGGG ACTCTGACTT GCAAAGTGAT TGCCTTTCTG  300

GGGGTTTTGT CCTGTTTCCA CACTGCTTTC ATGCTCTTCT GCATCAGTGT CACCAGATAC  360

TTAGCTATCG CCCATCACCG CTTCTATACA AAGAGGCTGA CCTTTTGGAC GTGTCTGGCT  420

GTGATCTGTA TGGTGTGGAC TCTGTCTGTG GCCATGGCAT TTCCCCCGGT TTTAGACGTG  480

GGCACTTACT CATTCATTAG GGAGGAAGAT CAATGCACCT TCCAACACCG CTCCTTCAGG 540

GCTAATGATT CCTTAGGATT TATGCTGCTT CTTGCTCTCA TCCTCCTAGC CACACAGCTT 600

GTCTACCTCA AGCTGATATT TTTCGTCCAC GATCGAAGAA AAATGAAGCC AGTCCAGTTT 660

GTAGCAGCAG TCAGCCAGAA CTGGACTTTT CATGGTCCTG GAGCCAGTGG CCAGGCAGCT 720

GCCAATTGGC TAGCAGGATT TGGAAGGGGT CCCACACCAC CCACCTTGCT GGGCATCAGG 780

CAAAATGCAA ACACCACAGG CAGAAGAAGG CTATTGGTCT TAGACGAGTT CAAAATGGAG 840

AAAAGAATCA GCAGAATGTT CTATATAATG ACTTTTCTGT TTCTAACCTT GTGGGGCCCC 900

TACCTGGTGG CCTGTTATTG GAGAGTTTTT GCAAGAGGGC CTGTAGTACC AGGGGGATTT 960

CTAACAGCTG CTGTCTGGAT GAGTTTTGCC CAAGCAGGAA TCAATCCTTT TGTCTGCATT1020

TTCTCAAACA GGGAGCTGAG GCGCTGTTTC AGCACAACCC TTCTTTACTG CAGAAAATCC1080

AGGTTACCAA GGGAACCTTA CTGTGTTATA TGA 1113

(27) INFORMATION FOR SEQ ID NO:26:

(i) SEQUENCE CHARACTERISTICS:
(A) LENGTH: 370 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:26:

Met Ala Asn Tyr Ser His Ala Ala Asp Asn Ile Leu Gln Asn Leu Ser
1               5                   10                  15

Pro Leu Thr Ala Phe Leu Lys Leu Thr Ser Leu Gly Phe Ile Ile Gly
            20                  25                  30

Val Ser Val Val Gly Asn Leu Leu Ile Ser Ile Leu Leu Val Lys Asp
        35                  40                  45

Lys Thr Leu His Arg Ala Pro Tyr Tyr Phe Leu Leu Asp Leu Cys Cys
    50                  55                  60

Ser Asp Ile Leu Arg Ser Ala Ile Cys Phe Pro Phe Val Phe Asn Ser
65                  70                  75                  80

Val Lys Asn Gly Ser Thr Trp Thr Tyr Gly Thr Leu Thr Cys Lys Val
                85                  90                  95

Ile Ala Phe Leu Gly Val Leu Ser Cys Phe His Thr Ala Phe Met Leu

                100                 105                 110

Phe Cys Ile Ser Val Thr Arg Tyr Leu Ala Ile Ala His His Arg Phe
        115                 120                 125

Tyr Thr Lys Arg Leu Thr Phe Trp Thr Cys Leu Ala Val Ile Cys Met
    130                 135                 140

Val Trp Thr Leu Ser Val Ala Met Ala Phe Pro Pro Val Leu Asp Val
145                 150                 155                 160

Gly Thr Tyr Ser Phe Ile Arg Glu Glu Asp Gln Cys Thr Phe Gln His
                165                 170                 175

Arg Ser Phe Arg Ala Asn Asp Ser Leu Gly Phe Met Leu Leu Leu Ala
            180                 185                 190

Leu Ile Leu Leu Ala Thr Gln Leu Val Tyr Leu Lys Leu Ile Phe Phe
        195                 200                 205

Val His Asp Arg Arg Lys Met Lys Pro Val Gln Phe Val Ala Ala Val
    210                 215                 220

Ser Gln Asn Trp Thr Phe His Gly Pro Gly Ala Ser Gly Gln Ala Ala
225                 230                 235                 240

Ala Asn Trp Leu Ala Gly Phe Gly Arg Gly Pro Thr Pro Pro Thr Leu
                245                 250                 255

Leu Gly Ile Arg Gln Asn Ala Asn Thr Thr Gly Arg Arg Arg Leu Leu
            260                 265                 270

Val Leu Asp Glu Phe Lys Met Glu Lys Arg Ile Ser Arg Met Phe Tyr
        275                 280                 285

Ile Met Thr Phe Leu Phe Leu Thr Leu Trp Gly Pro Tyr Leu Val Ala
    290                 295                 300

Cys Tyr Trp Arg Val Phe Ala Arg Gly Pro Val Val Pro Gly Gly Phe
305                 310                 315                 320

Leu Thr Ala Ala Val Trp Met Ser Phe Ala Gln Ala Gly Ile Asn Pro
                325                 330                 335

Phe Val Cys Ile Phe Ser Asn Arg Glu Leu Arg Arg Cys Phe Ser Thr
            340                 345                 350

Thr Leu Leu Tyr Cys Arg Lys Ser Arg Leu Pro Arg Glu Pro Tyr Cys
        355                 360                 365

Val Ile
    370

(28) INFORMATION FOR SEQ ID NO:27:

(i) SEQUENCE CHARACTERISTICS:
(A) LENGTH: 1080 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:27:

ATGCAGGTCC CGAACAGCAC CGGCCCGGAC AACGCGACGC TGCAGATGCT GCGGAACCCG 60

GCGATCGCGG TGGCCCTGCC CGTGGTGTAC TCGCTGGTGG CGGCGGTCAG CATCCCGGGC 120

AACCTCTTCT CTCTGTGGGT GCTGTGCCGG CGCATGGGGC CCAGATCCCC GTCGGTCATC 180

TTCATGATCA ACCTGAGCGT CACGGACCTG ATGCTGGCCA GCGTGTTGCC TTTCCAAATC 240

TACTACCATT GCAACCGCCA CCACTGGGTA TTCGGGGTGC TGCTTTGCAA CGTGGTGACC 300

GTGGCCTTTT ACGCAAACAT GTATTCCAGC ATCCTCACCA TGACCTGTAT CAGCGTGGAG 360

CGCTTCCTGG GGGTCCTGTA CCCGCTCAGC TCCAAGCGCT GGCGCCGCCG TCGTTACGCG 420

GTGGCCGCGT GTGCAGGGAC CTGGCTGCTG CTCCTGACCG CCCTGTGCCC GCTGGCGCGC 480

ACCGATCTCA CCTACCCGGT GCACGCCCTG GGCATCATCA CCTGCTTCGA CGTCCTCAAG 540

TGGACGATGC TCCCCAGCGT GGCCATGTGG GCCGTGTTCC TCTTCACCAT CTTCATCCTG 600

CTGTTCCTCA TCCCGTTCGT GATCACCGTG GCTTGTTACA CGGCCACCAT CCTCAAGCTG 660

TTGCGCACGG AGGAGGCGCA CGGCCGGGAG CAGCGGAGGC GCGCGGTGGG CCTGGCCGCG 720

GTGGTCTTGC TGGCCTTTGT CACCTGCTTC GCCCCCAACA ACTTCGTGCT CCTGGCGCAC 780

ATCGTGAGCC GCCTGTTCTA CGGCAAGAGC TACTACCACG TGTACAAGCT CACGCTGTGT 840

CTCAGCTGCC TCAACAACTG TCTGGACCCG TTTGTTTATT ACTTTGCGTC CCGGGAATTC 900

CAGCTGCGCC TGCGGGAATA TTTGGGCTGC CGCCGGGTGC CCAGAGACAC CCTGGACACG 960

CGCCGCGAGA GCCTCTTCTC CGCCAGGACC ACGTCCGTGC GCTCCGAGGC CGGTGCGCAC1020

CCTGAAGGGA TGGAGGGAGC CACCAGGCCC GGCCTCCAGA GGCAGGAGAG TGTGTTCTGA1080

(29) INFORMATION FOR SEQ ID NO:28:

(i) SEQUENCE CHARACTERISTICS:
(A) LENGTH: 359 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:28:

Met Gln Val Pro Asn Ser Thr Gly Pro Asp Asn Ala Thr Leu Gln Met
1               5                   10                  15

Leu Arg Asn Pro Ala Ile Ala Val Ala Leu Pro Val Val Tyr Ser Leu
            20                  25                  30

Val Ala Ala Val Ser Ile Pro Gly Asn Leu Phe Ser Leu Trp Val Leu
        35                  40                  45

Cys Arg Arg Met Gly Pro Arg Ser Pro Ser Val Ile Phe Met Ile Asn
    50                  55                  60

Leu Ser Val Thr Asp Leu Met Leu Ala Ser Val Leu Pro Phe Gln Ile
65                  70                  75                  80

Tyr Tyr His Cys Asn Arg His His Trp Val Phe Gly Val Leu Leu Cys
                85                  90                  95

Asn Val Val Thr Val Ala Phe Tyr Ala Asn Met Tyr Ser Ser Ile Leu
            100                 105                 110

Thr Met Thr Cys Ile Ser Val Glu Arg Phe Leu Gly Val Leu Tyr Pro
        115                 120                 125

Leu Ser Ser Lys Arg Trp Arg Arg Arg Arg Tyr Ala Val Ala Ala Cys
    130                 135                 140

Ala Gly Thr Trp Leu Leu Leu Leu Thr Ala Leu Cys Pro Leu Ala Arg
145                 150                 155                 160

Thr Asp Leu Thr Tyr Pro Val His Ala Leu Gly Ile Ile Thr Cys Phe
                165                 170                 175

Asp Val Leu Lys Trp Thr Met Leu Pro Ser Val Ala Met Trp Ala Val
            180                 185                 190

Phe Leu Phe Thr Ile Phe Ile Leu Leu Phe Leu Ile Pro Phe Val Ile
        195                 200                 205

Thr Val Ala Cys Tyr Thr Ala Thr Ile Leu Lys Leu Leu Arg Thr Glu
    210                 215                 220

Glu Ala His Gly Arg Glu Gln Arg Arg Arg Ala Val Gly Leu Ala Ala
225                 230                 235                 240

Val Val Leu Leu Ala Phe Val Thr Cys Phe Ala Pro Asn Asn Phe Val
                245                 250                 255

Leu Leu Ala His Ile Val Ser Arg Leu Phe Tyr Gly Lys Ser Tyr Tyr
            260                 265                 270

His Val Tyr Lys Leu Thr Leu Cys Leu Ser Cys Leu Asn Asn Cys Leu
        275                 280                 285

Asp Pro Phe Val Tyr Tyr Phe Ala Ser Arg Glu Phe Gln Leu Arg Leu
    290                 295                 300

Arg Glu Tyr Leu Gly Cys Arg Arg Val Pro Arg Asp Thr Leu Asp Thr
305                 310                 315                 320

Arg Arg Glu Ser Leu Phe Ser Ala Arg Thr Thr Ser Val Arg Ser Glu
                325                 330                 335

Ala Gly Ala His Pro Glu Gly Met Glu Gly Ala Thr Arg Pro Gly Leu
            340                 345                 350

Gln Arg Gln Glu Ser Val Phe
        355

(30) INFORMATION FOR SEQ ID NO:29:

(i) SEQUENCE CHARACTERISTICS:
(A) LENGTH: 1503 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:29:

ATGGAGCGTC CCTGGGAGGA CAGCCCAGGC CCGGAGGGGG CAGCTGAGGG CTCGCCTGTG 60

CCAGTCGCCG CCGGGGCGCG CTCCGGTGCC GCGGCGAGTG GCACAGGCTG GCAGCCATGG 120

GCTGAGTGCC CGGGACCCAA GGGGAGGGGG CAACTGCTGG CGACCGCCGG CCCTTTGCGT 180

CGCTGGCCCG CCCCCTCGCC TGCCAGCTCC AGCCCCGCCC CCGGAGCGGC GTCCGCTCAC 240

TCGGTTCAAG GCAGCGCGAC TGCGGGTGGC GCACGACCAG GGCGCAGACC TTGGGGCGCG 300

CGGCCCATGG AGTCGGGGCT GCTGCGGCCG GCGCCGGTGA GCGAGGTCAT CGTCCTGCAT 360

TACAACTACA CCGGCAAGCT CCGCGGTGCG AGCTACCAGC CGGGTGCCGG CCTGCGCGCC 420

GACGCCGTGG TGTGCCTGGC GGTGTGCGCC TTCATCGTGC TAGAGAATCT AGCCGTGTTG 480

TTGGTGCTCG GACGCCACCC GCGCTTCCAC GCTCCCATGT TCCTGCTCCT GGGCAGCCTC 540

ACGTTGTCGG ATCTGCTGGC AGGCGCCGCC TACGCCGCCA ACATCCTACT GTCGGGGCCG 600

CTCACGCTGA AACTGTCCCC CGCGCTCTGG TTCGCACGGG AGGGAGGCGT CTTCGTGGCA 660

CTCACTGCGT CCGTGCTGAG CCTCCTGGCC ATCGCGCTGG AGCGCAGCCT CACCATGGCG 720

CGCAGGGGGC CCGCGCCCGT CTCCAGTCGG GGGCGCACGC TGGCGATGGC AGCCGCGGCC 780

TGGGGCGTGT CGCTGCTCCT CGGGCTCCTG CCAGCGCTGG GCTGGAATTG CCTGGGTCGC 840

CTGGACGCTT GCTCCACTGT CTTGCCGCTC TACGCCAAGG CCTACGTGCT CTTCTGCGTG 900

CTCGCCTTCG TGGGCATCCT GGCCGCGATC TGTGCACTCT ACGCGCGCAT CTACTGCCAG 960

GTACGCGCCA ACGCGCGGCG CCTGCCGGCA CGGCCCGGGA CTGCGGGGAC CACCTCGACC1020

CGGGCGCGTC GCAAGCCGCG CTCTCTGGCC TTGCTGCGCA CGCTCAGCGT GGTGCTCCTG1080

GCCTTTGTGG CATGTTGGGG CCCCCTCTTC CTGCTGCTGT TGCTCGACGT GGCGTGCCCG1140

GCGCGCACCT GTCCTGTACT CCTGCAGGCC GATCCCTTCC TGGGACTGGC CATGGCCAAC1200

TCACTTCTGA ACCCCATCAT CTACACGCTC ACCAACCGCG ACCTGCGCCA CGCGCTCCTG1260

CGCCTGGTCT GCTGCGGACG CCACTCCTGC GGCAGAGACC CGAGTGGCTC CCAGCAGTCG1320

GCGAGCGCGG CTGAGGCTTC CGGGGGCCTG CGCCGCTGCC TGCCCCCGGG CCTTGATGGG1380

AGCTTCAGCG GCTCGGAGCG CTCATCGCCC CAGCGCGACG GGCTGGACAC CAGCGGCTCC1440

ACAGGCAGCC CCGGTGCACC CACAGCCGCC CGGACTCTGG TATCAGAACC GGCTGCAGAC1500

TGA 1503

(31) INFORMATION FOR SEQ ID NO:30:

(i) SEQUENCE CHARACTERISTICS:
(A) LENGTH: 500 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:30:

Met Glu Arg Pro Trp Glu Asp Ser Pro Gly Pro Glu Gly Ala Ala Glu
1               5                   10                  15

Gly Ser Pro Val Pro Val Ala Ala Gly Ala Arg Ser Gly Ala Ala Ala
            20                  25                  30

Ser Gly Thr Gly Trp Gln Pro Trp Ala Glu Cys Pro Gly Pro Lys Gly
        35                  40                  45

Arg Gly Gln Leu Leu Ala Thr Ala Gly Pro Leu Arg Arg Trp Pro Ala
    50                  55                  60

Pro Ser Pro Ala Ser Ser Ser Pro Ala Pro Gly Ala Ala Ser Ala His
65                  70                  75                  80

Ser Val Gln Gly Ser Ala Thr Ala Gly Gly Ala Arg Pro Gly Arg Arg
                85                  90                  95

Pro Trp Gly Ala Arg Pro Met Glu Ser Gly Leu Leu Arg Pro Ala Pro
            100                 105                 110

Val Ser Glu Val Ile Val Leu His Tyr Asn Tyr Thr Gly Lys Leu Arg
        115                 120                 125

Gly Ala Ser Tyr Gln Pro Gly Ala Gly Leu Arg Ala Asp Ala Val Val
    130                 135                 140

Cys Leu Ala Val Cys Ala Phe Ile Val Leu Glu Asn Leu Ala Val Leu
145                 150                 155                 160

Leu Val Leu Gly Arg His Pro Arg Phe His Ala Pro Met Phe Leu Leu
                165                 170                 175

Leu Gly Ser Leu Thr Leu Ser Asp Leu Leu Ala Gly Ala Ala Tyr Ala
            180                 185                 190

Ala Asn Ile Leu Leu Ser Gly Pro Leu Thr Leu Lys Leu Ser Pro Ala
        195                 200                 205

Leu Trp Phe Ala Arg Glu Gly Gly Val Phe Val Ala Leu Thr Ala Ser
    210                 215                 220

Val Leu Ser Leu Leu Ala Ile Ala Leu Glu Arg Ser Leu Thr Met Ala
225                 230                 235                 240

Arg Arg Gly Pro Ala Pro Val Ser Ser Arg Gly Arg Thr Leu Ala Met
                245                 250                 255

Ala Ala Ala Ala Trp Gly Val Ser Leu Leu Leu Gly Leu Leu Pro Ala
            260                 265                 270

Leu Gly Trp Asn Cys Leu Gly Arg Leu Asp Ala Cys Ser Thr Val Leu
        275                 280                 285

Pro Leu Tyr Ala Lys Ala Tyr Val Leu Phe Cys Val Leu Ala Phe Val
    290                 295                 300

Gly Ile Leu Ala Ala Ile Cys Ala Leu Tyr Ala Arg Ile Tyr Cys Gln
305                 310                 315                 320

Val Arg Ala Asn Ala Arg Arg Leu Pro Ala Arg Pro Gly Thr Ala Gly
                325                 330                 335

Thr Thr Ser Thr Arg Ala Arg Arg Lys Pro Arg Ser Leu Ala Leu Leu
            340                 345                 350

Arg Thr Leu Ser Val Val Leu Leu Ala Phe Val Ala Cys Trp Gly Pro
        355                 360                 365

Leu Phe Leu Leu Leu Leu Leu Asp Val Ala Cys Pro Ala Arg Thr Cys

370                 375                 380

Pro Val Leu Leu Gln Ala Asp Pro Phe Leu Gly Leu Ala Met Ala Asn
385                 390                 395                 400

Ser Leu Leu Asn Pro Ile Ile Tyr Thr Leu Thr Asn Arg Asp Leu Arg
                405                 410                 415

His Ala Leu Leu Arg Leu Val Cys Cys Gly Arg His Ser Cys Gly Arg
            420                 425                 430

Asp Pro Ser Gly Ser Gln Gln Ser Ala Ser Ala Ala Glu Ala Ser Gly
        435                 440                 445

Gly Leu Arg Arg Cys Leu Pro Pro Gly Leu Asp Gly Ser Phe Ser Gly
    450                 455                 460

Ser Glu Arg Ser Ser Pro Gln Arg Asp Gly Leu Asp Thr Ser Gly Ser
465                 470                 475                 480

Thr Gly Ser Pro Gly Ala Pro Thr Ala Ala Arg Thr Leu Val Ser Glu
                485                 490                 495

Pro Ala Ala Asp
            500

(32) INFORMATION FOR SEQ ID NO:31:

(i) SEQUENCE CHARACTERISTICS:
(A) LENGTH: 1029 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:31:

ATGCAAGCCG TCGACAATCT CACCTCTGCG CCTGGGAACA CCAGTCTGTG CACCAGAGAC 60

TACAAAATCA CCCAGGTCCT CTTCCCACTG CTCTACACTG TCCTGTTTTT TGTTGGACTT 120

ATCACAAATG GCCTGGCGAT GAGGATTTTC TTTCAAATCC GGAGTAAATC AAACTTTATT 180

ATTTTTCTTA AGAACACAGT CATTTCTGAT CTTCTCATGA TTCTGACTTT TCCATTCAAA 240

ATTCTTAGTG ATGCCAAACT GGGAACAGGA CCACTGAGAA CTTTTGTGTG TCAAGTTACC 300

TCCGTCATAT TTTATTTCAC AATGTATATC AGTATTTCAT TCCTGGGACT GATAACTATC 360

GATCGCTACC AGAAGACCAC CAGGCCATTT AAAACATCCA ACCCCAAAAA TCTCTTGGGG 420

GCTAAGATTC TCTCTGTTGT CATCTGGGCA TTCATGTTCT TACTCTCTTT GCCTAACATG 480

ATTCTGACCA ACAGGCAGCC GAGAGACAAG AATGTGAAGA AATGCTCTTT CCTTAAATCA 540

GAGTTCGGTC TAGTCTGGCA TGAAATAGTA AATTACATCT GTCAAGTCAT TTTCTGGATT 600

AATTTCTTAA TTGTTATTGT ATGTTATACA CTCATTACAA AAGAACTGTA CCGGTCATAC 660

GTAAGAACGA GGGGTGTAGG TAAAGTCCCC AGGAAAAAGG TGAACGTCAA AGTTTTCATT 720

ATCATTGCTG TATTCTTTAT TTGTTTTGTT CCTTTCCATT TTGCCCGAAT TCCTTACACC 780

CTGAGCCAAA CCCGGGATGT CTTTGACTGC ACTGCTGAAA ATACTCTGTT CTATGTGAAA 840

GAGAGCACTC TGTGGTTAAC TTCCTTAAAT GCATGCCTGG ATCCGTTCAT CTATTTTTTC 900

CTTTGCAAGT CCTTCAGAAA TTCCTTGATA AGTATGCTGA AGTGCCCCAA TTCTGCAACA 960

TCTCTGTCCC AGGACAATAG GAAAAAAGAA CAGGATGGTG GTGACCCAAA TGAAGAGACT1020

CCAATGTAA 1029

(33) INFORMATION FOR SEQ ID NO:32:

(i) SEQUENCE CHARACTERISTICS:
(A) LENGTH: 342 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:32:

(34) INFORMATION FOR SEQ ID NO:33:

     (i) SEQUENCE CHARACTERISTICS:
          (A) LENGTH: 1077 base pairs
          (B) TYPE: nucleic acid
          (C) STRANDEDNESS: single
          (D) TOPOLOGY: linear

    (ii) MOLECULE TYPE: DNA (genomic)

(xi) SEQUENCE DESCRIPTION: SEQ ID NO:33:

ATGTCGGTCT GCTACCGTCC CCCAGGGAAC GAGACACTGC TGAGCTGGAA GACTTCGCGG 60

GCCACAGGCA CAGCCTTCCT GCTGCTGGCG GCGCTGCTGG GGCTGCCTGG CAACGGCTTC 120

GTGGTGTGGA GCTTGGCGGG CTGGCGGCCT GCACGGGGGC GACCGCTGGC GGCCACGCTT 180

GTGCTGCACC TGGCGCTGGC CGACGGCGCG GTGCTGCTGC TCACGCCGCT CTTTGTGGCC 240

TTCCTGACCC GGCAGGCCTG GCCGCTGGGC CAGGCGGGCT GCAAGGCGGT GTACTACGTG 300

TGCGCGCTCA GCATGTACGC CAGCGTGCTG CTCACCGGCC TGCTCAGCCT GCAGCGCTGC 360

CTCGCAGTCA CCCGCCCCTT CCTGGCGCCT CGGCTGCGCA GCCCGGCCCT GGCCCGCCGC 420

CTGCTGCTGG CGGTCTGGCT GGCCGCCCTG TTGCTCGCCG TCCCGGCCGC CGTCTACCGC 480

CACCTGTGGA GGGACCGCGT ATGCCAGCTG TGCCACCCGT CGCCGGTCCA CGCCGCCGCC 540

CACCTGAGCC TGGAGACTCT GACCGCTTTC GTGCTTCCTT TCGGGCTGAT GCTCGGCTGC 600

TACAGCGTGA CGCTGGCACG GCTGCGGGGC GCCCGCTGGG GCTCCGGGCG GCACGGGGCG 660

CGGGTGGGCC GGCTGGTGAG CGCCATCGTG CTTGCCTTCG GCTTGCTCTG GGCCCCCTAC 720

CACGCAGTCA ACCTTCTGCA GGCGGTCGCA GCGCTGGCTC CACCGGAAGG GGCCTTGGCG 780

AAGCTGGGCG GAGCCGGCCA GGCGGCGCGA GCGGGAACTA CGGCCTTGGC CTTCTTCAGT 840

TCTAGCGTCA ACCCGGTGCT CTACGTCTTC ACCGCTGGAG ATCTGCTGCC CCGGGCAGGT 900

CCCCGTTTCC TCACGCGGCT CTTCGAAGGC TCTGGGGAGG CCCGAGGGGG CGGCCGCTCT 960

AGGGAAGGGA CCATGGAGCT CCGAACTACC CCTCAGCTGA AAGTGGTGGG GCAGGGCCGC1020

GGCAATGGAG ACCCGGGGGG TGGGATGGAG AAGGACGGTC CGGAATGGGA CCTTTGA 1077

(35) INFORMATION FOR SEQ ID NO:34:

(i) SEQUENCE CHARACTERISTICS:
(A) LENGTH: 358 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:34:

Met Ser Val Cys Tyr Arg Pro Pro Gly Asn Glu Thr Leu Leu Ser Trp
1               5                   10                  15

Lys Thr Ser Arg Ala Thr Gly Thr Ala Phe Leu Leu Leu Ala Ala Leu

            20                  25                  30

Leu Gly Leu Pro Gly Asn Gly Phe Val Val Trp Ser Leu Ala Gly Trp
        35                  40                  45

Arg Pro Ala Arg Gly Arg Pro Leu Ala Ala Thr Leu Val Leu His Leu
    50                  55                  60

Ala Leu Ala Asp Gly Ala Val Leu Leu Leu Thr Pro Leu Phe Val Ala
65                  70                  75                  80

Phe Leu Thr Arg Gln Ala Trp Pro Leu Gly Gln Ala Gly Cys Lys Ala
                85                  90                  95

Val Tyr Tyr Val Cys Ala Leu Ser Met Tyr Ala Ser Val Leu Leu Thr
            100                 105                 110

Gly Leu Leu Ser Leu Gln Arg Cys Leu Ala Val Thr Arg Pro Phe Leu
        115                 120                 125

Ala Pro Arg Leu Arg Ser Pro Ala Leu Ala Arg Arg Leu Leu Leu Ala
    130                 135                 140

Val Trp Leu Ala Ala Leu Leu Leu Ala Val Pro Ala Ala Val Tyr Arg
145                 150                 155                 160

His Leu Trp Arg Asp Arg Val Cys Gln Leu Cys His Pro Ser Pro Val
                165                 170                 175

His Ala Ala Ala His Leu Ser Leu Glu Thr Leu Thr Ala Phe Val Leu
            180                 185                 190

Pro Phe Gly Leu Met Leu Gly Cys Tyr Ser Val Thr Leu Ala Arg Leu
        195                 200                 205

Arg Gly Ala Arg Trp Gly Ser Gly Arg His Gly Ala Arg Val Gly Arg
    210                 215                 220

Leu Val Ser Ala Ile Val Leu Ala Phe Gly Leu Leu Trp Ala Pro Tyr
225                 230                 235                 240

His Ala Val Asn Leu Leu Gln Ala Val Ala Ala Leu Ala Pro Pro Glu
                245                 250                 255

Gly Ala Leu Ala Lys Leu Gly Gly Ala Gly Gln Ala Ala Arg Ala Gly
            260                 265                 270

Thr Thr Ala Leu Ala Phe Phe Ser Ser Ser Val Asn Pro Val Leu Tyr
        275                 280                 285

Val Phe Thr Ala Gly Asp Leu Leu Pro Arg Ala Gly Pro Arg Phe Leu
    290                 295                 300

Thr Arg Leu Phe Glu Gly Ser Gly Glu Ala Arg Gly Gly Gly Arg Ser
305                 310                 315                 320

Arg Glu Gly Thr Met Glu Leu Arg Thr Thr Pro Gln Leu Lys Val Val
                325                 330                 335

Gly Gln Gly Arg Gly Asn Gly Asp Pro Gly Gly Gly Met Glu Lys Asp
            340                 345                 350

Gly Pro Glu Trp Asp Leu
        355

(36) INFORMATION FOR SEQ ID NO:35:

(i) SEQUENCE CHARACTERISTICS:
(A) LENGTH: 1005 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:35:

ATGCTGGGGA TCATGGCATG GAATGCAACT TGCAAAAACT GGCTGGCAGC AGAGGCTGCC 60

CTGGAAAAGT ACTACCTTTC CATTTTTTAT GGGATTGAGT TCGTTGTGGG AGTCCTTGGA 120

AATACCATTG TTGTTTACGG CTACATCTTC TCTCTGAAGA ACTGGAACAG CAGTAATATT 180

TATCTCTTTA ACCTCTCTGT CTCTGACTTA GCTTTTCTGT GCACCCTCCC CATGCTGATA 240

AGGAGTTATG CCAATGGAAA CTGGATATAT GGAGACGTGC TCTGCATAAG CAACCGATAT 300

GTGCTTCATG CCAACCTCTA TACCAGCATT CTCTTTCTCA CTTTTATCAG CATAGATCGA 360

TACTTGATAA TTAAGTATCC TTTCCGAGAA CACCTTCTGC AAAAGAAAGA GTTTGCTATT 420

TTAATCTCCT TGGCCATTTG GGTTTTAGTA ACCTTAGAGT TACTACCCAT ACTTCCCCTT 480

ATAAATCCTG TTATAACTGA CAATGGCACC ACCTGTAATG ATTTTGCAAG TTCTGGAGAC 540

CCCAACTACA ACCTCATTTA CAGCATGTGT CTAACACTGT TGGGGTTCCT TATTCCTCTT 600

TTTGTGATGT GTTTCTTTTA TTACAAGATT GCTCTCTTCC TAAAGCAGAG GAATAGGCAG 660

GTTGCTACTG CTCTGCCCCT TGAAAAGCCT CTCAACTTGG TCATCATGGC AGTGGTAATC 720

TTCTCTGTGC TTTTTACACC CTATCACGTC ATGCGGAATG TGAGGATCGC TTCACGCCTG 780

GGGAGTTGGA AGCAGTATCA GTGCACTCAG GTCGTCATCA ACTCCTTTTA CATTGTGACA 840

CGGCCTTTGG CCTTTCTGAA CAGTGTCATC AACCCTGTCT TCTATTTTCT TTTGGGAGAT 900

CACTTCAGGG ACATGCTGAT GAATCAACTG AGACACAACT TCAAATCCCT TACATCCTTT 960

AGCAGATGGG CTCATGAACT CCTACTTTCA TTCAGAGAAA AGTGA 1005

(37) INFORMATION FOR SEQ ID NO:36:

(i) SEQUENCE CHARACTERISTICS:
(A) LENGTH: 334 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:36:

Met Leu Gly Ile Met Ala Trp Asn Ala Thr Cys Lys Asn Trp Leu Ala
1               5                   10                  15

Ala Glu Ala Ala Leu Glu Lys Tyr Tyr Leu Ser Ile Phe Tyr Gly Ile
            20                  25                  30

Glu Phe Val Val Gly Val Leu Gly Asn Thr Ile Val Val Tyr Gly Tyr
        35                  40                  45

Ile Phe Ser Leu Lys Asn Trp Asn Ser Ser Asn Ile Tyr Leu Phe Asn
    50                  55                  60

Leu Ser Val Ser Asp Leu Ala Phe Leu Cys Thr Leu Pro Met Leu Ile
65                  70                  75                  80

Arg Ser Tyr Ala Asn Gly Asn Trp Ile Tyr Gly Asp Val Leu Cys Ile
                85                  90                  95

Ser Asn Arg Tyr Val Leu His Ala Asn Leu Tyr Thr Ser Ile Leu Phe
            100                 105                 110

Leu Thr Phe Ile Ser Ile Asp Arg Tyr Leu Ile Ile Lys Tyr Pro Phe
        115                 120                 125

Arg Glu His Leu Leu Gln Lys Lys Glu Phe Ala Ile Leu Ile Ser Leu
    130                 135                 140

Ala Ile Trp Val Leu Val Thr Leu Glu Leu Leu Pro Ile Leu Pro Leu
145                 150                 155                 160

Ile Asn Pro Val Ile Thr Asp Asn Gly Thr Thr Cys Asn Asp Phe Ala
                165                 170                 175

Ser Ser Gly Asp Pro Asn Tyr Asn Leu Ile Tyr Ser Met Cys Leu Thr
            180                 185                 190

Leu Leu Gly Phe Leu Ile Pro Leu Phe Val Met Cys Phe Phe Tyr Tyr
        195                 200                 205

Lys Ile Ala Leu Phe Leu Lys Gln Arg Asn Arg Gln Val Ala Thr Ala
    210                 215                 220

Leu Pro Leu Glu Lys Pro Leu Asn Leu Val Ile Met Ala Val Val Ile
225                 230                 235                 240

Phe Ser Val Leu Phe Thr Pro Tyr His Val Met Arg Asn Val Arg Ile
                245                 250                 255

Ala Ser Arg Leu Gly Ser Trp Lys Gln Tyr Gln Cys Thr Gln Val Val
            260                 265                 270

Ile Asn Ser Phe Tyr Ile Val Thr Arg Pro Leu Ala Phe Leu Asn Ser
        275                 280                 285

Val Ile Asn Pro Val Phe Tyr Phe Leu Leu Gly Asp His Phe Arg Asp
    290                 295                 300

Met Leu Met Asn Gln Leu Arg His Asn Phe Lys Ser Leu Thr Ser Phe
305                 310                 315                 320

Ser Arg Trp Ala His Glu Leu Leu Leu Ser Phe Arg Glu Lys
                325                 330

(38) INFORMATION FOR SEQ ID NO:37:

(i) SEQUENCE CHARACTERISTICS:
(A) LENGTH: 1296 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:37:

ATGCAGGCGC TTAACATTAC CCCGGAGCAG TTCTCTCGGC TGCTGCGGGA CCACAACCTG 60

ACGCGGGAGC AGTTCATCGC TCTGTACCGG CTGCGACCGC TCGTCTACAC CCCAGAGCTG 120

CCGGGACGCG CCAAGCTGGC CCTCGTGCTC ACCGGCGTGC TCATCTTCGC CCTGGCGCTC 180

TTTGGCAATG CTCTGGTGTT CTACGTGGTG ACCCGCAGCA AGGCCATGCG CACCGTCACC 240

AACATCTTTA TCTGCTCCTT GGCGCTCAGT GACCTGCTCA TCACCTTCTT CTGCATTCCC 300

GTCACCATGC TCCAGAACAT TTCCGACAAC TGGCTGGGGG GTGCTTTCAT TTGCAAGATG 360

GTGCCATTTG TCCAGTCTAC CGCTGTTGTG ACAGAAATGC TCACTATGAC CTGCATTGCT 420

GTGGAAAGGC ACCAGGGACT TGTGCATCCT TTTAAAATGA AGTGGCAATA CACCAACCGA 480

AGGGCTTTCA CAATGCTAGG TGTGGTCTGG CTGGTGGCAG TCATCGTAGG ATCACCCATG 540

TGGCACGTGC AACAACTTGA GATCAAATAT GACTTCCTAT ATGAAAAGGA ACACATCTGC 600

TGCTTAGAAG AGTGGACCAG CCCTGTGCAC CAGAAGATCT ACACCACCTT CATCCTTGTC 660

ATCCTCTTCC TCCTGCCTCT TATGGTGATG CTTATTCTGT ACAGTAAAAT TGGTTATGAA 720

CTTTGGATAA AGAAAAGAGT TGGGGATGGT TCAGTGCTTC GAACTATTCA TGGAAAAGAA 780

ATGTCCAAAA TAGCCAGGAA GAAGAAACGA GCTGTCATTA TGATGGTGAC AGTGGTGGCT 840

CTCTTTGCTG TGTGCTGGGC ACCATTCCAT GTTGTCCATA TGATGATTGA ATACAGTAAT 900

TTTGAAAAGG AATATGATGA TGTCACAATC AAGATGATTT TTGCTATCGT GCAAATTATT 960

GGATTTTCCA ACTCCATCTG TAATCCCATT GTCTATGCAT TTATGAATGA AAACTTCAAA1020

AAAAATGTTT TGTCTGCAGT TTGTTATTGC ATAGTAAATA AAACCTTCTC TCCAGCACAA1080

AGGCATGGAA ATTCAGGAAT TACAATGATG CGGAAGAAAG CAAAGTTTTC CCTCAGAGAG1140

AATCCAGTGG AGGAAACCAA AGGAGAAGCA TTCAGTGATG GCAACATTGA AGTCAAATTG1200

TGTGAACAGA CAGAGGAGAA GAAAAAGCTC AAACGACATC TTGCTCTCTT TAGGTCTGAA1260

CTGGCTGAGA ATTCTCCTTT AGACAGTGGG CATTAA 1296

(39) INFORMATION FOR SEQ ID NO:38:

(i) SEQUENCE CHARACTERISTICS:
(A) LENGTH: 431 amino acids
(B) TYPE: amino acid
(C) STRANDEDNESS:
(D) TOPOLOGY: not relevant

(ii) MOLECULE TYPE: protein

(xi) SEQUENCE DESCRIPTION: SEQ ID NO:38:

Met Gln Ala Leu Asn Ile Thr Pro Glu Gln Phe Ser Arg Leu Leu Arg
1               5                   10                  15

Asp His Asn Leu Thr Arg Glu Gln Phe Ile Ala Leu Tyr Arg Leu Arg
            20                  25                  30

Pro Leu Val Tyr Thr Pro Glu Leu Pro Gly Arg Ala Lys Leu Ala Leu
        35                  40                  45

Val Leu Thr Gly Val Leu Ile Phe Ala Leu Ala Leu Phe Gly Asn Ala
    50                  55                  60

Leu Val Phe Tyr Val Val Thr Arg Ser Lys Ala Met Arg Thr Val Thr
65                  70                  75                  80

Asn Ile Phe Ile Cys Ser Leu Ala Leu Ser Asp Leu Leu Ile Thr Phe
                85                  90                  95

Phe Cys Ile Pro Val Thr Met Leu Gln Asn Ile Ser Asp Asn Trp Leu

            100                 105                 110

Gly Gly Ala Phe Ile Cys Lys Met Val Pro Phe Val Gln Ser Thr Ala
        115                 120                 125

Val Val Thr Glu Met Leu Thr Met Thr Cys Ile Ala Val Glu Arg His
    130                 135                 140

Gln Gly Leu Val His Pro Phe Lys Met Lys Trp Gln Tyr Thr Asn Arg
145                 150                 155                 160

Arg Ala Phe Thr Met Leu Gly Val Val Trp Leu Val Ala Val Ile Val
                165                 170                 175

Gly Ser Pro Met Trp His Val Gln Gln Leu Glu Ile Lys Tyr Asp Phe
            180                 185                 190

Leu Tyr Glu Lys Glu His Ile Cys Cys Leu Glu Glu Trp Thr Ser Pro
        195                 200                 205

Val His Gln Lys Ile Tyr Thr Thr Phe Ile Leu Val Ile Leu Phe Leu
    210                 215                 220

Leu Pro Leu Met Val Met Leu Ile Leu Tyr Ser Lys Ile Gly Tyr Glu
225                 230                 235                 240

Leu Trp Ile Lys Lys Arg Val Gly Asp Gly Ser Val Leu Arg Thr Ile
                245                 250                 255

His Gly Lys Glu Met Ser Lys Ile Ala Arg Lys Lys Lys Arg Ala Val
            260                 265                 270

Ile Met Met Val Thr Val Val Ala Leu Phe Ala Val Cys Trp Ala Pro
        275                 280                 285

Phe His Val Val His Met Met Ile Glu Tyr Ser Asn Phe Glu Lys Glu
    290                 295                 300

Tyr Asp Asp Val Thr Ile Lys Met Ile Phe Ala Ile Val Gln Ile Ile
305                 310                 315                 320

Gly Phe Ser Asn Ser Ile Cys Asn Pro Ile Val Tyr Ala Phe Met Asn
                325                 330                 335

Glu Asn Phe Lys Lys Asn Val Leu Ser Ala Val Cys Tyr Cys Ile Val
            340                 345                 350

Asn Lys Thr Phe Ser Pro Ala Gln Arg His Gly Asn Ser Gly Ile Thr
        355                 360                 365

Met Met Arg Lys Lys Ala Lys Phe Ser Leu Arg Glu Asn Pro Val Glu
    370                 375                 380

Glu Thr Lys Gly Glu Ala Phe Ser Asp Gly Asn Ile Glu Val Lys Leu
385                 390                 395                 400

Cys Glu Gln Thr Glu Glu Lys Lys Lys Leu Lys Arg His Leu Ala Leu
                405                 410                 415

Phe Arg Ser Glu Leu Ala Glu Asn Ser Pro Leu Asp Ser Gly His
            420                 425                 430

(40) INFORMATION FOR SEQ ID NO:39:

     (i) SEQUENCE CHARACTERISTICS:
          (A) LENGTH: 24 base pairs
          (B) TYPE: nucleic acid
          (C) STRANDEDNESS: single
          (D) TOPOLOGY: linear

    (ii) MOLECULE TYPE: DNA (genomic)

    (xi) SEQUENCE DESCRIPTION: SEQ ID NO:39:

CTGTGTACAG CAGTTCGCAG AGTG                                          24

(41) INFORMATION FOR SEQ ID NO:40:

     (i) SEQUENCE CHARACTERISTICS:
          (A) LENGTH: 24 base pairs
          (B) TYPE: nucleic acid
          (C) STRANDEDNESS: single
          (D) TOPOLOGY: linear

    (ii) MOLECULE TYPE: DNA (genomic)

    (xi) SEQUENCE DESCRIPTION: SEQ ID NO:40:

GAGTGCCAGG CAGAGCAGGT AGAC                                          24

(42) INFORMATION FOR SEQ ID NO:41:

     (i) SEQUENCE CHARACTERISTICS:
          (A) LENGTH: 31 base pairs
          (B) TYPE: nucleic acid
          (C) STRANDEDNESS: single
          (D) TOPOLOGY: linear

    (ii) MOLECULE TYPE: DNA (genomic)

    (iv) ANTI-SENSE: NO

    (xi) SEQUENCE DESCRIPTION: SEQ ID NO:41:

CCCGAATTCC TGCTTGCTCC CAGCTTGGCC C                                  31

(43) INFORMATION FOR SEQ ID NO:42:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:42:

TGTGGATCCT GCTGTCAAAG GTCCCATTCC GG 32

10 (44) INFORMATION FOR SEQ ID NO:43:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:43:

TCACAATGCT AGGTGTGGTC 20

20 (45) INFORMATION FOR SEQ ID NO:44:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:44:

TGCATAGACA ATGGGATTAC AG 22

(46) INFORMATION FOR SEQ ID NO:45:

(i) SEQUENCE CHARACTERISTICS:
(A) LENGTH: 511 base pairs
(B) TYPE: nucleic acid
(C) STRANDEDNESS: single

(D) TOPOLOGY: linear

(ii) MOLECULE TYPE: DNA (genomic)

(xi) SEQUENCE DESCRIPTION: SEQ ID NO:45:

TCACAATGCT AGGTGTGGTC TGGCTGGTGG CAGTCATCGT AGGATCACCC ATGTGGCACG 60

TGCAACAACT TGAGATCAAA TATGACTTCC TATATGAAAA GGAACACATC TGCTGCTTAG 120

AAGAGTGGAC CAGCCCTGTG CACCAGAAGA TCTACACCAC CTTCATCCTT GTCATCCTCT 180

TCCTCCTGCC TCTTATGGTG ATGCTTATTC TGTACGTAAA ATTGGTTATG AACTTTGGAT 240

AAAGAAAAGA GTTGGGGATG GTTCAGTGCT TCGAACTATT CATGGAAAAG AAATGTCCAA 300

AATAGCCAGG AAGAAGAAAC GAGCTGTCAT TATGATGGTG ACAGTGGTGG CTCTCTTTGC 360

TGTGTGCTGG GCACCATTCC ATGTTGTCCA TATGATGATT GAATACAGTA ATTTTGAAAA 420

GGAATATGAT GATGTCACAA TCAAGATGAT TTTTGCTATC GTGCAAATTA TTGGATTTTC 480

CAACTCCATC TGTAATCCCA TTGTCTATGC A 511

(47) INFORMATION FOR SEQ ID NO:46:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:46:

CTGCTTAGAA GAGTGGACCA G 21

(48) INFORMATION FOR SEQ ID NO:47:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:47:

CTGTGCACCA GAAGATCTAC AC 22

(49) INFORMATION FOR SEQ ID NO:48:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:48:

CAAGGATGAA GGTGGTGTAG A 21

(50) INFORMATION FOR SEQ ID NO:49:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:49:

GTGTAGATCT TCTGGTGCAC AGG 23

(51) INFORMATION FOR SEQ ID NO:50:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: DNA (genomic)

(xi) SEQUENCE DESCRIPTION: SEQ ID NO:50:

GCAATGCAGG TCATAGTGAG C 21

(52) INFORMATION FOR SEQ ID NO:51:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: YES

(iv) ANTI-SENSE: YES

(xi) SEQUENCE DESCRIPTION: SEQ ID NO:51:

TGGAGCATGG TGACGGGAAT GCAGAAG 27

(53) INFORMATION FOR SEQ ID NO:52:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:52:

GTGATGAGCA GGTCACTGAG CGCCAAG 27

(54) INFORMATION FOR SEQ ID NO:53:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:53:

GCAATGCAGG CGCTTAACAT TAC 23

(55) INFORMATION FOR SEQ ID NO:54:

(i) SEQUENCE CHARACTERISTICS:
(A) LENGTH: 22 base pairs

(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:54:

TTGGGTTACA ATCTGAAGGG CA 22

(56) INFORMATION FOR SEQ ID NO:55:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:55:

ACTCCGTGTC CAGCAGGACT CTG 23

(57) INFORMATION FOR SEQ ID NO:56:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:56:

TGCGTGTTCC TGGACCCTCA CGTG 24

(58) INFORMATION FOR SEQ ID NO:57:

(i) SEQUENCE CHARACTERISTICS:
(A) LENGTH: 29 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:57:

CAGGCCTTGG ATTTTAATGT CAGGGATGG 29

(59) INFORMATION FOR SEQ ID NO:58:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:58:

GGAGAGTCAG CTCTGAAAGA ATTCAGG 27

(60) INFORMATION FOR SEQ ID NO:59:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:59:

TGATGTGATG CCAGATACTA ATAGCAC 27

(61) INFORMATION FOR SEQ ID NO:60:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:60:

CCTGATTCAT TTAGGTGAGA TTGAGAC 27

(62) INFORMATION FOR SEQ ID NO:61:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:61:

GACAGGTACC TTGCCATCAA G 21

(63) INFORMATION FOR SEQ ID NO:62:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:62:

CTGCACAATG CCAGTGATAA GG 22

(64) INFORMATION FOR SEQ ID NO:63:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:63:

CTGACTTCTT GTTCCTGGCA GCAGCGG 27

(65) INFORMATION FOR SEQ ID NO:64:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:64:

10 AGACCAGCCA GGGCACGCTG AAGAGTG 27

(66) INFORMATION FOR SEQ ID NO:65:

(i) SEQUENCE CHARACTERISTICS:
(A) LENGTH: 32 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:65:

20 GATCAAGCTT CCATCCTACT GAAACCATGG TC 32

(67) INFORMATION FOR SEQ ID NO:66:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:66:

30 GATCAGATCT CAGTTCCAAT ATTCACACCA CCGTC 35

(68) INFORMATION FOR SEQ ID NO:67:

(i) SEQUENCE CHARACTERISTICS:

(A) LENGTH: 22 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:67:

CTGGTGTGCT CCATGGCATC CC 22

(69) INFORMATION FOR SEQ ID NO:68:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:68:

GTAAGCCTCC CAGAACGAGA GG 22

(70) INFORMATION FOR SEQ ID NO:69:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:69:

CAGCGCAGGG TGAAGCCTGA GAGC 24

(71) INFORMATION FOR SEQ ID NO:70:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:70:

GGCACCTGCT GTGACCTGTG CAGG 24

(72) INFORMATION FOR SEQ ID NO:71:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:71:

GTCCTGCCAC TTCGAGACAT GG 22

(73) INFORMATION FOR SEQ ID NO:72:

(i) SEQUENCE CHARACTERISTICS:
(A) LENGTH: 23 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:72:

GAAACTTCTC TGCCCTTACC GTC 23

(74) INFORMATION FOR SEQ ID NO:73:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(iv) ANTI-SENSE: NO

(xi) SEQUENCE DESCRIPTION: SEQ ID NO:73:

CCAACACCAG CATCCATGGC ATCAAG 26

(75) INFORMATION FOR SEQ ID NO:74:

(i) SEQUENCE CHARACTERISTICS:
(A) LENGTH: 27 base pairs
(B) TYPE: nucleic acid
(C) STRANDEDNESS: single
(D) TOPOLOGY: linear

(ii) MOLECULE TYPE: DNA (genomic)

(iv) ANTI-SENSE: YES

(xi) SEQUENCE DESCRIPTION: SEQ ID NO:74:

GGAGAGTCAG CTCTGAAAGA ATTCAGG 27

SEQUENCE LISTING

<110> Arena Pharmaceuticals, Inc.

<120> Human Orphan G Protein-Coupled Receptors

<130> JEC/FP6717193

<140> Divisional of EP 07001452.7
<141> 1999 10 13

<150> EP 05003040.2
<151> 1999 10 13

<150> EP 99972682.1
<151> 1999 10 13

<150> PCT/US99/23687
<151> 1999 10 13

<150> 60/109,213
<151> 1998 11 20

<150> 60/120,416
<151> 1999 02 16

<150> 60/121,852
<151> 1999 02 26

<150> 60/123,946
<151> 1999 03 12

<150> 60/123,949
<151> 1999 03 12

<150> 60/136,436
<151> 1999 05 28

<150> 60/136,437
<151> 1999 05 28

<150> 60/136,439
<151> 1999 05 28

<150> 60/136,567
<151> 1999 05 28

<150> 60/137,127
<151> 1999 05 28

<150> 60/137,131
<151> 1999 05 28

<150> 60/141,448

<151> 1999 06 29

<150> 60/156,653
<151> 1999 09 29

<150> 60/156,333
<151> 1999 09 29

<150> 60/156,555
<151> 1999 09 29

<150> 60/156,634
<151> 1999 09 29

<151> 1999 09 29

<150> 60/157,280
<151> 1999 10 01

<150> 60/157,294
<151> 1999 10 01

<150> 60/157,281
<151> 1999 10 01

<150> 60/157,293
<151> 1999 10 01

<150> 60/157,282
<151> 1999 10 01

<150> 09/417,044
<151> 1999 10 12

<150> 09/416,760
<151> 1999 10 12

<160> 74

<170> PatentIn Ver. 2.1

<210> 1
<211> 1260
<212> DNA
<213> Homo sapiens

<400> 1
atggtcttct cggcagtgtt gactgcgttc cataccggga catccaacac aacatttgtc 60
gtgtatgaaa acacctacat gaatattaca ctccctccac cattccagca tcctgacctc 120
agtccattgc ttagatatag ttttgaaacc atggctccca ctggtttgag ttccttgacc 180
gtgaatagta cagctgtgcc cacaacacca gcagcattta agagcctaaa cttgcctctt 240
cagatcaccc tttctgctat aatgatattc attctgtttg tgtcttttct tgggaacttg 300
gttgtttgcc tcatggttta ccaaaaagct gccatgaggt ctgcaattaa catcctcctt 360
gccagcctag cttttgcaga catgttgctt gcagtgctga acatgccctt tgccctggta 420
actattctta ctacccgatg gatttttggg aaattcttct gtagggtatc tgctatgttt 480
ttctggttat ttgtgataga aggagtagcc atcctgctca tcattagcat agataggttc 540
cttattatag tccagaggca ggataagcta aacccatata gagctaaggt tctgattgca 600
gtttcttggg caacttcctt ttgtgtagct tttcctttag ccgtaggaaa ccccgacctg 660
cagatacctt cccgagctcc ccagtgtgtg tttgggtaca caaccaatcc aggctaccag 720
gcttatgtga ttttgatttc tctcatttct ttcttcatac ccttcctggt aatactgtac 780
tcatttatgg gcatactcaa cacccttcgg cacaatgcct tgaggatcca tagctaccct 840
gaaggtatat gcctcagcca ggccagcaaa ctgggtctca tgagtctgca gagacctttc 900
cagatgagca ttgacatggg ctttaaaaca cgtgccttca ccactatttt gattctcttt 960
gctgtcttca ttgtctgctg ggccccattc accacttaca gccttgtggc aacattcagt 1020
aagcactttt actatcagca caactttttt gagattagca cctggctact gtggctctgc 1080
tacctcaagt ctgcattgaa tccgctgatc tactactgga ggattaagaa attccatgat 1140
gcttgcctgg acatgatgcc taagtccttc aagtttttgc cgcagctccc tggtcacaca 1200
aagcgacgga tacgtcctag tgctgtctat gtgtgtgggg aacatcggac ggtggtgtga 1260

<210> 2
<211> 419
<212> PRT
<213> Homo sapiens

<400> 2
Met Val Phe Ser Ala Val Leu Thr Ala Phe His Thr Gly Thr Ser Asn
1               5                   10                  15

Thr Thr Phe Val Val Tyr Glu Asn Thr Tyr Met Asn Ile Thr Leu Pro
            20                  25                  30

Pro Pro Phe Gln His Pro Asp Leu Ser Pro Leu Leu Arg Tyr Ser Phe
            35                  40                  45

Glu Thr Met Ala Pro Thr Gly Leu Ser Ser Leu Thr Val Asn Ser Thr
    50                  55                  60

Ala Val Pro Thr Thr Pro Ala Ala Phe Lys Ser Leu Asn Leu Pro Leu
65                  70                  75                  80

Gln Ile Thr Leu Ser Ala Ile Met Ile Phe Ile Leu Phe Val Ser Phe
                85                  90                  95

Leu Gly Asn Leu Val Val Cys Leu Met Val Tyr Gln Lys Ala Ala Met
            100                 105                 110

Arg Ser Ala Ile Asn Ile Leu Leu Ala Ser Leu Ala Phe Ala Asp Met
        115                 120                 125

Leu Leu Ala Val Leu Asn Met Pro Phe Ala Leu Val Thr Ile Leu Thr
    130                 135                 140

Thr Arg Trp Ile Phe Gly Lys Phe Phe Cys Arg Val Ser Ala Met Phe
145                 150                 155                 160

Phe Trp Leu Phe Val Ile Glu Gly Val Ala Ile Leu Leu Ile Ile Ser
                165                 170                 175

Ile Asp Arg Phe Leu Ile Ile Val Gln Arg Gln Asp Lys Leu Asn Pro
            180                 185                 190

Tyr Arg Ala Lys Val Leu Ile Ala Val Ser Trp Ala Thr Ser Phe Cys
        195                 200                 205

Val Ala Phe Pro Leu Ala Val Gly Asn Pro Asp Leu Gln Ile Pro Ser
    210                 215                 220

Arg Ala Pro Gln Cys Val Phe Gly Tyr Thr Thr Asn Pro Gly Tyr Gln
225                 230                 235                 240

Ala Tyr Val Ile Leu Ile Ser Leu Ile Ser Phe Phe Ile Pro Phe Leu
                245                 250                 255

Val Ile Leu Tyr Ser Phe Met Gly Ile Leu Asn Thr Leu Arg His Asn
            260                 265                 270

Ala Leu Arg Ile His Ser Tyr Pro Glu Gly Ile Cys Leu Ser Gln Ala
        275                 280                 285

Ser Lys Leu Gly Leu Met Ser Leu Gln Arg Pro Phe Gln Met Ser Ile
    290                 295                 300

Asp Met Gly Phe Lys Thr Arg Ala Phe Thr Thr Ile Leu Ile Leu Phe
305                 310                 315                 320

Ala Val Phe Ile Val Cys Trp Ala Pro Phe Thr Thr Tyr Ser Leu Val
                325                 330                 335

Ala Thr Phe Ser Lys His Phe Tyr Tyr Gln His Asn Phe Phe Glu Ile
            340                 345                 350

Ser Thr Trp Leu Leu Trp Leu Cys Tyr Leu Lys Ser Ala Leu Asn Pro
        355                 360                 365

Leu Ile Tyr Tyr Trp Arg Ile Lys Lys Phe His Asp Ala Cys Leu Asp
    370                 375                 380

Met Met Pro Lys Ser Phe Lys Phe Leu Pro Gln Leu Pro Gly His Thr
385                 390                 395                 400

Lys Arg Arg Ile Arg Pro Ser Ala Val Tyr Val Cys Gly Glu His Arg
            405                 410                 415

Thr Val Val

<210> 3
<211> 1119
<212> DNA
<213> Homo sapiens

<400> 3
atgttagcca acagctcctc aaccaacagt tctgttctcc cgtgtcctga ctaccgacct 60
acccaccgcc tgcacttggt ggtctacagc ttggtgctgg ctgccgggct ccccctcaac 120
gcgctagccc tctgggtctt cctgcgcgcg ctgcgcgtgc actcggtggt gagcgtgtac 180
atgtgtaacc tggcggccag cgacctgctc ttcaccctct cgctgcccgt tcgtctctcc 240
tactacgcac tgcaccactg gcccttcccc gacctcctgt gccagacgac gggcgccatc 300
ttccagatga acatgtacgg cagctgcatc ttcctgatgc tcatcaagct ggaccgctac 360
gccgccatcg tgcacccgct gcgactgcgc cacctgcggc ggccccgcgt ggcgcggctg 420
ctctgcctgg gcgtgtgggc gctcatcctg gtgtttgccg tgcccgccgc ccgcgtgcac 480
aggccctcgc gttgccgcta ccgggacctc gaggtgcgcc tatgcttcga gagcttcagc 540
gacgagctgt ggaaaggcag gctgctgccc ctcgtgctgc tggccgaggc gctgggcttc 600
ctgctgcccc tggcggcggt ggtctactcg tcgggccgag tcttctggac gctggcgcgc 660
cccgacgcca cgcagagcca gcggcggcgg aagaccgtgc gcctcctgct ggctaacctc 720
gtcatcttcc tgctgtgctt cgtgccctac aacagcacgc tggcggtcta cgggctgctg 780
cggagcaagc tggtggcggc cagcgtgcct gcccgcgatc gcgtgcgcgg ggtgctgatg 840
gtgatggtgc tgctggccgg cgccaactgc gtgctggacc cgctggtgta ctactttagc 900
gccgagggct tccgcaacac cctgcgcggc ctgggcactc cgcaccgggc caggacctcg 960
gccaccaacg ggacgcgggc ggcgctcgcg caatccgaaa ggtccgccgt caccaccgac 1020
gccaccaggc cggatgccgc cagtcagggg ctgctccgac cctccgactc ccactctctg 1080
tcttccttca cacagtgtcc ccaggattcc gccctctga                        1119

<210> 4
<211> 372
<212> PRT
<213> Homo sapiens

<400> 4


<210> 5
<211> 1107
<212> DNA
<213> Homo sapiens

<400> 5
atggccaact ccacagggct gaacgcctca gaagtcgcag gctcgttggg gttgatcctg 60
gcagctgtcg tggaggtggg ggcactgctg ggcaacggcg cgctgctggt cgtggtgctg 120
cgcacgccgg gactgcgcga cgcgctctac ctggcgcacc tgtgcgtcgt ggacctgctg 180
gcggccgcct ccatcatgcc gctgggcctg ctggccgcac cgccgcccgg gctgggccgc 240
gtgcgcctgg gccccgcgcc atgccgcgcc gctcgcttcc tctccgccgc tctgctgccg 300
gcctgcacgc tcggggtggc cgcacttggc ctggcacgct accgcctcat cgtgcacccg 360
ctgcggccag gctcgcggcc gccgcctgtg ctcgtgctca ccgccgtgtg ggccgcggcg 420
ggactgctgg gcgcgctctc cctgctcggc ccgccgcccg caccgccccc tgctcctgct 480
cgctgctcgg tcctggctgg gggcctcggg cccttccggc cgctctgggc cctgctggcc 540
ttcgcgctgc ccgccctcct gctgctcggc gcctacggcg gcatcttcgt ggtggcgcgt 600
cgcgctgccc tgaggccccc acggccggcg cgcgggtccc gactccgctc ggactctctg 660
gatagccgcc tttccatctt gccgccgctc cggcctcgcc tgcccggggg caaggcggcc 720
ctggccccag cgctggccgt gggccaattt gcagcctgct ggctgcctta tggctgcgcg 780
tgcctggcgc ccgcagcgcg ggccgcggaa gccgaagcgg ctgtcacctg ggtcgcctac 840
tcggccttcg cggctcaccc cttcctgtac gggctgctgc agcgccccgt gcgcttggca 900
ctgggccgcc tctctcgccg tgcactgcct ggacctgtgc gggcctgcac tccgcaagcc 960

tggcacccgc gggcactctt gcaatgcctc cagagacccc cagagggccc tgccgtaggc 1020
ccttctgagg ctccagaaca gacccccgag ttggcaggag ggcggagccc cgcataccag 1080
gggccacctg agagttctct ctcctga 1107

<210> 6
<211> 368
<212> PRT
<213> Homo sapiens

<400> 6
Met Ala Asn Ser Thr Gly Leu Asn Ala Ser Glu Val Ala Gly Ser Leu
1               5                   10                  15

Gly Leu Ile Leu Ala Ala Val Val Glu Val Gly Ala Leu Leu Gly Asn
            20                  25                  30

Gly Ala Leu Leu Val Val Val Leu Arg Thr Pro Gly Leu Arg Asp Ala
        35                  40                  45

Leu Tyr Leu Ala His Leu Cys Val Val Asp Leu Leu Ala Ala Ala Ser
    50                  55                  60

Ile Met Pro Leu Gly Leu Leu Ala Ala Pro Pro Pro Gly Leu Gly Arg
65                  70                  75                  80

Val Arg Leu Gly Pro Ala Pro Cys Arg Ala Ala Arg Phe Leu Ser Ala
                85                  90                  95

Ala Leu Leu Pro Ala Cys Thr Leu Gly Val Ala Ala Leu Gly Leu Ala
            100                 105                 110

Arg Tyr Arg Leu Ile Val His Pro Leu Arg Pro Gly Ser Arg Pro Pro
        115                 120                 125

Pro Val Leu Val Leu Thr Ala Val Trp Ala Ala Ala Gly Leu Leu Gly
    130                 135                 140

Ala Leu Ser Leu Leu Gly Pro Pro Pro Ala Pro Pro Pro Ala Pro Ala
145                 150                 155                 160

Arg Cys Ser Val Leu Ala Gly Gly Leu Gly Pro Phe Arg Pro Leu Trp
                165                 170                 175

Ala Leu Leu Ala Phe Ala Leu Pro Ala Leu Leu Leu Leu Gly Ala Tyr
            180                 185                 190

Gly Gly Ile Phe Val Val Ala Arg Arg Ala Ala Leu Arg Pro Pro Arg
        195                 200                 205

Pro Ala Arg Gly Ser Arg Leu Arg Ser Asp Ser Leu Asp Ser Arg Leu
    210                 215                 220

Ser Ile Leu Pro Pro Leu Arg Pro Arg Leu Pro Gly Gly Lys Ala Ala
225                 230                 235                 240

Leu Ala Pro Ala Leu Ala Val Gly Gln Phe Ala Ala Cys Trp Leu Pro
                245                 250                 255

Tyr Gly Cys Ala Cys Leu Ala Pro Ala Ala Arg Ala Ala Glu Ala Glu
            260                 265                 270

Ala Ala Val Thr Trp Val Ala Tyr Ser Ala Phe Ala Ala His Pro Phe
        275                 280                 285

Leu Tyr Gly Leu Leu Gln Arg Pro Val Arg Leu Ala Leu Gly Arg Leu
    290                 295                 300

Ser Arg Arg Ala Leu Pro Gly Pro Val Arg Ala Cys Thr Pro Gln Ala
305                 310                 315                 320

Trp His Pro Arg Ala Leu Leu Gln Cys Leu Gln Arg Pro Pro Glu Gly
                325                 330                 335

Pro Ala Val Gly Pro Ser Glu Ala Pro Glu Gln Thr Pro Glu Leu Ala
            340                 345                 350

Gly Gly Arg Ser Pro Ala Tyr Gln Gly Pro Pro Glu Ser Ser Leu Ser
        355                 360                 365

<210> 7
<211> 1008
<212> DNA
<213> Homo sapiens

<400> 7
atggaatcat ctttctcatt tggagtgatc cttgctgtcc tggcctccct catcattgct 60
actaacacac tagtggctgt ggctgtgctg ctgttgatcc acaagaatga tggtgtcagt 120
ctctgcttca ccttgaatct ggctgtggct gacaccttga ttggtgtggc catctctggc 180
ctactcacag accagctctc cagcccttct cggcccacac agaagaccct gtgcagcctg 240
cggatggcat ttgtcacttc ctccgcagct gcctctgtcc tcacggtcat gctgatcacc 300
tttgacaggt accttgccat caagcagccc ttccgctact tgaagatcat gagtgggttc 360
gtggccgggg cctgcattgc cgggctgtgg ttagtgtctt acctcattgg cttcctccca 420
ctcggaatcc ccatgttcca gcagactgcc tacaaagggc agtgcagctt ctttgctgta 480
tttcaccctc acttcgtgct gaccctctcc tgcgttggct tcttcccagc catgctcctc 540
tttgtcttct tctactgcga catgctcaag attgcctcca tgcacagcca gcagattcga 600
aagatggaac atgcaggagc catggctgga ggttatcgat ccccacggac tcccagcgac 660
ttcaaagctc tccgtactgt gtctgttctc attgggagct ttgctctatc ctggaccccc 720
ttccttatca ctggcattgt gcaggtggcc tgccaggagt gtcacctcta cctagtgctg 780
gaacggtacc tgtggctgct cggcgtgggc aactccctgc tcaacccact catctatgcc 840
tattggcaga aggaggtgcg actgcagctc taccacatgg ccctaggagt gaagaaggtg 900
ctcacctcat tcctcctctt tctctcggcc aggaattgtg gcccagagag gcccagggaa 960
agttcctgtc acatcgtcac tatctccagc tcagagtttg atggctaa          1008

<210> 8
<211> 335
<212> PRT
<213> Homo sapiens

<400> 8
Met Glu Ser Ser Phe Ser Phe Gly Val Ile Leu Ala Val Leu Ala Ser
1               5                   10                  15

Leu Ile Ile Ala Thr Asn Thr Leu Val Ala Val Ala Val Leu Leu Leu
            20                  25                  30

Ile His Lys Asn Asp Gly Val Ser Leu Cys Phe Thr Leu Asn Leu Ala
        35                  40                  45

Val Ala Asp Thr Leu Ile Gly Val Ala Ile Ser Gly Leu Leu Thr Asp
    50                  55                  60

Gln Leu Ser Ser Pro Ser Arg Pro Thr Gln Lys Thr Leu Cys Ser Leu
65                  70                  75                  80

Arg Met Ala Phe Val Thr Ser Ser Ala Ala Ala Ser Val Leu Thr Val
                85                  90                  95

Met Leu Ile Thr Phe Asp Arg Tyr Leu Ala Ile Lys Gln Pro Phe Arg
            100                 105                 110

Tyr Leu Lys Ile Met Ser Gly Phe Val Ala Gly Ala Cys Ile Ala Gly
        115                 120                 125

Leu Trp Leu Val Ser Tyr Leu Ile Gly Phe Leu Pro Leu Gly Ile Pro
    130                 135                 140

Met Phe Gln Gln Thr Ala Tyr Lys Gly Gln Cys Ser Phe Phe Ala Val
145                 150                 155                 160

Phe His Pro His Phe Val Leu Thr Leu Ser Cys Val Gly Phe Phe Pro
                165                 170                 175

Ala Met Leu Leu Phe Val Phe Phe Tyr Cys Asp Met Leu Lys Ile Ala
            180                 185                 190

Ser Met His Ser Gln Gln Ile Arg Lys Met Glu His Ala Gly Ala Met
        195                 200                 205

Ala Gly Gly Tyr Arg Ser Pro Arg Thr Pro Ser Asp Phe Lys Ala Leu
    210                 215                 220

Arg Thr Val Ser Val Leu Ile Gly Ser Phe Ala Leu Ser Trp Thr Pro
225                 230                 235                 240

Phe Leu Ile Thr Gly Ile Val Gln Val Ala Cys Gln Glu Cys His Leu
                245                 250                 255

Tyr Leu Val Leu Glu Arg Tyr Leu Trp Leu Leu Gly Val Gly Asn Ser
            260                 265                 270

Leu Leu Asn Pro Leu Ile Tyr Ala Tyr Trp Gln Lys Glu Val Arg Leu
        275                 280                 285

Gln Leu Tyr His Met Ala Leu Gly Val Lys Lys Val Leu Thr Ser Phe
    290                 295                 300

Leu Leu Phe Leu Ser Ala Arg Asn Cys Gly Pro Glu Arg Pro Arg Glu
305                 310                 315                 320

Ser Ser Cys His Ile Val Thr Ile Ser Ser Ser Glu Phe Asp Gly
                325                 330                 335


<210> 9
<211> 1413
<212> DNA
<213> Homo sapiens

<400> 9
atggacacta ccatggaagc tgacctgggt gccactggcc acaggccccg cacagagctt 60
gatgatgagg actcctaccc ccaaggtggc tgggacacgg tcttcctggt ggccctgctg 120
ctccttgggc tgccagccaa tgggttgatg gcgtggctgg ccggctccca ggcccggcat 180
ggagctggca cgcgtctggc gctgctcctg ctcagcctgg ccctctctga cttcttgttc 240
ctggcagcag cggccttcca gatcctagag atccggcatg ggggacactg gccgctgggg 300
acagctgcct gccgcttcta ctacttccta tggggcgtgt cctactcctc cggcctcttc 360
ctgctggccg ccctcagcct cgaccgctgc ctgctggcgc tgtgcccaca ctggtaccct 420
gggcaccgcc cagtccgcct gcccctctgg gtctgcgccg gtgtctgggt gctggccaca 480
ctcttcagcg tgccctggct ggtcttcccc gaggctgccg tctggtggta cgacctggtc 540
atctgcctgg acttctggga cagcgaggag ctgtcgctga ggatgctgga ggtcctgggg 600
ggcttcctgc ctttcctcct gctgctcgtc tgccacgtgc tcacccaggc cacagcctgt 660
cgcacctgcc accgccaaca gcagcccgca gcctgccggg gcttcgcccg tgtggccagg 720
accattctgt cagcctatgt ggtcctgagg ctgccctacc agctggccca gctgctctac 780
ctggccttcc tgtgggacgt ctactctggc tacctgctct gggaggccct ggtctactcc 840
gactacctga tcctactcaa cagctgcctc agccccttcc tctgcctcat ggccagtgcc 900
gacctccgga ccctgctgcg ctccgtgctc tcgtccttcg cggcagctct ctgcgaggag 960
cggccgggca gcttcacgcc cactgagcca cagacccagc tagattctga gggtccaact 1020
ctgccagagc cgatggcaga ggcccagtca cagatggatc ctgtggccca gcctcaggtg 1080
aaccccacac tccagccacg atcggatccc acagctcagc cacagctgaa ccctacggcc 1140

cagccacagt cggatcccac agcccagcca cagctgaacc tcatggccca gccacagtca 1200
gattctgtgg cccagccaca ggcagacact aacgtccaga cccctgcacc tgctgccagt 1260
tctgtgccca gtccctgtga tgaagcttcc ccaaccccat cctcgcatcc taccccaggg 1320
gcccttgagg acccagccac acctcctgcc tctgaaggag aaagccccag cagcaccccg 1380
ccagaggcgg ccccgggcgc aggccccacg tga 1413

<210> 10
<211> 468
<212> PRT
<213> Homo sapiens

<400> 10
Met Asp Thr Thr Met Glu Ala Asp Leu Gly Ala Thr Gly His Arg Pro
1               5                   10                  15

Arg Thr Glu Leu Asp Asp Glu Asp Ser Tyr Pro Gln Gly Gly Trp Asp
            20                  25                  30

Thr Val Phe Leu Val Ala Leu Leu Leu Leu Gly Leu Pro Ala Asn Gly
        35                  40                  45

Leu Met Ala Trp Leu Ala Gly Ser Gln Ala Arg His Gly Ala Gly Thr
    50                  55                  60

Arg Leu Ala Leu Leu Leu Leu Ser Leu Ala Leu Ser Asp Phe Leu Phe
65                  70                  75                  80

Leu Ala Ala Ala Ala Phe Gln Ile Leu Glu Ile Arg His Gly Gly His
                85                  90                  95

Trp Pro Leu Gly Thr Ala Ala Cys Arg Phe Tyr Tyr Phe Leu Trp Gly
            100                 105                 110

Val Ser Tyr Ser Ser Gly Leu Phe Leu Leu Ala Ala Leu Ser Leu Asp
        115                 120                 125

Arg Cys Leu Leu Ala Leu Cys Pro His Trp Tyr Pro Gly His Arg Pro
    130                 135                 140

Val Arg Leu Pro Leu Trp Val Cys Ala Gly Val Trp Val Leu Ala Thr
145                 150                 155                 160

Leu Phe Ser Val Pro Trp Leu Val Phe Pro Glu Ala Ala Val Trp Trp
                165                 170                 175

Tyr Asp Leu Val Ile Cys Leu Asp Phe Trp Asp Ser Glu Glu Leu Ser
            180                 185                 190

Leu Arg Met Leu Glu Val Leu Gly Gly Phe Leu Pro Phe Leu Leu Leu
        195                 200                 205

Leu Val Cys His Val Leu Thr Gln Ala Thr Arg Thr Cys His Arg Gln
    210                 215                 220

Gln Gln Pro Ala Ala Cys Arg Gly Phe Ala Arg Val Ala Arg Thr Ile
225                 230                 235                 240

Leu Ser Ala Tyr Val Val Leu Arg Leu Pro Tyr Gln Leu Ala Gln Leu
                245                 250                 255

Leu Tyr Leu Ala Phe Leu Trp Asp Val Tyr Ser Gly Tyr Leu Leu Trp
            260                 265                 270

Glu Ala Leu Val Tyr Ser Asp Tyr Leu Ile Leu Leu Asn Ser Cys Leu
        275                 280                 285

Ser Pro Phe Leu Cys Leu Met Ala Ser Ala Asp Leu Arg Thr Leu Leu

290                 295                 300

Arg Ser Val Leu Ser Ser Phe Ala Ala Ala Leu Cys Glu Glu Arg Pro
305                 310                 315                 320

Gly Ser Phe Thr Pro Thr Glu Pro Gln Thr Gln Leu Asp Ser Glu Gly
                325                 330                 335

Pro Thr Leu Pro Glu Pro Met Ala Glu Ala Gln Ser Gln Met Asp Pro
            340                 345                 350

Val Ala Gln Pro Gln Val Asn Pro Thr Leu Gln Pro Arg Ser Asp Pro
        355                 360                 365

Thr Ala Gln Pro Gln Leu Asn Pro Thr Ala Gln Pro Gln Ser Asp Pro
    370                 375                 380

Thr Ala Gln Pro Gln Leu Asn Leu Met Ala Gln Pro Gln Ser Asp Ser
385                 390                 395                 400

Val Ala Gln Pro Gln Ala Asp Thr Asn Val Gln Thr Pro Ala Pro Ala
                405                 410                 415

Ala Ser Ser Val Pro Ser Pro Cys Asp Glu Ala Ser Pro Thr Pro Ser
            420                 425                 430

Ser His Pro Thr Pro Gly Ala Leu Glu Asp Pro Ala Thr Pro Pro Ala
        435                 440                 445

Ser Glu Gly Glu Ser Pro Ser Ser Thr Pro Pro Glu Ala Ala Pro Gly
    450                 455                 460

Ala Gly Pro Thr
465


<210> 11
<211> 1248
<212> DNA
<213> Homo sapiens

<400> 11
atgtcaggga tggaaaaact tcagaatgct tcctggatct accagcagaa actagaagat 60
ccattccaga aacacctgaa cagcaccgag gagtatctgg ccttcctctg cggacctcgg 120
cgcagccact tcttcctccc cgtgtctgtg gtgtatgtgc caatttttgt ggtgggggtc 180
attggcaatg tcctggtgtg cctggtgatt ctgcagcacc aggctatgaa gacgcccacc 240
aactactacc tcttcagcct ggcggtctct gacctcctgg tcctgctcct tggaatgccc 300
ctggaggtct atgagatgtg gcgcaactac cctttcttgt tcgggcccgt gggctgctac 360
ttcaagacgg ccctctttga gaccgtgtgc ttcgcctcca tcctcagcat caccaccgtc 420
agcgtggagc gctacgtggc catcctacac ccgttccgcg ccaaactgca gagcacccgg 480
cgccgggccc tcaggatcct cggcatcgtc tggggcttct ccgtgctctt ctccctgccc 540
aacaccagca tccatggcat caagttccac tacttcccca atgggtccct ggtcccaggt 600
tcggccacct gtacggtcat caagcccatg tggatctaca atttcatcat ccaggtcacc 660
tccttcctat tctacctcct ccccatgact gtcatcagtg tcctctacta cctcatggca 720
ctcagactaa agaaagacaa atctcttgag gcagatgaag ggaatgcaaa tattcaaaga 780
ccctgcagaa aatcagtcaa caagatgctg tttgtcttgg tcttagtgtt tgctatctgt 840
tgggccccgt tccacattga ccgactcttc ttcagctttg tggaggagtg gagtgaatcc 900
ctggctgctg tgttcaacct cgtccatgtg gtgtcaggtg tcttcttcta cctgagctca 960
gctgtcaacc ccattatcta taacctactg tctcgccgct tccaggcagc attccagaat 1020
gtgatctctt ctttccacaa acagtggcac tcccagcatg acccacagtt gccacctgcc 1080
cagcggaaca tcttcctgac agaatgccac tttgtggagc tgaccgaaga tataggtccc 1140
caattcccat gtcagtcatc catgcacaac tctcacctcc caacagccct ctctagtgaa 1200
cagatgtcaa gaacaaacta tcaaagcttc cactttaaca aaacctga             1248


<210> 12
<211> 415
<212> PRT

<213> Homo sapiens

<400> 12


<210> 13
<211> 1173
<212> DNA
<213> Homo sapiens

<400> 13
atgccagata ctaatagcac aatcaattta tcactaagca ctcgtgttac tttagcattt 60
tttatgtcct tagtagcttt tgctataatg ctaggaaatg ctttggtcat tttagctttt 120
gtggtggaca aaaaccttag acatcgaagt agttattttt ttcttaactt ggccatctct 180
gacttctttg tgggtgtgat ctccattcct ttgtacatcc ctcacacgct gttcgaatgg 240
gattttggaa aggaaatctg tgtattttgg ctcactactg actatctgtt atgtacagca 300
tctgtatata acattgtcct catcagctat gatcgatacc tgtcagtctc aaatgctgtg 360
tcttatagaa ctcaacatac tggggtcttg aagattgtta ctctgatggt ggccgtttgg 420
gtgctggcct tcttagtgaa tgggccaatg attctagttt cagagtcttg gaaggatgaa 480
ggtagtgaat gtgaacctgg atttttttcg gaatggtaca tccttgccat cacatcattc 540
ttggaattcg tgatcccagt catcttagtc gcttatttca acatgaatat ttattggagc 600
ctgtggaagc gtgatcatct cagtaggtgc caaagccatc ctggactgac tgctgtctct 660
tccaacatct gtggacactc attcagaggt agactatctt caaggagatc tctttctgca 720
tcgacagaag ttcctgcatc ctttcattca gagagacaga ggagaaagag tagtctcatg 780
ttttcctcaa gaaccaagat gaatagcaat acaattgctt ccaaaatggg ttccttctcc 840
caatcagatt ctgtagctct tcaccaaagg gaacatgttg aactgcttag agccaggaga 900
ttagccaagt cactggccat tctcttaggg gtttttgctg tttgctgggc tccatattct 960
ctgttcacaa ttgtcctttc attttattcc tcagcaacag gtcctaaatc agtttggtat 1020
agaattgcat tttggcttca gtggttcaat tcctttgtca atcctctttt gtatccattg 1080
tgtcacaagc gctttcaaaa ggctttcttg aaaatatttt gtataaaaaa gcaacctcta 1140
ccatcacaac acagtcggtc agtatcttct taa                              1173


<210> 14
<211> 390
<212> PRT
<213> Homo sapiens

<400> 14
Met Pro Asp Thr Asn Ser Thr Ile Asn Leu Ser Leu Ser Thr Arg Val
1               5                   10                  15

Thr Leu Ala Phe Phe Met Ser Leu Val Ala Phe Ala Ile Met Leu Gly
            20                  25                  30

Asn Ala Leu Val Ile Leu Ala Phe Val Val Asp Lys Asn Leu Arg His
        35                  40                  45

Arg Ser Ser Tyr Phe Phe Leu Asn Leu Ala Ile Ser Asp Phe Phe Val
    50                  55                  60

Gly Val Ile Ser Ile Pro Leu Tyr Ile Pro His Thr Leu Phe Glu Trp
65                  70                  75                  80

Asp Phe Gly Lys Glu Ile Cys Val Phe Trp Leu Thr Thr Asp Tyr Leu
                85                  90                  95

Leu Cys Thr Ala Ser Val Tyr Asn Ile Val Leu Ile Ser Tyr Asp Arg
            100                 105                 110

Tyr Leu Ser Val Ser Asn Ala Val Ser Tyr Arg Thr Gln His Thr Gly
        115                 120                 125

Val Leu Lys Ile Val Thr Leu Met Val Ala Val Trp Val Leu Ala Phe
    130                 135                 140

Leu Val Asn Gly Pro Met Ile Leu Val Ser Glu Ser Trp Lys Asp Glu
145                 150                 155                 160

Gly Ser Glu Cys Glu Pro Gly Phe Phe Ser Glu Trp Tyr Ile Leu Ala
                165                 170                 175

Ile Thr Ser Phe Leu Glu Phe Val Ile Pro Val Ile Leu Val Ala Tyr
            180                 185                 190

Phe Asn Met Asn Ile Tyr Trp Ser Leu Trp Lys Arg Asp His Leu Ser
        195                 200                 205

Arg Cys Gln Ser His Pro Gly Leu Thr Ala Val Ser Ser Asn Ile Cys
    210                 215                 220

Gly His Ser Phe Arg Gly Arg Leu Ser Ser Arg Arg Ser Leu Ser Ala
225                 230                 235                 240

Ser Thr Glu Val Pro Ala Ser Phe His Ser Glu Arg Gln Arg Arg Lys
                245                 250                 255

Ser Ser Leu Met Phe Ser Ser Arg Thr Lys Met Asn Ser Asn Thr Ile
            260                 265                 270

Ala Ser Lys Met Gly Ser Phe Ser Gln Ser Asp Ser Val Ala Leu His
        275                 280                 285

Gln Arg Glu His Val Glu Leu Leu Arg Ala Arg Arg Leu Ala Lys Ser
    290                 295                 300

Leu Ala Ile Leu Leu Gly Val Phe Ala Val Cys Trp Ala Pro Tyr Ser
305                 310                 315                 320

Leu Phe Thr Ile Val Leu Ser Phe Tyr Ser Ser Ala Thr Gly Pro Lys
                325                 330                 335

Ser Val Trp Tyr Arg Ile Ala Phe Trp Leu Gln Trp Phe Asn Ser Phe
            340                 345                 350

Val Asn Pro Leu Leu Tyr Pro Leu Cys His Lys Arg Phe Gln Lys Ala
        355                 360                 365

Phe Leu Lys Ile Phe Cys Ile Lys Lys Gln Pro Leu Pro Ser Gln His
    370                 375                 380

Ser Arg Ser Val Ser Ser
385                 390


<210> 15
<211> 1128
<212> DNA
<213> Homo sapiens

<400> 15
atggcgaacg cgagcgagcc gggtggcagc ggcggcggcg aggcggccgc cctgggcctc 60
aagctggcca cgctcagcct gctgctgtgc gtgagcctag cgggcaacgt gctgttcgcg 120
ctgctgatcg tgcgggagcg cagcctgcac cgcgccccgt actacctgct gctcgacctg 180
tgcctggccg acgggctgcg cgcgctcgcc tgcctcccgg ccgtcatgct ggcggcgcgg 240
cgtgcggcgg ccgcggcggg ggcgccgccg ggcgcgctgg gctgcaagct gctcgccttc 300
ctggccgcgc tcttctgctt ccacgccgcc ttcctgctgc tgggcgtggg cgtcacccgc 360

tacctggcca tcgcgcacca ccgcttctat gcagagcgcc tggccggctg gccgtgcgcc 420
gccatgctgg tgtgcgccgc ctgggcgctg gcgctggccg cggccttccc gccagtgctg 480
gacggcggtg gcgacgacga ggacgcgccg tgcgccctgg agcagcggcc cgacggcgcc 540
cccggcgcgc tgggcttcct gctgctgctg gccgtggtgg tgggcgccac gcacctcgtc 600
tacctccgcc tgctcttctt catccacgac cgccgcaaga tgcggcccgc gcgcctggtg 660
cccgccgtca gccacgactg gaccttccac ggcccgggcg ccaccggcca ggcggccgcc 720
aactggacgg cgggcttcgg ccgcgggccc acgccgcccg cgcttgtggg catccggccc 780
gcagggccgg gccgcggcgc gcgccgcctc ctcgtgctgg aagaattcaa gacggagaag 840
aggctgtgca agatgttcta cgccgtcacg ctgctcttcc tgctcctctg gggcccctac 900
gtcgtggcca gctacctgcg ggtcctggtg cggcccggcg ccgtccccca ggcctacctg 960
acggcctccg tgtggctgac cttcgcgcag gccggcatca accccgtcgt gtgcttcctc 1020
ttcaacaggg agctgaggga ctgcttcagg gcccagttcc cctgctgcca gagcccccgg 1080
accacccagg cgacccatcc ctgcgacctg aaaggcattg gtttatga 1128

<210> 16
<211> 375
<212> PRT
<213> Homo sapiens

<400> 16
Met Ala Asn Ala Ser Glu Pro Gly Gly Ser Gly Gly Gly Glu Ala Ala
1               5                   10                  15

Ala Leu Gly Leu Lys Leu Ala Thr Leu Ser Leu Leu Leu Cys Val Ser
            20                  25                  30

Leu Ala Gly Asn Val Leu Phe Ala Leu Leu Ile Val Arg Glu Arg Ser
        35                  40                  45

Leu His Arg Ala Pro Tyr Tyr Leu Leu Leu Asp Leu Cys Leu Ala Asp
    50                  55                  60

Gly Leu Arg Ala Leu Ala Cys Leu Pro Ala Val Met Leu Ala Ala Arg
65                  70                  75                  80

Arg Ala Ala Ala Ala Ala Gly Ala Pro Pro Gly Ala Leu Gly Cys Lys
                85                  90                  95

Leu Leu Ala Phe Leu Ala Ala Leu Phe Cys Phe His Ala Ala Phe Leu
            100                 105                 110

Leu Leu Gly Val Gly Val Thr Arg Tyr Leu Ala Ile Ala His His Arg
        115                 120                 125

Phe Tyr Ala Glu Arg Leu Ala Gly Trp Pro Cys Ala Ala Met Leu Val
    130                 135                 140

Cys Ala Ala Trp Ala Leu Ala Leu Ala Ala Ala Phe Pro Pro Val Leu
145                 150                 155                 160

Asp Gly Gly Gly Asp Asp Glu Asp Ala Pro Cys Ala Leu Glu Gln Arg
                165                 170                 175

Pro Asp Gly Ala Pro Gly Ala Leu Gly Phe Leu Leu Leu Leu Ala Val
            180                 185                 190

Val Val Gly Ala Thr His Leu Val Tyr Leu Arg Leu Leu Phe Phe Ile
        195                 200                 205

His Asp Arg Arg Lys Met Arg Pro Ala Arg Leu Val Pro Ala Val Ser
    210                 215                 220

His Asp Trp Thr Phe His Gly Pro Gly Ala Thr Gly Gln Ala Ala Ala
225                 230                 235                 240

Asn Trp Thr Ala Gly Phe Gly Arg Gly Pro Thr Pro Pro Ala Leu Val
                245                 250                 255

Gly Ile Arg Pro Ala Gly Pro Gly Arg Gly Ala Arg Arg Leu Leu Val
            260                 265                 270

Leu Glu Glu Phe Lys Thr Glu Lys Arg Leu Cys Lys Met Phe Tyr Ala
        275                 280                 285

Val Thr Leu Leu Phe Leu Leu Leu Trp Gly Pro Tyr Val Val Ala Ser
    290                 295                 300

Tyr Leu Arg Val Leu Val Arg Pro Gly Ala Val Pro Gln Ala Tyr Leu
305                 310                 315                 320

Thr Ala Ser Val Trp Leu Thr Phe Ala Gln Ala Gly Ile Asn Pro Val
                325                 330                 335

Val Cys Phe Leu Phe Asn Arg Glu Leu Arg Asp Cys Phe Arg Ala Gln
            340                 345                 350

Phe Pro Cys Cys Gln Ser Pro Arg Thr Thr Gln Ala Thr His Pro Cys
        355                 360                 365

Asp Leu Lys Gly Ile Gly Leu
    370                 375


<210> 17
<211> 1002
<212> DNA
<213> Homo sapiens

<400> 17
atgaacacca cagtgatgca aggcttcaac agatctgagc ggtgccccag agacactcgg 60
atagtacagc tggtattccc agccctctac acagtggttt tcttgaccgg catcctgctg 120
aatactttgg ctctgtgggt gtttgttcac atccccagct cctccacctt catcatctac 180
ctcaaaaaca ctttggtggc cgacttgata atgacactca tgcttccttt caaaatcctc 240
tctgactcac acctggcacc ctggcagctc agagcttttg tgtgtcgttt ttcttcggtg 300
atattttatg agaccatgta tgtgggcatc gtgctgttag ggctcatagc ctttgacaga 360
ttcctcaaga tcatcagacc tttgagaaat atttttctaa aaaaacctgt ttttgcaaaa 420
acggtctcaa tcttcatctg gttctttttg ttcttcatct ccctgccaaa tacgatcttg 480
agcaacaagg aagcaacacc atcgtctgtg aaaaagtgtg cttccttaaa ggggcctctg 540
gggctgaaat ggcatcaaat ggtaaataac atatgccagt ttattttctg gactgttttt 600
atcctaatgc ttgtgtttta tgtggttatt gcaaaaaaag tatatgattc ttatagaaag 660
tccaaaagta aggacagaaa aaacaacaaa aagctggaag gcaaagtatt tgttgtcgtg 720
gctgtcttct ttgtgtgttt tgctccattt cattttgcca gagttccata tactcacagt 780
caaaccaaca ataagactga ctgtagactg caaaatcaac tgtttattgc taaagaaaca 840
actctctttt tggcagcaac taacatttgt atggatccct taatatacat attcttatgt 900
aaaaaattca cagaaaagct accatgtatg caagggagaa agaccacagc atcaagccaa 960
gaaaatcata gcagtcagac agacaacata accttaggct ga                    1002


<210> 18
<211> 333
<212> PRT
<213> Homo sapiens

<400> 18
Met Asn Thr Thr Val Met Gln Gly Phe Asn Arg Ser Glu Arg Cys Pro
1               5                   10                  15

Arg Asp Thr Arg Ile Val Gln Leu Val Phe Pro Ala Leu Tyr Thr Val
            20                  25                  30

Val Phe Leu Thr Gly Ile Leu Leu Asn Thr Leu Ala Leu Trp Val Phe
        35                  40                  45

Val His Ile Pro Ser Ser Ser Thr Phe Ile Ile Tyr Leu Lys Asn Thr
    50                  55                  60

Leu Val Ala Asp Leu Ile Met Thr Leu Met Leu Pro Phe Lys Ile Leu
65                  70                  75                  80

Ser Asp Ser His Leu Ala Pro Trp Gln Leu Arg Ala Phe Val Cys Arg
                85                  90                  95

Phe Ser Ser Val Ile Phe Tyr Glu Thr Met Tyr Val Gly Ile Val Leu
            100                 105                 110

Leu Gly Leu Ile Ala Phe Asp Arg Phe Leu Lys Ile Ile Arg Pro Leu
        115                 120                 125

Arg Asn Ile Phe Leu Lys Lys Pro Val Phe Ala Lys Thr Val Ser Ile
    130                 135                 140

Phe Ile Trp Phe Phe Leu Phe Phe Ile Ser Leu Pro Asn Thr Ile Leu
145                 150                 155                 160

Ser Asn Lys Glu Ala Thr Pro Ser Ser Val Lys Lys Cys Ala Ser Leu
                165                 170                 175

Lys Gly Pro Leu Gly Leu Lys Trp His Gln Met Val Asn Asn Ile Cys
            180                 185                 190

Gln Phe Ile Phe Trp Thr Val Phe Ile Leu Met Leu Val Phe Tyr Val
        195                 200                 205

Val Ile Ala Lys Lys Val Tyr Asp Ser Tyr Arg Lys Ser Lys Ser Lys
    210                 215                 220

Asp Arg Lys Asn Asn Lys Lys Leu Glu Gly Lys Val Phe Val Val Val
225                 230                 235                 240

Ala Val Phe Phe Val Cys Phe Ala Pro Phe His Phe Ala Arg Val Pro
                245                 250                 255

Tyr Thr His Ser Gln Thr Asn Asn Lys Thr Asp Cys Arg Leu Gln Asn
            260                 265                 270

Gln Leu Phe Ile Ala Lys Glu Thr Thr Leu Phe Leu Ala Ala Thr Asn
        275                 280                 285

Ile Cys Met Asp Pro Leu Ile Tyr Ile Phe Leu Cys Lys Lys Phe Thr
    290                 295                 300

Glu Lys Leu Pro Cys Met Gln Gly Arg Lys Thr Thr Ala Ser Ser Gln
305                 310                 315                 320

Glu Asn His Ser Ser Gln Thr Asp Asn Ile Thr Leu Gly
                325                 330

<210> 19
<211> 1122
<212> DNA
<213> Homo sapiens

<400> 19
   atggccaaca ctaccggaga gcctgaggag gtgagcggcg ctctgtcccc accgtccgca 60
   tcagcttatg tgaagctggt actgctggga ctgattatgt gcgtgagcct ggcgggtaac 120
   gccatcttgt ccctgctggt gctcaaggag cgtgccctgc acaaggctcc ttactacttc 180
   ctgctggacc tgtgcctggc cgatggcata cgctctgccg tctgcttccc ctttgtgctg 240
   gcttctgtgc gccacggctc ttcatggacc ttcagtgcac tcagctgcaa gattgtggcc 300
   tttatggccg tgctcttttg cttccatgcg gccttcatgc tgttctgcat cagcgtcacc 360
   cgctacatgg ccatcgccca ccaccgcttc tacgccaagc gcatgacact ctggacatgc 420
   gcggctgtca tctgcatggc ctggaccctg tctgtggcca tggccttccc acctgtcttt 480
   gacgtgggca cctacaagtt tattcgggag gaggaccagt gcatctttga gcatcgctac 540
   ttcaaggcca atgacacgct gggcttcatg cttatgttgg ctgtgctcat ggcagctacc 600
   catgctgtct acggcaagct gctcctcttc gagtatcgtc accgcaagat gaagccagtg 660
   cagatggtgc cagccatcag ccagaactgg acattccatg gtcccggggc caccggccag 720
   gctgctgcca actggatcgc cggctttggc cgtgggccca tgccaccaac cctgctgggt 780
   atccggcaga atgggcatgc agccagccgg cggctactgg gcatggacga ggtcaagggt 840
   gaaaagcagc tgggccgcat gttctacgcg atcacactgc tctttctgct cctctggtca 900
   ccctacatcg tggcctgcta ctggcgagtg tttgtgaaag cctgtgctgt gccccaccgc 960
   tacctggcca ctgctgtttg gatgagcttc gcccaggctg ccgtcaaccc aattgtctgc 1020
   ttcctgctca acaaggacct caagaagtgc ctgaccactc acgccccctg ctggggcaca 1080
   ggaggtgccc cggctcccag agaaccctac tgtgtcatgt ga 1122

<210> 20
<211> 373
<212> PRT
<213> Homo sapiens

<400> 20
Met Ala Asn Thr Thr Gly Glu Pro Glu Glu Val Ser Gly Ala Leu Ser
1               5                   10                  15

Pro Pro Ser Ala Ser Ala Tyr Val Lys Leu Val Leu Leu Gly Leu Ile
            20                  25                  30

Met Cys Val Ser Leu Ala Gly Asn Ala Ile Leu Ser Leu Leu Val Leu
        35                  40                  45

Lys Glu Arg Ala Leu His Lys Ala Pro Tyr Tyr Phe Leu Leu Asp Leu
    50                  55                  60

Cys Leu Ala Asp Gly Ile Arg Ser Ala Val Cys Phe Pro Phe Val Leu
65                  70                  75                  80

Ala Ser Val Arg His Gly Ser Ser Trp Thr Phe Ser Ala Leu Ser Cys
                85                  90                  95

Lys Ile Val Ala Phe Met Ala Val Leu Phe Cys Phe His Ala Ala Phe
            100                 105                 110

Met Leu Phe Cys Ile Ser Val Thr Arg Tyr Met Ala Ile Ala His His
        115                 120                 125

Arg Phe Tyr Ala Lys Arg Met Thr Leu Trp Thr Cys Ala Ala Val Ile
    130                 135                 140

Cys Met Ala Trp Thr Leu Ser Val Ala Met Ala Phe Pro Pro Val Phe
145                 150                 155                 160

Asp Val Gly Thr Tyr Lys Phe Ile Arg Glu Glu Asp Gln Cys Ile Phe
                165                 170                 175

Glu His Arg Tyr Phe Lys Ala Asn Asp Thr Leu Gly Phe Met Leu Met
            180                 185                 190

Leu Ala Val Leu Met Ala Ala Thr His Ala Val Tyr Gly Lys Leu Leu
        195                 200                 205

Leu Phe Glu Tyr Arg His Arg Lys Met Lys Pro Val Gln Met Val Pro
    210                 215                 220

Ala Ile Ser Gln Asn Trp Thr Phe His Gly Pro Gly Ala Thr Gly Gln
225                 230                 235                 240

Ala Ala Ala Asn Trp Ile Ala Gly Phe Gly Arg Gly Pro Met Pro Pro
                245                 250                 255

Thr Leu Leu Gly Ile Arg Gln Asn Gly His Ala Ala Ser Arg Arg Leu
            260                 265                 270

Leu Gly Met Asp Glu Val Lys Gly Glu Lys Gln Leu Gly Arg Met Phe
        275                 280                 285

Tyr Ala Ile Thr Leu Leu Phe Leu Leu Leu Trp Ser Pro Tyr Ile Val
    290                 295                 300

Ala Cys Tyr Trp Arg Val Phe Val Lys Ala Cys Ala Val Pro His Arg
305                 310                 315                 320

Tyr Leu Ala Thr Ala Val Trp Met Ser Phe Ala Gln Ala Ala Val Asn
                325                 330                 335

Pro Ile Val Cys Phe Leu Leu Asn Lys Asp Leu Lys Lys Cys Leu Thr
            340                 345                 350

Thr His Ala Pro Cys Trp Gly Thr Gly Gly Ala Pro Ala Pro Arg Glu
        355                 360                 365

Pro Tyr Cys Val Met
    370


<210> 21
<211> 1053
<212> DNA
<213> Homo sapiens

<400> 21
   atggctttgg aacagaacca gtcaacagat tattattatg aggaaaatga aatgaatggc 60
   acttatgact acagtcaata tgaattgatc tgtatcaaag aagatgtcag agaatttgca 120
   aaagttttcc tccctgtatt cctcacaata gctttcgtca ttggacttgc aggcaattcc 180
   atggtagtgg caatttatgc ctattacaag aaacagagaa ccaaaacaga tgtgtacatc 240
   ctgaatttgg ctgtagcaga tttactcctt ctattcactc tgcctttttg ggctgttaat 300
   gcagttcatg ggtgggtttt agggaaaata atgtgcaaaa taacttcagc cttgtacaca 360
   ctaaactttg tctctggaat gcagtttctg gcttgcatca gcatagacag atatgtggca 420
   gtaactaatg tccccagcca atcaggagtg ggaaaaccat gctggatcat ctgtttctgt 480
   gtctggatgg ctgccatctt gctgagcata ccccagctgg ttttttatac agtaaatgac 540
   aatgctaggt gcattcccat tttcccccgc tacctaggaa catcaatgaa agcattgatt 600
   caaatgctag agatctgcat tggatttgta gtaccctttc ttattatggg ggtgtgctac 660
   tttatcacgg caaggacact catgaagatg ccaaacatta aaatatctcg acccctaaaa 720
   gttctgctca cagtcgttat agttttcatt gtcactcaac tgccttataa cattgtcaag 780
   ttctgccgag ccatagacat catctactcc ctgatcacca gctgcaacat gagcaaacgc 840
   atggacatcg ccatccaagt cacagaaagc attgcactct ttcacagctg cctcaaccca 900
   atcctttatg tttttatggg agcatctttc aaaaactacg ttatgaaagt ggccaagaaa 960
   tatgggtcct ggagaagaca gagacaaagt gtggaggagt ttccttttga ttctgagggt 1020
   cctacagagc caaccagtac ttttagcatt taa                              1053


<210> 22
<211> 350
<212> PRT
<213> Homo sapiens

<400> 22

<210> 23
<211> 1116
<212> DNA
<213> Homo sapiens

<400> 23
atgccaggaa acgccacccc agtgaccacc actgccccgt gggcctccct gggcctctcc 60
gccaagacct gcaacaacgt gtccttcgaa gagagcagga tagtcctggt cgtggtgtac 120
agcgcggtgt gcacgctggg ggtgccggcc aactgcctga ctgcgtggct ggcgctgctg 180
caggtactgc agggcaacgt gctggccgtc tacctgctct gcctggcact ctgcgaactg 240
ctgtacacag gcacgctgcc actctgggtc atctatatcc gcaaccagca ccgctggacc 300
ctaggcctgc tggcctcgaa ggtgaccgcc tacatcttct tctgcaacat ctacgtcagc 360
atcctcttcc tgtgctgcat ctcctgcgac cgcttcgtgg ccgtggtgta cgcgctggag 420
agtcggggcc gccgccgccg gaggaccgcc atcctcatct ccgcctgcat cttcatcctc 480

gtcgggatcg ttcactaccc ggtgttccag acggaagaca aggagacctg ctttgacatg 540
ctgcagatgg acagcaggat tgccgggtac tactacgcca ggttcaccgt tggctttgcc 600
atccctctct ccatcatcgc cttcaccaac caccggattt tcaggagcat caagcagagc 660
atgggcttaa gcgctgccca gaaggccaag gtgaagcact cggccatcgc ggtggttgtc 720
atcttcctag tctgcttcgc cccgtaccac ctggttctcc tcgtcaaagc cgctgccttt 780
tcctactaca gaggagacag gaacgccatg tgcggcttgg aggaaaggct gtacacagcc 840
tctgtggtgt ttctgtgcct gtccacggtg aacggcgtgg ctgaccccat tatctacgtg 900
ctggccacgg accattcccg ccaagaagtg tccagaatcc ataaggggtg gaaagagtgg 960
tccatgaaga cagacgtcac caggctcacc cacagcaggg acaccgagga gctgcagtcg 1020
cccgtggccc ttgcagacca ctacaccttc tccaggcccg tgcacccacc agggtcacca 1080
tgccctgcaa agaggctgat tgaggagtcc tgctga 1116

<210> 24
<211> 371
<212> PRT
<213> Homo sapiens

<400> 24
Met Pro Gly Asn Ala Thr Pro Val Thr Thr Thr Ala Pro Trp Ala Ser
1               5                   10                  15

Leu Gly Leu Ser Ala Lys Thr Cys Asn Asn Val Ser Phe Glu Glu Ser
            20                  25                  30

Arg Ile Val Leu Val Val Val Tyr Ser Ala Val Cys Thr Leu Gly Val
        35                  40                  45

Pro Ala Asn Cys Leu Thr Ala Trp Leu Ala Leu Leu Gln Val Leu Gln
    50                  55                  60

Gly Asn Val Leu Ala Val Tyr Leu Leu Cys Leu Ala Leu Cys Glu Leu
65                  70                  75                  80

Leu Tyr Thr Gly Thr Leu Pro Leu Trp Val Ile Tyr Ile Arg Asn Gln
                85                  90                  95

His Arg Trp Thr Leu Gly Leu Leu Ala Ser Lys Val Thr Ala Tyr Ile
            100                 105                 110

Phe Phe Cys Asn Ile Tyr Val Ser Ile Leu Phe Leu Cys Cys Ile Ser
        115                 120                 125

Cys Asp Arg Phe Val Ala Val Val Tyr Ala Leu Glu Ser Arg Gly Arg
    130                 135                 140

Arg Arg Arg Arg Thr Ala Ile Leu Ile Ser Ala Cys Ile Phe Ile Leu
145                 150                 155                 160

Val Gly Ile Val His Tyr Pro Val Phe Gln Thr Glu Asp Lys Glu Thr
                165                 170                 175

Cys Phe Asp Met Leu Gln Met Asp Ser Arg Ile Ala Gly Tyr Tyr Tyr
            180                 185                 190

Ala Arg Phe Thr Val Gly Phe Ala Ile Pro Leu Ser Ile Ile Ala Phe
        195                 200                 205

Thr Asn His Arg Ile Phe Arg Ser Ile Lys Gln Ser Met Gly Leu Ser
    210                 215                 220

Ala Ala Gln Lys Ala Lys Val Lys His Ser Ala Ile Ala Val Val Val
225                 230                 235                 240

Ile Phe Leu Val Cys Phe Ala Pro Tyr His Leu Val Leu Leu Val Lys
                245                 250                 255

Ala Ala Ala Phe Ser Tyr Tyr Arg Gly Asp Arg Asn Ala Met Cys Gly

260                 265                 270

Leu Glu Glu Arg Leu Tyr Thr Ala Ser Val Val Phe Leu Cys Leu Ser
        275                 280                 285

Thr Val Asn Gly Val Ala Asp Pro Ile Ile Tyr Val Leu Ala Thr Asp
    290                 295                 300

His Ser Arg Gln Glu Val Ser Arg Ile His Lys Gly Trp Lys Glu Trp
305                 310                 315                 320

Ser Met Lys Thr Asp Val Thr Arg Leu Thr His Ser Arg Asp Thr Glu
                325                 330                 335

Glu Leu Gln Ser Pro Val Ala Leu Ala Asp His Tyr Thr Phe Ser Arg
            340                 345                 350

Pro Val His Pro Pro Gly Ser Pro Cys Pro Ala Lys Arg Leu Ile Glu
        355                 360                 365

Glu Ser Cys
    370

<210> 25
<211> 1113
<212> DNA
<213> Homo sapiens

<400> 25
atggcgaact atagccatgc agctgacaac attttgcaaa atctctcgcc tctaacagcc 60
tttctgaaac tgacttcctt gggtttcata ataggagtca gcgtggtggg caacctcctg 120
atctccattt tgctagtgaa agataagacc ttgcatagag caccttacta cttcctgttg 180
gatctttgct gttcagatat cctcagatct gcaatttgtt tcccatttgt gttcaactct 240
gtcaaaaatg gctctacctg gacttatggg actctgactt gcaaagtgat tgcctttctg 300
ggggttttgt cctgtttcca cactgctttc atgctcttct gcatcagtgt caccagatac 360
ttagctatcg cccatcaccg cttctataca aagaggctga ccttttggac gtgtctggct 420
gtgatctgta tggtgtggac tctgtctgtg gccatggcat ttcccccggt tttagacgtg 480
ggcacttact cattcattag ggaggaagat caatgcacct tccaacaccg ctccttcagg 540
gctaatgatt ccttaggatt tatgctgctt cttgctctca tcctcctagc cacacagctt 600
gtctacctca agctgatatt tttcgtccac gatcgaagaa aaatgaagcc agtccagttt 660
gtagcagcag tcagccagaa ctggactttt catggtcctg gagccagtgg ccaggcagct 720
gccaattggc tagcaggatt tggaaggggt cccacaccac ccaccttgct gggcatcagg 780
caaaatgcaa acaccacagg cagaagaagg ctattggtct tagacgagtt caaaatggag 840
aaaagaatca gcagaatgtt ctatataatg acttttctgt ttctaacctt gtggggcccc 900
tacctggtgg cctgttattg gagagttttt gcaagagggc ctgtagtacc agggggattt 960
ctaacagctg ctgtctggat gagttttgcc caagcaggaa tcaatccttt tgtctgcatt 1020
ttctcaaaca gggagctgag gcgctgtttc agcacaaccc ttctttactg cagaaaatcc 1080
aggttaccaa gggaacctta ctgtgttata tga                              1113

<210> 26
<211> 370
<212> PRT
<213> Homo sapiens

<400> 26
Met Ala Asn Tyr Ser His Ala Ala Asp Asn Ile Leu Gln Asn Leu Ser
1               5                   10                  15

Pro Leu Thr Ala Phe Leu Lys Leu Thr Ser Leu Gly Phe Ile Ile Gly
            20                  25                  30

Val Ser Val Val Gly Asn Leu Leu Ile Ser Ile Leu Leu Val Lys Asp
        35                  40                  45

Lys Thr Leu His Arg Ala Pro Tyr Tyr Phe Leu Leu Asp Leu Cys Cys
    50                  55                  60

Ser Asp Ile Leu Arg Ser Ala Ile Cys Phe Pro Phe Val Phe Asn Ser
65                  70                  75                  80

Val Lys Asn Gly Ser Thr Trp Thr Tyr Gly Thr Leu Thr Cys Lys Val
                85                  90                  95

Ile Ala Phe Leu Gly Val Leu Ser Cys Phe His Thr Ala Phe Met Leu
            100                 105                 110

Phe Cys Ile Ser Val Thr Arg Tyr Leu Ala Ile Ala His His Arg Phe
        115                 120                 125

Tyr Thr Lys Arg Leu Thr Phe Trp Thr Cys Leu Ala Val Ile Cys Met
    130                 135                 140

Val Trp Thr Leu Ser Val Ala Met Ala Phe Pro Pro Val Leu Asp Val
145                 150                 155                 160

Gly Thr Tyr Ser Phe Ile Arg Glu Glu Asp Gln Cys Thr Phe Gln His
                165                 170                 175

Arg Ser Phe Arg Ala Asn Asp Ser Leu Gly Phe Met Leu Leu Leu Ala
            180                 185                 190

Leu Ile Leu Leu Ala Thr Gln Leu Val Tyr Leu Lys Leu Ile Phe Phe
        195                 200                 205

Val His Asp Arg Arg Lys Met Lys Pro Val Gln Phe Val Ala Ala Val
    210                 215                 220

Ser Gln Asn Trp Thr Phe His Gly Pro Gly Ala Ser Gly Gln Ala Ala
225                 230                 235                 240

Ala Asn Trp Leu Ala Gly Phe Gly Arg Gly Pro Thr Pro Pro Thr Leu
                245                 250                 255

Leu Gly Ile Arg Gln Asn Ala Asn Thr Thr Gly Arg Arg Arg Leu Leu
            260                 265                 270

Val Leu Asp Glu Phe Lys Met Glu Lys Arg Ile Ser Arg Met Phe Tyr
        275                 280                 285

Ile Met Thr Phe Leu Phe Leu Thr Leu Trp Gly Pro Tyr Leu Val Ala
    290                 295                 300

Cys Tyr Trp Arg Val Phe Ala Arg Gly Pro Val Val Pro Gly Gly Phe
305                 310                 315                 320

Leu Thr Ala Ala Val Trp Met Ser Phe Ala Gln Ala Gly Ile Asn Pro
                325                 330                 335

Phe Val Cys Ile Phe Ser Asn Arg Glu Leu Arg Arg Cys Phe Ser Thr
            340                 345                 350

Thr Leu Leu Tyr Cys Arg Lys Ser Arg Leu Pro Arg Glu Pro Tyr Cys
        355                 360                 365

Val Ile
    370


<210> 27
<211> 1080
<212> DNA
<213> Homo sapiens

<400> 27

atgcaggtcc cgaacagcac cggcccggac aacgcgacgc tgcagatgct gcggaacccg 60
gcgatcgcgg tggccctgcc cgtggtgtac tcgctggtgg cggcggtcag catcccgggc 120
aacctcttct ctctgtgggt gctgtgccgg cgcatggggc ccagatcccc gtcggtcatc 180
ttcatgatca acctgagcgt cacggacctg atgctggcca gcgtgttgcc tttccaaatc 240
tactaccatt gcaaccgcca ccactgggta ttcggggtgc tgctttgcaa cgtggtgacc 300
gtggcctttt acgcaaacat gtattccagc atcctcacca tgacctgtat cagcgtggag 360
cgcttcctgg gggtcctgta cccgctcagc tccaagcgct ggcgccgccg tcgttacgcg 420
gtggccgcgt gtgcagggac ctggctgctg ctcctgaccg ccctgtgccc gctggcgcgc 480
accgatctca cctacccggt gcacgccctg ggcatcatca cctgcttcga cgtcctcaag 540
tggacgatgc tccccagcgt ggccatgtgg gccgtgttcc tcttcaccat cttcatcctg 600
ctgttcctca tcccgttcgt gatcaccgtg gcttgttaca cggccaccat cctcaagctg 660
ttgcgcacgg aggaggcgca cggccgggag cagcggaggc gcgcggtggg cctggccgcg 720
gtggtcttgc tggcctttgt cacctgcttc gcccccaaca acttcgtgct cctggcgcac 780
atcgtgagcc gcctgttcta cggcaagagc tactaccacg tgtacaagct cacgctgtgt 840
ctcagctgcc tcaacaactg tctggacccg tttgtttatt actttgcgtc ccgggaattc 900
cagctgcgcc tgcgggaata tttgggctgc cgccgggtgc ccagagacac cctggacacg 960
cgccgcgaga gcctcttctc cgccaggacc acgtccgtgc gctccgaggc cggtgcgcac 1020
cctgaaggga tggagggagc caccaggccc ggcctccaga ggcaggagag tgtgttctga 1080

<210> 28
<211> 359
<212> PRT
<213> Homo sapiens

<400> 28
Met Gln Val Pro Asn Ser Thr Gly Pro Asp Asn Ala Thr Leu Gln Met
1               5                   10                  15

Leu Arg Asn Pro Ala Ile Ala Val Ala Leu Pro Val Val Tyr Ser Leu
            20                  25                  30

Val Ala Ala Val Ser Ile Pro Gly Asn Leu Phe Ser Leu Trp Val Leu
        35                  40                  45

Cys Arg Arg Met Gly Pro Arg Ser Pro Ser Val Ile Phe Met Ile Asn
    50                  55                  60

Leu Ser Val Thr Asp Leu Met Leu Ala Ser Val Leu Pro Phe Gln Ile
65                  70                  75                  80

Tyr Tyr His Cys Asn Arg His His Trp Val Phe Gly Val Leu Leu Cys
                85                  90                  95

Asn Val Val Thr Val Ala Phe Tyr Ala Asn Met Tyr Ser Ser Ile Leu
            100                 105                 110

Thr Met Thr Cys Ile Ser Val Glu Arg Phe Leu Gly Val Leu Tyr Pro
        115                 120                 125

Leu Ser Ser Lys Arg Trp Arg Arg Arg Arg Tyr Ala Val Ala Ala Cys
    130                 135                 140

Ala Gly Thr Trp Leu Leu Leu Leu Thr Ala Leu Cys Pro Leu Ala Arg
145                 150                 155                 160

Thr Asp Leu Thr Tyr Pro Val His Ala Leu Gly Ile Ile Thr Cys Phe
                165                 170                 175

Asp Val Leu Lys Trp Thr Met Leu Pro Ser Val Ala Met Trp Ala Val
            180                 185                 190

Phe Leu Phe Thr Ile Phe Ile Leu Leu Phe Leu Ile Pro Phe Val Ile
        195                 200                 205

Thr Val Ala Cys Tyr Thr Ala Thr Ile Leu Lys Leu Leu Arg Thr Glu
    210                 215                 220

Glu Ala His Gly Arg Glu Gln Arg Arg Arg Ala Val Gly Leu Ala Ala
225                 230                 235                 240

Val Val Leu Leu Ala Phe Val Thr Cys Phe Ala Pro Asn Asn Phe Val
                245                 250                 255

Leu Leu Ala His Ile Val Ser Arg Leu Phe Tyr Gly Lys Ser Tyr Tyr
            260                 265                 270

His Val Tyr Lys Leu Thr Leu Cys Leu Ser Cys Leu Asn Asn Cys Leu
        275                 280                 285

Asp Pro Phe Val Tyr Tyr Phe Ala Ser Arg Glu Phe Gln Leu Arg Leu
    290                 295                 300

Arg Glu Tyr Leu Gly Cys Arg Arg Val Pro Arg Asp Thr Leu Asp Thr
305                 310                 315                 320

Arg Arg Glu Ser Leu Phe Ser Ala Arg Thr Thr Ser Val Arg Ser Glu
                325                 330                 335

Ala Gly Ala His Pro Glu Gly Met Glu Gly Ala Thr Arg Pro Gly Leu
            340                 345                 350

Gln Arg Gln Glu Ser Val Phe
        355

<210> 29
<211> 1503
<212> DNA
<213> Homo sapiens

<400> 29
atggagcgtc cctgggagga cagcccaggc ccggaggggg cagctgaggg ctcgcctgtg 60
ccagtcgccg ccggggcgcg ctccggtgcc gcggcgagtg gcacaggctg gcagccatgg 120
gctgagtgcc cgggacccaa ggggaggggg caactgctgg cgaccgccgg ccctttgcgt 180
cgctggcccg ccccctcgcc tgccagctcc agccccgccc ccggagcggc gtccgctcac 240
tcggttcaag gcagcgcgac tgcgggtggc gcacgaccag ggcgcagacc ttggggcgcg 300
cggcccatgg agtcggggct gctgcggccg gcgccggtga gcgaggtcat cgtcctgcat 360
tacaactaca ccggcaagct ccgcggtgcg agctaccagc cgggtgccgg cctgcgcgcc 420
gacgccgtgg tgtgcctggc ggtgtgcgcc ttcatcgtgc tagagaatct agccgtgttg 480
ttggtgctcg gacgccaccc gcgcttccac gctcccatgt tcctgctcct gggcagcctc 540
acgttgtcgg atctgctggc aggcgccgcc tacgccgcca acatcctact gtcggggccg 600
ctcacgctga aactgtcccc cgcgctctgg ttcgcacggg agggaggcgt cttcgtggca 660
ctcactgcgt ccgtgctgag cctcctggcc atcgcgctgg agcgcagcct caccatggcg 720
cgcagggggc ccgcgcccgt ctccagtcgg gggcgcacgc tggcgatggc agccgcggcc 780
tggggcgtgt cgctgctcct cgggctcctg ccagcgctgg gctggaattg cctgggtcgc 840
ctggacgctt gctccactgt cttgccgctc tacgccaagg cctacgtgct cttctgcgtg 900
ctcgccttcg tgggcatcct ggccgcgatc tgtgcactct acgcgcgcat ctactgccag 960
gtacgcgcca acgcgcggcg cctgccggca cggcccggga ctgcggggac cacctcgacc 1020
cgggcgcgtc gcaagccgcg ctctctggcc ttgctgcgca cgctcagcgt ggtgctcctg 1080
gcctttgtgg catgttgggg cccccctcttc ctgctgctgt tgctcgacgt ggcgtgcccg 1140
gcgcgcacct gtcctgtact cctgcaggcc gatcccttcc tgggactggc catggccaac 1200
tcacttctga accccatcat ctacacgctc accaaccgcg acctgcgcca cgcgctcctg 1260
cgcctggtct gctgcggacg ccactcctgc ggcagagacc cgagtggctc ccagcagtcg 1320
gcgagcgcgg ctgaggcttc cgggggcctg cgccgctgcc tgcccccggg ccttgatggg 1380
agcttcagcg gctcggagcg ctcatcgccc cagcgcgacg ggctggacac cagcggctcc 1440
acaggcagcc ccggtgcacc cacagccgcc cggactctgg tatcagaacc ggctgcagac 1500
tga                                                               1503

<210> 30
<211> 500
<212> PRT
<213> Homo sapiens

<400> 30

Met Glu Arg Pro Trp Glu Asp Ser Pro Gly Pro Glu Gly Ala Ala Glu
1               5                   10                  15

Gly Ser Pro Val Pro Val Ala Ala Gly Ala Arg Ser Gly Ala Ala Ala
            20                  25                  30

Ser Gly Thr Gly Trp Gln Pro Trp Ala Glu Cys Pro Gly Pro Lys Gly
        35                  40                  45

Arg Gly Gln Leu Leu Ala Thr Ala Gly Pro Leu Arg Arg Trp Pro Ala
    50                  55                  60

Pro Ser Pro Ala Ser Ser Ser Pro Ala Pro Gly Ala Ala Ser Ala His
65                  70                  75                  80

Ser Val Gln Gly Ser Ala Thr Ala Gly Gly Ala Arg Pro Gly Arg Arg
                85                  90                  95

Pro Trp Gly Ala Arg Pro Met Glu Ser Gly Leu Leu Arg Pro Ala Pro
            100                 105                 110

Val Ser Glu Val Ile Val Leu His Tyr Asn Tyr Thr Gly Lys Leu Arg
        115                 120                 125

Gly Ala Ser Tyr Gln Pro Gly Ala Gly Leu Arg Ala Asp Ala Val Val
    130                 135                 140

Cys Leu Ala Val Cys Ala Phe Ile Val Leu Glu Asn Leu Ala Val Leu
145                 150                 155                 160

Leu Val Leu Gly Arg His Pro Arg Phe His Ala Pro Met Phe Leu Leu
                165                 170                 175

Leu Gly Ser Leu Thr Leu Ser Asp Leu Leu Ala Gly Ala Ala Tyr Ala
            180                 185                 190

Ala Asn Ile Leu Leu Ser Gly Pro Leu Thr Leu Lys Leu Ser Pro Ala
        195                 200                 205

Leu Trp Phe Ala Arg Glu Gly Gly Val Phe Val Ala Leu Thr Ala Ser
    210                 215                 220

Val Leu Ser Leu Leu Ala Ile Ala Leu Glu Arg Ser Leu Thr Met Ala
225                 230                 235                 240

Arg Arg Gly Pro Ala Pro Val Ser Ser Arg Gly Arg Thr Leu Ala Met
                245                 250                 255

Ala Ala Ala Ala Trp Gly Val Ser Leu Leu Leu Gly Leu Leu Pro Ala
            260                 265                 270

Leu Gly Trp Asn Cys Leu Gly Arg Leu Asp Ala Cys Ser Thr Val Leu
        275                 280                 285

Pro Leu Tyr Ala Lys Ala Tyr Val Leu Phe Cys Val Leu Ala Phe Val
    290                 295                 300

Gly Ile Leu Ala Ala Ile Cys Ala Leu Tyr Ala Arg Ile Tyr Cys Gln
305                 310                 315                 320

Val Arg Ala Asn Ala Arg Arg Leu Pro Ala Arg Pro Gly Thr Ala Gly
                325                 330                 335

Thr Thr Ser Thr Arg Ala Arg Arg Lys Pro Arg Ser Leu Ala Leu Leu
            340                 345                 350

Arg Thr Leu Ser Val Val Leu Leu Ala Phe Val Ala Cys Trp Gly Pro
        355                 360                 365

Leu Phe Leu Leu Leu Leu Leu Asp Val Ala Cys Pro Ala Arg Thr Cys
    370                 375                 380

Pro Val Leu Leu Gln Ala Asp Pro Phe Leu Gly Leu Ala Met Ala Asn
385                 390                 395                 400

Ser Leu Leu Asn Pro Ile Ile Tyr Thr Leu Thr Asn Arg Asp Leu Arg
                405                 410                 415

His Ala Leu Leu Arg Leu Val Cys Cys Gly Arg His Ser Cys Gly Arg
            420                 425                 430

Asp Pro Ser Gly Ser Gln Gln Ser Ala Ser Ala Ala Glu Ala Ser Gly
        435                 440                 445

Gly Leu Arg Arg Cys Leu Pro Pro Gly Leu Asp Gly Ser Phe Ser Gly
    450                 455                 460

Ser Glu Arg Ser Ser Pro Gln Arg Asp Gly Leu Asp Thr Ser Gly Ser
465                 470                 475                 480

Thr Gly Ser Pro Gly Ala Pro Thr Ala Ala Arg Thr Leu Val Ser Glu
                485                 490                 495

Pro Ala Ala Asp
            500


<210> 31
<211> 1029
<212> DNA
<213> Homo sapiens

<400> 31
atgcaagccg tcgacaatct cacctctgcg cctgggaaca ccagtctgtg caccagagac 60
tacaaaatca cccaggtcct cttcccactg ctctacactg tcctgttttt tgttggactt 120
atcacaaatg gcctggcgat gaggattttc tttcaaatcc ggagtaaatc aaactttatt 180
atttttctta agaacacagt catttctgat cttctcatga ttctgacttt tccattcaaa 240
attcttagtg atgccaaact gggaacagga ccactgagaa cttttgtgtg tcaagttacc 300
tccgtcatat tttatttcac aatgtatatc agtatttcat tcctgggact gataactatc 360
gatcgctacc agaagaccac caggccattt aaaacatcca accccaaaaa tctcttgggg 420
gctaagattc tctctgttgt catctgggca ttcatgttct tactctcttt gcctaacatg 480
attctgacca acaggcagcc gagagacaag aatgtgaaga aatgctcttt ccttaaatca 540
gagttcggtc tagtctggca tgaaatagta aattacatct gtcaagtcat tttctggatt 600
aatttcttaa ttgttattgt atgttataca ctcattacaa aagaactgta ccggtcatac 660
gtaagaacga ggggtgtagg taaagtcccc aggaaaaagg tgaacgtcaa agttttcatt 720
atcattgctg tattctttat ttgttttgtt cctttccatt ttgcccgaat tccttacacc 780
ctgagccaaa cccgggatgt ctttgactgc actgctgaaa atactctgtt ctatgtgaaa 840
gagagcactc tgtggttaac ttccttaaat gcatgcctgg atccgttcat ctattttttc 900
ctttgcaagt ccttcagaaa ttccttgata agtatgctga agtgccccaa ttctgcaaca 960
tctctgtccc aggacaatag gaaaaaagaa caggatggtg gtgacccaaa tgaagagact 1020
ccaatgtaa                                                         1029


<210> 32
<211> 342
<212> PRT
<213> Homo sapiens

<400> 32

<210> 33
<211> 1077
<212> DNA
<213> Homo sapiens

<400> 33
atgtcggtct gctaccgtcc cccagggaac gagacactgc tgagctggaa gacttcgcgg 60
gccacaggca cagccttcct gctgctggcg gcgctgctgg ggctgcctgg caacggcttc 120

gtggtgtgga gcttggcggg ctggcggcct gcacgggggc gaccgctggc ggccacgctt 180
gtgctgcacc tggcgctggc cgacggcgcg gtgctgctgc tcacgccgct ctttgtggcc 240
ttcctgaccc ggcaggcctg gccgctgggc caggcgggct gcaaggcggt gtactacgtg 300
tgcgcgctca gcatgtacgc cagcgtgctg ctcaccggcc tgctcagcct gcagcgctgc 360
ctcgcagtca cccgcccctt cctggcgcct cggctgcgca gcccggccct ggcccgccgc 420
ctgctgctgg cggtctggct ggccgccctg ttgctcgccg tcccggccgc cgtctaccgc 480
cacctgtgga gggaccgcgt atgccagctg tgccacccgt cgccggtcca cgccgccgcc 540
cacctgagcc tggagactct gaccgctttc gtgcttcctt tcgggctgat gctcggctgc 600
tacagcgtga cgctggcacg gctgcggggc gcccgctggg gctccgggcg gcacggggcg 660
cgggtgggcc ggctggtgag cgccatcgtg cttgccttcg gcttgctctg ggccccctac 720
cacgcagtca accttctgca ggcggtcgca gcgctggctc caccggaagg ggccttggcg 780
aagctgggcg gagccggcca ggcggcgcga gcgggaacta cggccttggc cttcttcagt 840
tctagcgtca acccggtgct ctacgtcttc accgctggag atctgctgcc ccgggcaggt 900
ccccgtttcc tcacgcggct cttcgaaggc tctggggagg cccgaggggg cggccgctct 960
agggaaggga ccatggagct ccgaactacc cctcagctga aagtggtggg gcagggccgc 1020
ggcaatggag acccgggggg tgggatggag aaggacggtc cggaatggga cctttga 1077

<210> 34
<211> 358
<212> PRT
<213> Homo sapiens

<400> 34
Met Ser Val Cys Tyr Arg Pro Pro Gly Asn Glu Thr Leu Leu Ser Trp
1               5                   10                  15

Lys Thr Ser Arg Ala Thr Gly Thr Ala Phe Leu Leu Leu Ala Ala Leu
            20                  25                  30

Leu Gly Leu Pro Gly Asn Gly Phe Val Val Trp Ser Leu Ala Gly Trp
        35                  40                  45

Arg Pro Ala Arg Gly Arg Pro Leu Ala Ala Thr Leu Val Leu His Leu
    50                  55                  60

Ala Leu Ala Asp Gly Ala Val Leu Leu Leu Thr Pro Leu Phe Val Ala
65                  70                  75                  80

Phe Leu Thr Arg Gln Ala Trp Pro Leu Gly Gln Ala Gly Cys Lys Ala
                85                  90                  95

Val Tyr Tyr Val Cys Ala Leu Ser Met Tyr Ala Ser Val Leu Leu Thr
            100                 105                 110

Gly Leu Leu Ser Leu Gln Arg Cys Leu Ala Val Thr Arg Pro Phe Leu
        115                 120                 125

Ala Pro Arg Leu Arg Ser Pro Ala Leu Ala Arg Arg Leu Leu Leu Ala
    130                 135                 140

Val Trp Leu Ala Ala Leu Leu Leu Ala Val Pro Ala Ala Val Tyr Arg
145                 150                 155                 160

His Leu Trp Arg Asp Arg Val Cys Gln Leu Cys His Pro Ser Pro Val
                165                 170                 175

His Ala Ala Ala His Leu Ser Leu Glu Thr Leu Thr Ala Phe Val Leu
            180                 185                 190

Pro Phe Gly Leu Met Leu Gly Cys Tyr Ser Val Thr Leu Ala Arg Leu
        195                 200                 205

Arg Gly Ala Arg Trp Gly Ser Gly Arg His Gly Ala Arg Val Gly Arg
    210                 215                 220

Leu Val Ser Ala Ile Val Leu Ala Phe Gly Leu Leu Trp Ala Pro Tyr
225                 230                 235                 240

His Ala Val Asn Leu Leu Gln Ala Val Ala Ala Leu Ala Pro Pro Glu
                245                 250                 255

Gly Ala Leu Ala Lys Leu Gly Gly Ala Gly Gln Ala Ala Arg Ala Gly
            260                 265                 270

Thr Thr Ala Leu Ala Phe Phe Ser Ser Ser Val Asn Pro Val Leu Tyr
        275                 280                 285

Val Phe Thr Ala Gly Asp Leu Leu Pro Arg Ala Gly Pro Arg Phe Leu
    290                 295                 300

Thr Arg Leu Phe Glu Gly Ser Gly Glu Ala Arg Gly Gly Gly Arg Ser
305                 310                 315                 320

Arg Glu Gly Thr Met Glu Leu Arg Thr Thr Pro Gln Leu Lys Val Val
                325                 330                 335

Gly Gln Gly Arg Gly Asn Gly Asp Pro Gly Gly Gly Met Glu Lys Asp
            340                 345                 350

Gly Pro Glu Trp Asp Leu
        355

<210> 35
<211> 1005
<212> DNA
<213> Homo sapiens

<400> 35
atgctgggga tcatggcatg gaatgcaact tgcaaaaact ggctggcagc agaggctgcc 60
ctggaaaagt actacctttc catttttat gggattgagt tcgttgtggg agtccttgga 120
aataccattg ttgtttacgg ctacatcttc tctctgaaga actggaacag cagtaatatt 180
tatctcttta acctctctgt ctctgactta gcttttctgt gcaccctccc catgctgata 240
aggagttatg ccaatggaaa ctggatatat ggagacgtgc tctgcataag caaccgatat 300
gtgcttcatg ccaacctcta taccagcatt ctctttctca cttttatcag catagatcga 360
tacttgataa ttaagtatcc tttccgagaa caccttctgc aaaagaaaga gtttgctatt 420
ttaatctcct tggccatttg ggttttagta accttagagt tactacccat acttcccctt 480
ataaatcctg ttataactga caatggcacc acctgtaatg attttgcaag ttctggagac 540
cccaactaca acctcattta cagcatgtgt ctaacactgt tggggttcct tattcctctt 600
tttgtgatgt gtttctttta ttacaagatt gctctcttcc taaagcagag gaataggcag 660
gttgctactg ctctgcccct tgaaaagcct ctcaacttgg tcatcatggc agtggtaatc 720
ttctctgtgc tttttacacc ctatcacgtc atgcggaatg tgaggatcgc ttcacgcctg 780
gggagttgga agcagtatca gtgcactcag gtcgtcatca actcctttta cattgtgaca 840
cggcctttgg cctttctgaa cagtgtcatc aaccctgtct tctattttct tttgggagat 900
cacttcaggg acatgctgat gaatcaactg agacacaact tcaaatccct tacatccttt 960
agcagatggg ctcatgaact cctactttca ttcagagaaa agtga                 1005

<210> 36
<211> 334
<212> PRT
<213> Homo sapiens

<400> 36
Met Leu Gly Ile Met Ala Trp Asn Ala Thr Cys Lys Asn Trp Leu Ala
1               5                   10                  15

Ala Glu Ala Ala Leu Glu Lys Tyr Tyr Leu Ser Ile Phe Tyr Gly Ile
            20                  25                  30

Glu Phe Val Val Gly Val Leu Gly Asn Thr Ile Val Val Tyr Gly Tyr
        35                  40                  45

Ile Phe Ser Leu Lys Asn Trp Asn Ser Ser Asn Ile Tyr Leu Phe Asn
    50                  55                  60

Leu Ser Val Ser Asp Leu Ala Phe Leu Cys Thr Leu Pro Met Leu Ile
65                  70                  75                  80

Arg Ser Tyr Ala Asn Gly Asn Trp Ile Tyr Gly Asp Val Leu Cys Ile
                85                  90                  95

Ser Asn Arg Tyr Val Leu His Ala Asn Leu Tyr Thr Ser Ile Leu Phe
            100                 105                 110

Leu Thr Phe Ile Ser Ile Asp Arg Tyr Leu Ile Ile Lys Tyr Pro Phe
        115                 120                 125

Arg Glu His Leu Leu Gln Lys Lys Glu Phe Ala Ile Leu Ile Ser Leu
    130                 135                 140

Ala Ile Trp Val Leu Val Thr Leu Glu Leu Leu Pro Ile Leu Pro Leu
145                 150                 155                 160

Ile Asn Pro Val Ile Thr Asp Asn Gly Thr Thr Cys Asn Asp Phe Ala
                165                 170                 175

Ser Ser Gly Asp Pro Asn Tyr Asn Leu Ile Tyr Ser Met Cys Leu Thr
            180                 185                 190

Leu Leu Gly Phe Leu Ile Pro Leu Phe Val Met Cys Phe Phe Tyr Tyr
        195                 200                 205

Lys Ile Ala Leu Phe Leu Lys Gln Arg Asn Arg Gln Val Ala Thr Ala
    210                 215                 220

Leu Pro Leu Glu Lys Pro Leu Asn Leu Val Ile Met Ala Val Val Ile
225                 230                 235                 240

Phe Ser Val Leu Phe Thr Pro Tyr His Val Met Arg Asn Val Arg Ile
                245                 250                 255

Ala Ser Arg Leu Gly Ser Trp Lys Gln Tyr Gln Cys Thr Gln Val Val
            260                 265                 270

Ile Asn Ser Phe Tyr Ile Val Thr Arg Pro Leu Ala Phe Leu Asn Ser
        275                 280                 285

Val Ile Asn Pro Val Phe Tyr Phe Leu Leu Gly Asp His Phe Arg Asp
    290                 295                 300

Met Leu Met Asn Gln Leu Arg His Asn Phe Lys Ser Leu Thr Ser Phe
305                 310                 315                 320

Ser Arg Trp Ala His Glu Leu Leu Leu Ser Phe Arg Glu Lys
                325                 330


<210> 37
<211> 1296
<212> DNA
<213> Homo sapiens

<400> 37
atgcaggcgc ttaacattac cccggagcag ttctctcggc tgctgcggga ccacaacctg 60
acgcgggagc agttcatcgc tctgtaccgg ctgcgaccgc tcgtctacac cccagagctg 120
ccgggacgcg ccaagctggc cctcgtgctc accggcgtgc tcatcttcgc cctggcgctc 180
tttggcaatg ctctggtgtt ctacgtggtg acccgcagca aggccatgcg caccgtcacc 240
aacatcttta tctgctcctt ggcgctcagt gacctgctca tcaccttctt ctgcattccc 300
gtcaccatgc tccagaacat ttccgacaac tggctggggg gtgctttcat ttgcaagatg 360
gtgccatttg tccagtctac cgctgttgtg acagaaatgc tcactatgac ctgcattgct 420
gtggaaaggc accagggact tgtgcatcct tttaaaatga agtggcaata caccaaccga 480
agggctttca caatgctagg tgtggtctgg ctggtggcag tcatcgtagg atcacccatg 540

tggcacgtgc aacaacttga gatcaaatat gacttcctat atgaaaagga acacatctgc 600
tgcttagaag agtggaccag ccctgtgcac cagaagatct acaccacctt catccttgtc 660
atcctcttcc tcctgcctct tatggtgatg cttattctgt acagtaaaat tggttatgaa 720
ctttggataa agaaaagagt tggggatggt tcagtgcttc gaactattca tggaaaagaa 780
atgtccaaaa tagccaggaa gaagaaacga gctgtcatta tgatggtgac agtggtggct 840
ctctttgctg tgtgctgggc accattccat gttgtccata tgatgattga atacagtaat 900
tttgaaaagg aatatgatga tgtcacaatc aagatgattt ttgctatcgt gcaaattatt 960
ggattttcca actccatctg taatcccatt gtctatgcat ttatgaatga aaacttcaaa 1020
aaaaatgttt tgtctgcagt ttgttattgc atagtaaata aaaccttctc tccagcacaa 1080
aggcatggaa attcaggaat tacaatgatg cggaagaaag caaagttttc cctcagagag 1140
aatccagtgg aggaaaccaa aggagaagca ttcagtgatg gcaacattga agtcaaattg 1200
tgtgaacaga cagaggagaa gaaaaagctc aaacgacatc ttgctctctt taggtctgaa 1260
ctggctgaga attctccttt agacagtggg cattaa 1296

<210> 38
<211> 431
<212> PRT
<213> Homo sapiens

<400> 38
Met Gln Ala Leu Asn Ile Thr Pro Glu Gln Phe Ser Arg Leu Leu Arg
1               5                   10                  15

Asp His Asn Leu Thr Arg Glu Gln Phe Ile Ala Leu Tyr Arg Leu Arg
            20                  25                  30

Pro Leu Val Tyr Thr Pro Glu Leu Pro Gly Arg Ala Lys Leu Ala Leu
        35                  40                  45

Val Leu Thr Gly Val Leu Ile Phe Ala Leu Ala Leu Phe Gly Asn Ala
    50                  55                  60

Leu Val Phe Tyr Val Val Thr Arg Ser Lys Ala Met Arg Thr Val Thr
65                  70                  75                  80

Asn Ile Phe Ile Cys Ser Leu Ala Leu Ser Asp Leu Leu Ile Thr Phe
                85                  90                  95

Phe Cys Ile Pro Val Thr Met Leu Gln Asn Ile Ser Asp Asn Trp Leu
            100                 105                 110

Gly Gly Ala Phe Ile Cys Lys Met Val Pro Phe Val Gln Ser Thr Ala
        115                 120                 125

Val Val Thr Glu Met Leu Thr Met Thr Cys Ile Ala Val Glu Arg His
    130                 135                 140

Gln Gly Leu Val His Pro Phe Lys Met Lys Trp Gln Tyr Thr Asn Arg
145                 150                 155                 160

Arg Ala Phe Thr Met Leu Gly Val Val Trp Leu Val Ala Val Ile Val
                165                 170                 175

Gly Ser Pro Met Trp His Val Gln Gln Leu Glu Ile Lys Tyr Asp Phe
            180                 185                 190

Leu Tyr Glu Lys Glu His Ile Cys Cys Leu Glu Glu Trp Thr Ser Pro
        195                 200                 205

Val His Gln Lys Ile Tyr Thr Thr Phe Ile Leu Val Ile Leu Phe Leu
    210                 215                 220

Leu Pro Leu Met Val Met Leu Ile Leu Tyr Ser Lys Ile Gly Tyr Glu
225                 230                 235                 240

Leu Trp Ile Lys Lys Arg Val Gly Asp Gly Ser Val Leu Arg Thr Ile
                245                 250                 255

His Gly Lys Glu Met Ser Lys Ile Ala Arg Lys Lys Lys Arg Ala Val
            260                 265                 270

Ile Met Met Val Thr Val Val Ala Leu Phe Ala Val Cys Trp Ala Pro
        275                 280                 285

Phe His Val Val His Met Met Ile Glu Tyr Ser Asn Phe Glu Lys Glu
    290                 295                 300

Tyr Asp Asp Val Thr Ile Lys Met Ile Phe Ala Ile Val Gln Ile Ile
305                 310                 315                 320

Gly Phe Ser Asn Ser Ile Cys Asn Pro Ile Val Tyr Ala Phe Met Asn
                325                 330                 335

Glu Asn Phe Lys Lys Asn Val Leu Ser Ala Val Cys Tyr Cys Ile Val
            340                 345                 350

Asn Lys Thr Phe Ser Pro Ala Gln Arg His Gly Asn Ser Gly Ile Thr
        355                 360                 365

Met Met Arg Lys Lys Ala Lys Phe Ser Leu Arg Glu Asn Pro Val Glu
    370                 375                 380

Glu Thr Lys Gly Glu Ala Phe Ser Asp Gly Asn Ile Glu Val Lys Leu
385                 390                 395                 400

Cys Glu Gln Thr Glu Glu Lys Lys Lys Leu Lys Arg His Leu Ala Leu
                405                 410                 415

Phe Arg Ser Glu Leu Ala Glu Asn Ser Pro Leu Asp Ser Gly His
            420                 425                 430

<210> 39
<211> 24
<212> DNA
<213> Homo sapiens

<400> 39
ctgtgtacag cagttcgcag agtg                                          24

<210> 40
<211> 24
<212> DNA
<213> Homo sapiens

<400> 40
gagtgccagg cagagcaggt agac                                          24

<210> 41
<211> 31
<212> DNA
<213> Homo sapiens

<400> 41
cccgaattcc tgcttgctcc cagcttggcc c                                  31

<210> 42
<211> 32
<212> DNA
<213> Homo sapiens

<400> 42

tgtggatcct gctgtcaaag gtcccattcc gg                                    32

<210> 43
<211> 20
<212> DNA
<213> Homo sapiens

<400> 43
tcacaatgct aggtgtggtc                                                  20

<210> 44
<211> 22
<212> DNA
<213> Homo sapiens

<400> 44
tgcatagaca atgggattac ag                                               22

<210> 45
<211> 511
<212> DNA
<213> Homo sapiens

<400> 45
tcacaatgct aggtgtggtc tggctggtgg cagtcatcgt aggatcaccc atgtggcacg 60
tgcaacaact tgagatcaaa tatgacttcc tatatgaaaa ggaacacatc tgctgcttag 120
aagagtggac cagccctgtg caccagaaga tctacaccac cttcatcctt gtcatcctct 180
tcctcctgcc tcttatggtg atgcttattc tgtacgtaaa attggttatg aactttggat 240
aaagaaaaga gttggggatg gttcagtgct tcgaactatt catggaaaag aaatgtccaa 300
aatagccagg aagaagaaac gagctgtcat tatgatggtg acagtggtgg ctctctttgc 360
tgtgtgctgg gcaccattcc atgttgtcca tatgatgatt gaatacagta attttgaaaa 420
ggaatatgat gatgtcacaa tcaagatgat ttttgctatc gtgcaaatta ttggattttc 480
caactccatc tgtaatccca ttgtctatgc a                                    511

<210> 46
<211> 21
<212> DNA
<213> Homo sapiens

<400> 46
ctgcttagaa gagtggacca g                                                21

<210> 47
<211> 22
<212> DNA
<213> Homo sapiens

<400> 47
ctgtgcacca gaagatctac ac                                               22

<210> 48
<211> 21
<212> DNA
<213> Homo sapiens

<400> 48
caaggatgaa ggtggtgtag a                                                21

<210> 49
<211> 23
<212> DNA

<213> Homo sapiens

<400> 49
gtgtagatct tctggtgcac agg                                              23


<210> 50
<211> 21
<212> DNA
<213> Homo sapiens

<400> 50
gcaatgcagg tcatagtgag c                                                21


<210> 51
<211> 27
<212> DNA
<213> Homo sapiens

<400> 51
tggagcatgg tgacgggaat gcagaag                                          27


<210> 52
<211> 27
<212> DNA
<213> Homo sapiens

<400> 52
gtgatgagca ggtcactgag cgccaag                                          27


<210> 53
<211> 23
<212> DNA
<213> Homo sapiens

<400> 53
gcaatgcagg cgcttaacat tac                                              23


<210> 54
<211> 22
<212> DNA
<213> Homo sapiens

<400> 54
ttgggttaca atctgaaggg ca                                               22


<210> 55
<211> 23
<212> DNA
<213> Homo sapiens

<400> 55
actccgtgtc cagcaggact ctg                                              23


<210> 56
<211> 24
<212> DNA
<213> Homo sapiens

<400> 56
tgcgtgttcc tggaccctca cgtg                                             24

<210> 57
<211> 29
<212> DNA
<213> Homo sapiens

<400> 57
caggccttgg attttaatgt cagggatgg                                          29


<210> 58
<211> 27
<212> DNA
<213> Homo sapiens

<400> 58
ggagagtcag ctctgaaaga attcagg                                            27


<210> 59
<211> 27
<212> DNA
<213> Homo sapiens

<400> 59
tgatgtgatg ccagatacta atagcac                                            27


<210> 60
<211> 27
<212> DNA
<213> Homo sapiens

<400> 60
cctgattcat ttaggtgaga ttgagac                                            27


<210> 61
<211> 21
<212> DNA
<213> Homo sapiens

<400> 61
gacaggtacc ttgccatcaa g                                                  21


<210> 62
<211> 22
<212> DNA
<213> Homo sapiens

<400> 62
ctgcacaatg ccagtgataa gg                                                 22


<210> 63
<211> 27
<212> DNA
<213> Homo sapiens

<400> 63
ctgacttctt gttcctggca gcagcgg                                            27


<210> 64
<211> 27
<212> DNA
<213> Homo sapiens

<400> 64
agaccagcca gggcacgctg aagagtg                                          27


<210> 65
<211> 32
<212> DNA
<213> Homo sapiens

<400> 65
gatcaagctt ccatcctact gaaaccatgg tc                                    32


<210> 66
<211> 35
<212> DNA
<213> Homo sapiens

<400> 66
gatcagatct cagttccaat attcacacca ccgtc                                 35


<210> 67
<211> 22
<212> DNA
<213> Homo sapiens

<400> 67
ctggtgtgct ccatggcatc cc                                               22


<210> 68
<211> 22
<212> DNA
<213> Homo sapiens

<400> 68
gtaagcctcc cagaacgaga gg                                               22


<210> 69
<211> 24
<212> DNA
<213> Homo sapiens

<400> 69
cagcgcaggg tgaagcctga gagc                                             24


<210> 70
<211> 24
<212> DNA
<213> Homo sapiens

<400> 70
ggcacctgct gtgacctgtg cagg                                             24


<210> 71
<211> 22
<212> DNA
<213> Homo sapiens

<400> 71
gtcctgccac ttcgagacat gg                                               22

<210> 72
<211> 23
<212> DNA
<213> Homo sapiens

<400> 72
gaaacttctc tgcccttacc gtc                                                23


<210> 73
<211> 26
<212> DNA
<213> Homo sapiens

<400> 73
ccaacaccag catccatggc atcaag                                             26


<210> 74
<211> 27
<212> DNA
<213> Homo sapiens

<400> 74
ggagagtcag ctctgaaaga attcagg                                            27




1

## Claims

**1.** A cDNA comprising a nucleotide sequence selected from:
a) a nucleotide sequence encoding a G protein-coupled receptor comprising the amino acid sequence of SEQ ID NO: 14; and
b) the nucleotide sequence encoding a human G protein coupled receptor comprising SEQ ID NO: 13.

**2.** A plasmid comprising the cDNA of claim 1.

**3.** A host cell comprising the plasmid of claim 2.

**4.** An expression vector comprising a cDNA having a nucleotide sequence selected from:
a) a nucleotide sequence encoding a G protein-coupled receptor comprising the amino acid sequence of SEQ ID NO: 14; and
b) the nucleotide sequence encoding a human G protein coupled receptor comprising SEQ ID NO: 13.

**5.** A host cell comprising the expression vector of claim 4.

**7.** An isolated G protein-coupled receptor comprising an amino acid sequence of SEQ ID NO: 14.

**8.** Use of a G-protein coupled receptor to screen candidate compounds as pharmaceutical agents for a disease or disorder state related to peripheral leukocytes, wherein the G-protein coupled receptor comprises a ligand-independent active version of a receptor having SEQ ID NO. 14.

**9.** The use of claim 8, wherein the ligand-independent active version of the receptor is a non-endogenous ligand-independent active version having a mutation positioned 16 amino acid residues N-terminal from the conserved proline residue within the TM6 domain of SEQ ID NO. 14.

**10.** The use of claim 9, wherein the mutated residue has been mutated to a lysine residue.

**11.** The use of claim 8, wherein the ligand-independent active version of the receptor is an endogenous ligand-independent active version having SEQ ID NO. 14.

**12.** The use of any one of claims 8 to 11, wherein the G protein-coupled receptor forms part of a fusion protein with a G protein.

**13.** The use of any one of claims 8 to 12, wherein the screen is for an agonist of the receptor.

**14.** The use of any one of claims 8 to 13, wherein the screen is for a partial agonist of the receptor.

**15.** The use of any one of claims 8 to 12, wherein the screen is for an inverse agonist of the receptor.

**16.** A G-protein coupled receptor which is a non-endogenous ligand-independent active version of a receptor comprising SEQ ID NO. 14, having a mutation positioned 16 amino acid residues N-terminal from the conserved proline residue within the TM6 domain of SEQ ID NO. 14.

**17.** The G-protein coupled receptor of claim 16, wherein the mutated residue has been mutated to a lysine residue.

**18.** An isolated nucleic acid encoding a G-protein coupled receptor according to claim 16 or 17.

**19.** A vector comprising a nucleic acid according to claim 18.

**20.** A host cell comprising a vector according to claim 19.

**21.** A method of screening candidate compounds to identify a pharmaceutical agent for a disease or disorder state related to peripheral leukocytes, the method comprising:
providing a eukaryotic host cell comprising a G-protein coupled receptor or a membrane of a cell expressing a G protein-coupled receptor, wherein said G protein-coupled receptor is a ligand-independent active version of a receptor having SEQ ID NO: 14, wherein the receptor couples to a G-protein; and
screening candidate compounds against said G-protein coupled receptor.

**22.** The method of claim 21, wherein the cell is a eukaryotic host cell.

**23.** The method of claim 21, wherein the eukaryotic host cell is a mammalian host cell or a yeast cell.

**24.** The method of claim 21 or claim 22, wherein said screening comprises detecting cAMP.

**25.** The method of claim 24, wherein said detection of cAMP comprises ELISA using an anti-cAMP antibody.

**26.** The method of claim 24, wherein the cell comprises a reporter system comprising multiple cAMP responsive elements operably linked to a reporter gene.

**27.** The method of claim 21 or claim 22, wherein said screening comprises detecting IP3 accumulation.

**28.** The method of claim 21 or claim 22, wherein said screening comprises detecting [35S]GTPγS binding.

**29.** The method of any one of claims 21 to 28, wherein the method comprises identifying an agonist of the G-protein coupled receptor.

**30.** The method of any one of claims 21 to 28, wherein the method comprises identifying a partial agonist of the G-protein coupled receptor.

**31.** The method of any one of claims 21 to 28, wherein the method comprises identifying an inverse agonist of the G-protein coupled receptor.

**32.** A method according to any one of claims 29 to 31, wherein the method further comprises confirming that the candidate compound binds to the receptor.

**33.** The method of any one of claims 21 to 32, wherein the receptor is an endogenous receptor having SEQ ID NO:14.

**34.** The method of any one of claims 21 to 32, wherein said receptor is a non-endogenous receptor having a mutation positioned 16 amino acid residues N-terminal from the conserved proline residue within the TM6 domain of SEQ ID NO:14.
